# EUROPEAN PATENT APPLICATION

(11) **EP 4 375 294 A1**
(43) Date of publication of application: **29.05.2024**
(21) Application number: 22846209.9
(22) Date of filing: 20.07.2022
(51) Int. Cl.: C07K 16/18, A61P 35/00, A61K 39/00

(54) **CELL-PENETRATING DEGRADING ANTIBODY THAT PENETRATES INTO CELLS TO DEGRADE AND REMOVE TARGET PROTEIN AND USE THEREOF**

(30) Priority: 20.07.2021 KR 20210094792
(71) Applicant: Ajou University Industry-Academic Cooperation Foundation, Gyeonggi-do 16499 (KR)
(72) Inventor: KIM, Yong Sung, Suwon-si Gyeonggi-do 16504 (KR); LEE, Dasom, Gimcheon-si Gyeongsangbuk-do 39655 (KR); SEO, Jisu, Ansan-si Gyeonggi-do 15562 (KR); KWEON, Hae Jin, Yongin-si Gyeonggi-do 16925 (KR); KIM, Dae-Seong, Seoul 08346 (KR)
(74) Representative: Mewburn Ellis LLP
(86) International application number: PCT/KR2022/010589
(87) International publication number: WO 2023/003339

(57) **Abstract**

The present invention relates to: a cell-penetrating degrading antibody in which U-Box of E3 that can bind to E2 of an ubiquitin-proteasome system (UPS), which is an intracellular protein degradation system, or an E3 recruiter (E3 recruiting ligand) that can bind to E3 of the UPS is fused to a cell-penetrating interfering antibody that penetrates into the cytosol of living cells and specifically recognizes a target protein; a composition comprising same; and use thereof.

## Description

### [Technical Field]

The present invention relates to a cell-penetrating degrading antibody (also called a "Degrobody") that penetrates the cytosol of living cells and thereby specifically recognizes, degrades, and removes target proteins in the cytosol, a composition containing the same and the use thereof.

The present invention relates to a cell-penetrating degrading antibody that mimics the mechanism and function of the ubiquitin-proteasome system (UPS), which is an intracellular protein degradation system, and thereby specifically degrades target proteins recognized by the antibody, a composition containing the same, and the use thereof.

The present invention relates to a cell-penetrating degrading antibody that is formed by fusing a cell/tissue-specific cytosol-penetrating interfering antibody (iMab) that penetrates the cytosol of living cells and specifically recognizes target proteins with U-Box of E3 (ubiquitin ligase enzyme) that can bind to E2 (ubiquitin-conjugating enzyme), which is one of a series of enzymes of the UPS, a composition containing the same, and the use thereof.

More specifically, the present invention relates to a cell-penetrating degrading antibody that is formed by fusing a cell/tissue-specific cytosol-penetrating interfering antibody (iMab) that penetrates the cytosol of living cells and specifically recognizes target proteins with U-Box of E3 that can bind to E2, which is one of a series of enzymes of the UPS, to induce poly-ubiquitination (poly-Ub) of the target protein bound to the cytosol-penetrating interfering antibody by the E2 and thereby ultimately cause degradation of target proteins by the UPS.

The present invention relates to a cell-penetrating degrading antibody that is formed by fusing a cell/tissue-specific cytosol-penetrating interfering antibody (iMab) that penetrates the cytosol of living cells and specifically recognizes target proteins with U-Box of an E3 recruiter (E3 recruiting ligand) that can bind to E3, which is one of a series of enzymes of the UPS, a composition containing the same, and the use thereof.

More specifically, the present invention relates to a cell-penetrating degrading antibody in the form of a complete immunoglobulin, that is designed by fusing, with U-Box or E3 recruiter, a cytosol-penetrating interfering antibody that binds to a membrane protein receptor overexpressed on the surface of a target cell such as a tumor tissue, is internalized (undergoes endocytosis), is located in the cytosol due to endosomal escape ability thereof, and binds to target proteins in the cytosol, wherein the U-Box binds to E2 and localizes the E2 close to the target protein to induce poly-ubiquitination of the target protein, and the E3 recruiter binds to E3 and localizes the E3 close to the target protein to induce poly-ubiquitination of the target protein, thereby ultimately inducing degradation of poly-Ub-labeled target proteins by the ubiquitin-proteasome system (UPS).

The present invention relates to search and improvement of U-boxes that can be recruited with optimal degradation E2 depending on the target protein in the cytosol of the cell-penetrating degrading antibody.

The present invention relates to search and improvement of E3 recruiters that can be recruited with optimal degradation E3 depending on the target protein in the cytosol of the cell-penetrating degrading antibody.

In addition, the present invention relates to optimization of fusion sites and linkers of U-Boxes or E3 recruiters in the cell-penetrating degrading antibody.

The present invention relates to development of cell-penetrating degrading antibodies called "inBC2-E4B₁₂₂₇₋₁₃₀₂", which are designed by fusing an anti-β-catenin cytosol-penetrating interfering antibody (inBC2) with E4B₁₂₂₇₋₁₃₀₂, as U-Boxes, or cell-penetrating degrading antibodies called "inBC2-E3 recruiters", which are designed by fusing the anti-β-catenin cytosol-penetrating interfering antibody (inBC2) with E3 recruiters. More specifically, the present invention relates to cell-penetrating degrading antibodies called "inBC2-E4B₁₂₂₇₋₁₃₀₂", which are designed by fusing an anti-β-catenin cytosol-penetrating interfering antibody (inBC2) that is internalized through a receptor membrane protein on the surface of cancer cells, penetrates the cytosol by endosomal escape ability and binds to the target protein, β-catenin, with E4B₁₂₂₇₋₁₃₀₂, as U-Boxes, or cell-penetrating degrading antibodies called "inBC2-E3 recruiters", which are designed by fusing the anti-β-catenin cytosol-penetrating interfering antibody (inBC2) with E3 recruiters, wherein inBC2-E4B₁₂₂₇₋₁₃₀₂ recruit E2, locate the E2 close to the target protein, and induce poly-ubiquitination of the target protein, β-catenin, and the inBC2-E3 recruiters recruit E3, localize E2 binding to E3 close to the target protein and induce poly-ubiquitination of the target protein, thereby ultimately leading to the degradation of poly-ubiquitinated β-catenin, a composition containing the same, and the use thereof.

### [Background Art]

Intracellular protein degradation occurs through two pathways based on lysosome and proteasome. About 80% of cellular proteins are labeled with ubiquitin (Ub) and then degraded in the cytosol and the nucleus by the proteasome. This process is called "ubiquitin-proteasome system (UPS)". The degradation control system mediated by ubiquitin not only functions to maintain cellular homeostasis by removing damaged or unnecessary proteins, but also participates in cell cycle processes and transcription control processes that should be precisely regulated over time. Problems associated with UPS may cause various diseases, including cancer. On the other hand, it has been suggested that removal of disease-causing proteins using activation of the UPS pathway could be a fundamental treatment.

Ubiquitin is a protein composed of 76 amino acids and is present in almost all eukaryotic cells. A series of enzyme systems, namely, ubiquitin activating enzyme (E1), ubiquitin conjugating enzyme (E2), and ubiquitin ligase enzyme (E3) are involved in the process of labeling in order for ubiquitin to selectively degrade proteins, which is called "ubiquitination". Poly-ubiquitination (poly-Ub), in which a substrate protein (substrate) is chained/multi-labeled with ubiquitin chains, is induced and the poly-ubiquitinated target protein is transferred to 26S proteasome, which is an ATP-dependent protease complex, unfolded, and degraded. In other words, the UPS is a system composed of three types of enzymes, E1, E2, and E3. E1 activates Ub, E2 receives the activated Ub to form an E2-Ub (E2-ubiquitin conjugate), E3 binds to the E2-Ub and the substrate to poly-ubiquitinate the protein, and the poly-ubiquitinated substrate is degraded in the proteasome. Binding of the Ub to the protein substrate occurs through the formation of an isopeptide bond between the Lys (lysine) residue of the substrate molecule and another Ub may be bind to the Gly (glycine) residue of the C-terminus of Ub. By repeating this process, poly-Ub is labeled with multiple Ub molecules chained to the substrate protein, the protein is recognized by the 26S proteasome and selectively degraded.

The human UPS is expected to have 2 types of E1, 40 types of E2, and 600 types of E3. E1 activates Ub, and E2 receives the activated Ub to form E2-Ub. E3 binds to the substrate protein and the E2-Ub so that the substrate protein is located close to E2-Ub to induce poly-Ub conversion of the substrate protein. In particular, E3s are classified into RING (really interesting new gene) E3s, HECT (homologous to the E6AP carboxyl terminus) E3s, and RBR (RING-Between-RING) E3s, depending on the presence of the characteristic domain structure and differences in mechanism to deliver the Ub. Importantly, E3 binds to both E2-Ub and the substrate protein, providing specificity to recognize the substrate protein to be labeled with Ub. In other words, poly-ubiquitination of the protein to be degraded is determined by the E3 enzyme. In this case, all substrate proteins have a Ub linkage site and a recognition site for a specific E3. Considering the about 100,000 types of proteins in the cell, each E3 has not only substrate specificity, but also redundancy and multiplicity.

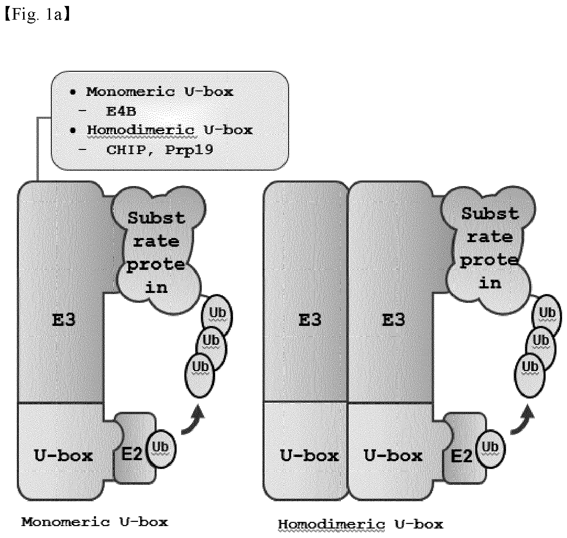

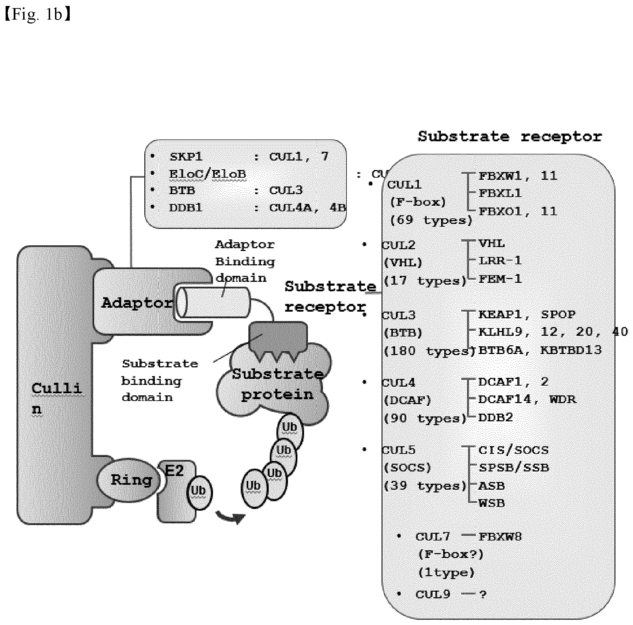

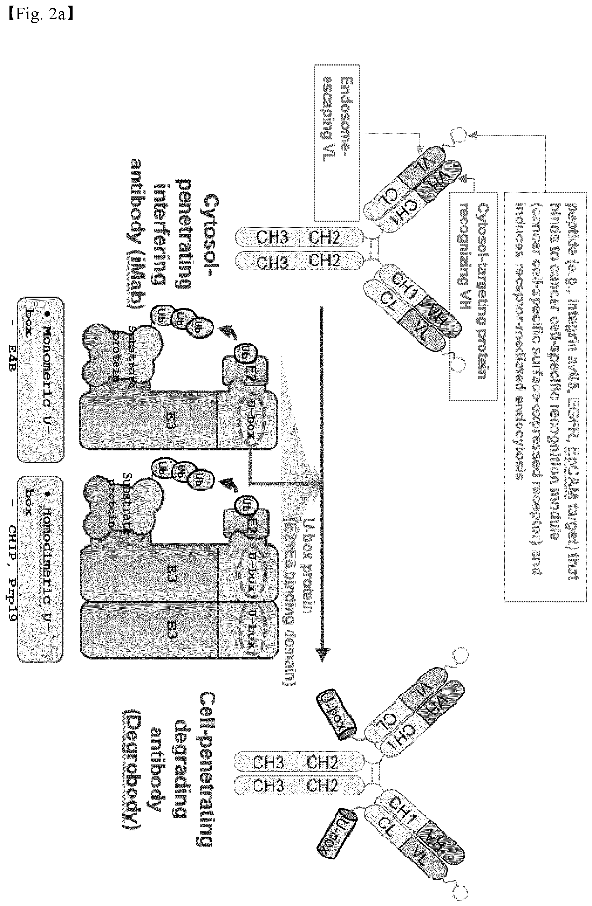

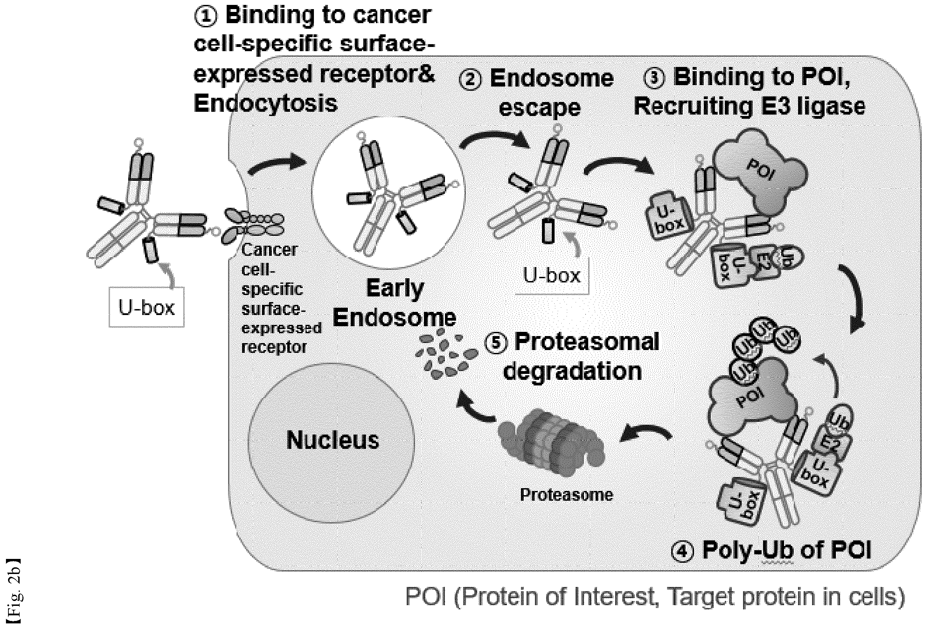

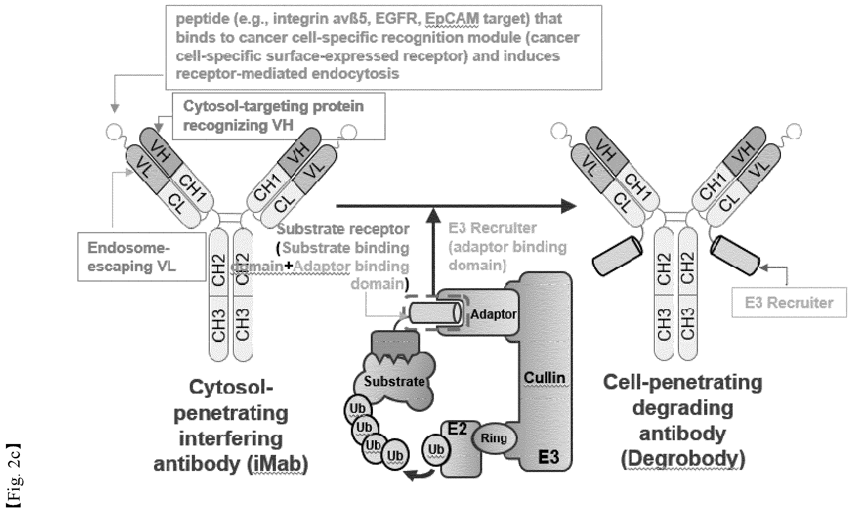

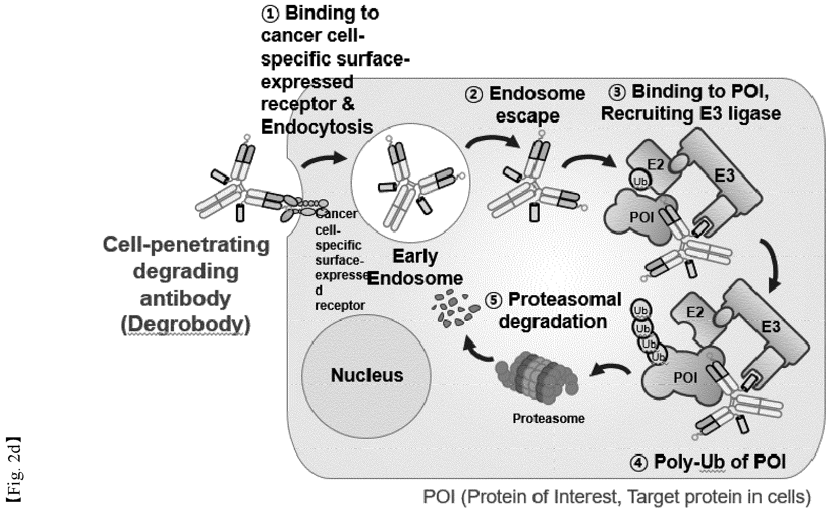

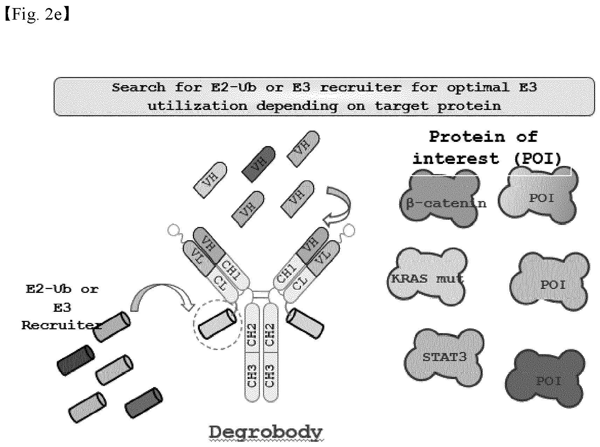

RING E3, which is used to degrade substrate proteins, is based on the mechanism by which E2-Ub and the substrate protein (substrate) spatially close to each other and the Ub of E2-Ub is directly transferred to the substrate protein without passing through E3. RING E3 includes 1) monomeric RING (e.g., c-CBL), homodimeric RING (e.g., cIAP), and heterodimeric RING (e.g., Mdm2-MdmX) as cullin-independent forms (having E3 activity only as a single protein), as shown in FIG. 1A, and 2) cullin-RING E3 ubiquitin ligases (CRLs) that act as complexes of cullin proteins and scaffolds, as shown in FIG. 1B [Morreale and Walden (2016). Cell, 165:248-248 e241]. The binding of the cullin-independent form of RING E3 to E2-Ub is formed via the RING subunit protein or the U-Box subunit protein. CRLs are subunit proteins in which RING-box proteins bind to E2-Ub (FIGS. 1A and 1B).

In humans, CRLs are the largest family of E3, and are classified into eight types, namely, CRL1 (CUL1), 2(CUL2), 3(CUL3), 4A(CUL4A), 4B(CUL4B), 5(CUL5), 7(CUL7), and 9(CUL9) depending on type of cullin (CUL), as shown in FIG. 1C. The CRL complex basically includes 1) cullin, which acts as a scaffold, 2) a RING domain to which E2-Ub binds, 3) a substrate receptor that determines substrate specificity, and 4) an adapter that links cullin to the substrate receptor. In particular, the substrate receptor includes a domain that binds to a substrate (substrate protein), which is a target protein, namely, a substrate protein-binding domain, and an adapter-binding domain that binds to an adapter protein. Therefore, the substrate receptor including two domains function to locate the substrate protein close to E3. About 300 substrate receptors present in humans are divided into representative families including 69 types of F-box, 17 types of VHL (von Hippel-Lindau), and 180 types of BTB (Bric-a-brac, Tramtrack, Broad-complex), 90 types of DCAF (DDB1-CUL4-associated factors), and 39 types of SOCS (suppressors of cytokine signaling), as shown in FIG. 1B.

The substance that degrades target proteins using UPS, an intracellular protein degradation system, is called "targeted protein degrader". Representative targeted protein degraders include proteolysis-targeting chimeras (PROTACs) and autophagy-targeting chimeras (AUTOTACs) based on small molecule compounds, and biological PROTACs (bioPROTAC) based on proteins. PROTACs are hetero-bifunctional small molecules that contain three components: an E3 binding module, a linker, and a target protein binding module. That is, a small molecule binding to a target protein, a small molecule binding to E3, and a linker molecule linking the two small molecules are synthesized into one compound. PROTACs simultaneously cause the target protein and E3 to be close to each other, thus leading to poly-ubiquitination and degradation of the target protein. AUTOTACs are small molecules that contain three components: an autophagy-targeting ligand (ATL), a linker, and a target-binding ligand (TBL), and AUTOTACs bind to the target protein through TBL, and the ATL binds to p62, which is an autophagy receptor, to induce degradation by lysosomes. AUTOTACs also have fundamental limitations of small molecules like PROTACs. In other words, AUTOTACs have limitations in undruggable targeting proteins that are difficult to target with small molecules, because they are small molecule drugs and thus have no hydrophobic binding site (pocket) capable of binding to the protein surface, and may cause side effects due to lack of tumor cell specificity. In addition, AUTOTACs have short half-life and thus poor bioavailability compared to antibody drugs. Also, five representative types of E3 used in PROTACs include β-TrCP, MDM2, cIAP/XIAP, VHL, and CRBN. Thereamong, β-TrCP and MDM2 have weak efficacy.

The protein-based targeted proteolytic agents, which have been researched to date are bioPROTACs, which are biological equivalents of PROTACs. BioPROTAC is a fusion protein constructed with a binder binding to a target protein and an E3-binding module. That is, the target protein binding module uses antibody fragments (nanobody, single chain variable fragment (scFv)), and the like, and the E3-binding module uses the substrate receptor domain of CRLs. representative bioPROTAC technologies include ubiqutibodies, antibody RING-mediated destruction (ARMeD), and affinity-directed protein missiles (AdPROM). However, bioPROTACs are disadvantageously inapplicable *in vivo,* for example, for therapeutic purposes, because they are incapable of penetrating the cytosol and thus should be delivered into cells using an artificial expression system such as DNA or mRNA transfection systems. In recent years, like antibody-PROTAC conjugates, antibody-drug conjugates (ADC) have been reported. However, antibody-PROTAC conjugates also require the development of PROTACs and thus having limitations of PROTACs associated with undruggable target proteins. In addition, antibody-PROTAC conjugates should disadvantageously solve CMC (chemistry, manufacturing, control) issues such as site-specific binding and manufacturing.

Despite intensive research into tumor biology, cancer remains a common and fatal disease. Cancer is caused and worsened by mutations in numerous proteins produced by transcription factors, receptor phosphatases, Ras proteins and the like. In particular, β-catenin, which is one of the representative transcription factors, is a cancer-causing protein that exists within cells and is difficult to target with small molecules because it has a flat surface having no deep binding pocket to which small molecules can bind. In addition, β-catenin is a protein, activity of which is difficult to inhibit with small molecule inhibitors because it interacts at a very high affinity and in a large area with transcription factor 4 (TCF4), which is a binding partner. The most representative strategy to treat such a tumor-causing protein is to use antibodies with a large surface area that can bind to antigens. Antibodies are therapeutic proteins that bind to antigens with high specificity and affinity, act as activators or inhibitors, and ultimately can treat tumors.

Antibodies for directly targeting proteins in the cytosol *in vitro* include cell/tissue-specific cytosol-penetrating interfering antibodies (iMab) (KR 10-1602870, KR 10-1602876, KR 10-1732553, KR 10-2000000, KR 10-2091195; US 10787487B2, US 10851177B2, US 20200385428A1, US 20200339681 A1, and US 20190144566A1). The cell/tissue-specific cytosol-penetrating interfering antibodies (iMab) are complete immunoglobulins that specifically bind to membrane protein receptors on the cell surface overexpressed in tumor cells or tissues, undergo endocytosis, are located in the cytosol through endosomal escape ability thereof and bind to target proteins of the cytosol. For example, iMab specifically binds to activated Ras (Ras·GTP) bound to GTP in the cytosol to inhibit the activity of tumor-derived mutant Ras (KR 10-2163305; Shin SM et al. 2020, Science Advances, 6(3): eaay2174; Shin SM et al. 2017, Nature Communications, 8:15090). However, cell/tissue-specific cytosol-penetrating interfering antibodies (iMab) that bind to the active site of the target antigens to inhibit protein-protein interactions do not exhibit sufficient effects. As a new strategy to overcome this limitation, development of cell-penetrating degrading antibodies that can degrade target proteins for higher activity based on the high specificity of antibodies is highly required. Some cell-penetrating antibodies fused with toxic molecules, enzymes, and the like have been reported to date, but antibodies that degrade cytosolic target proteins have not been reported.

### Prior art literature

### Patent literature

Korean Patent No. 10-1602870
Korean Patent No. 10-1602876
Korean Patent No. 10-1732553
Korean Patent No. 10-2000000
Korean Patent No. 10-2091195
US Patent No. 10787487 B2
US Patent No. 10851177 B2

### [Disclosure]

### [Technical Problem]

Therefore, the present invention has been made in view of the above problems, and it is one object of the present invention to provide a cell-penetrating degrading antibody (Degrobody), which is a targeted protein degrader based on a complete immunoglobulin antibody that specifically penetrates the cytosol of living target cells/tissues and specifically recognizes, degrades, and removes target proteins in the cytosol.

It is another object of the present invention to provide a cell-penetrating degrading antibody that can specifically degrade target proteins recognized by antibodies by mimicking the mechanism and function of the ubiquitin-proteasome system (UPS), which is an intracellular protein degradation system.

It is another object of the present invention to provide a cell-penetrating degrading antibody that is formed by fusing a cell/tissue-specific cytosol-penetrating interfering antibody (iMab) that penetrates the cytosol of living cells with a protein that can utilize UPS, which is an intracellular proteindegrading system, to induce poly-ubiquitination (poly-Ub) of the target protein and thereby ultimately cause degradation of target proteins.

It is another object of the present invention to explore a location at which the cell-penetrating degrading antibody is fused with a U-Box or E3 recruiter by fusing the U-Box or E3 recruiter with the N-terminus or C-terminus of the heavy chain of the antibody or the N-terminus or C-terminus of the light chain thereof, and decide an optimal location thereof.

It is another object of the present invention to provide a U-Box variant or E3 recruiter variant with improved expression, physicochemical properties, and/or biological functions, designed by fusing the cell-penetrating degrading antibody with a U-Box or E3 recruiter.

It is another object of the present invention to provide a cell-penetrating degrading antibody that is designed by fusing an anti-β-catenin cell-penetrating degrading antibody with an E3 recruiter to degrade β-catenin present in the cytosol of the target cells and thereby induce cell growth inhibition and death, a method of producing the same and the use thereof.

It is another object of the present invention to provide a method for producing a cell-penetrating degrading antibody with improved β-catenin degradation activity and cytotoxicity by optimizing the fusion site of the anti-β-catenin cell-penetrating degrading antibody with the E3 recruiter and linker.

It is another object of the present invention to provide a composition for preventing and/or treating cancer, an autoimmune disease or an infectious disease, containing the cell-penetrating degrading antibody. It is another object of the present invention to provide a method of preventing or treating cancer, an autoimmune disease, or an infectious disease, including administering the cell-penetrating degrading antibody to a patient. It is another object of the present invention to provide the use of the cell-penetrating degrading antibody for the preparation of a drug for preventing or treating cancer, an autoimmune disease, or an infectious disease.

It is another object of the present invention to provide a polynucleotide encoding the cell-penetrating degrading antibody.

### [Technical Solution]

In accordance with one aspect of the present invention, the above and other objects can be accomplished by the provision of an antibody binding to β-catenin or an antigen-binding fragment thereof including a light chain CDR1 of SEQ ID NO: 1, a light chain CDR2 of SEQ ID NO: 2, a light chain CDR3 of SEQ ID NO: 3 or 4, a heavy chain CDR1 of SEQ ID NO: 5, a heavy chain CDR2 of SEQ ID NO: 6 or 7, and a heavy chain CDR3 of SEQ ID NO: 8 or 9.

In accordance with another aspect of the present invention, provided is a cytosol-penetrating interfering antibody binding to β-catenin, wherein the cytosol-penetrating interfering antibody includes an antibody binding to β-catenin or antigen-binding fragment thereof, binds to membrane protein receptors on the cell surface overexpressed in tumor cells or tissues, undergoes endocytosis, and is located in the cytosol of cells through endosomal escape ability thereof.

In accordance with another aspect of the present invention, provided is a cell-penetrating degrading antibody (Degrobody), in which the cytosol-penetrating interfering antibody is further fused with a U-Box or E3 recruiter.

In accordance with another aspect of the present invention, provided is a nucleic acid encoding the antibody or antigen-binding fragment thereof.

In accordance with another aspect of the present invention, provided is a nucleic acid encoding the cytosol-penetrating interfering antibody.

In accordance with another aspect of the present invention, provided is a nucleic acid encoding the cell-penetrating degrading antibody.

In accordance with another aspect of the present invention, provided is an expression vector including the nucleic acid.

In accordance with another aspect of the present invention, provided is a recombinant cell transformed with the expression vector.

In accordance with another aspect of the present invention, provided is a method of producing an anti-β-catenin antibody or antigen-binding fragment thereof, including (a) culturing the cell to produce an antibody or antigen-binding fragment thereof, and (b) recovering the produced antibody or antigen-binding fragment thereof.

In accordance with another aspect of the present invention, provided is a method for producing the cell-penetrating degrading antibody including (1) cloning a nucleic acid including a heavy chain containing a heavy chain variable region (VH) capable of targeting β-catenin and a heavy chain constant region (CH1-hinge-CH2-CH3) of a human antibody, and an antibody and U-Box or E3 recruiter fused with an N-terminus or C-terminus of the heavy chain to prepare an anti-β-catenin heavy chain expression vector, (2) cloning a nucleic acid including a light chain containing a light chain variable region (VL) capable of penetrating the cytosol and a light chain constant region (CL) of a human antibody and an antibody and a U-Box or E3 recruiter fused to an N-terminus or C-terminus of the light chain to prepare an endosome-escaping light chain expression vector, (3) cotransforming the prepared heavy chain and light chain expression vectors into animal cells for protein expression to express a cell-penetrating degrading antibody in the form of a complete immunoglobulin, and (4) purifying and recovering the expressed cell-penetrating degrading antibody.

In accordance with another aspect of the present invention, provided is a conjugate including a bioactive molecule linked to the antibody or antigen-binding fragment thereof, the cytosol-penetrating interfering antibody, and the cell-penetrating degrading antibody.

In accordance with another aspect of the present invention, provided is a bispecific or multispecific antibody including the antibody or antigen-binding fragment thereof, the cytosol-penetrating interfering antibody, and the cell-penetrating degrading antibody.

In accordance with another aspect of the present invention, provided is a composition for preventing or treating cancer including the antibody or antigen-binding fragment thereof, the cytosol-penetrating interfering antibody, and the cell-penetrating degrading antibody. In accordance with another aspect of the present invention, provided is a method for preventing or treating cancer including administering to a patient the antibody or antigen-binding fragment thereof, the cytosol-penetrating interfering antibody, and the cell-penetrating degrading antibody. In accordance with another aspect of the present invention, provided is the use of the antibody or antigen-binding fragment thereof, the cytosol-penetrating interfering antibody, and the cell-penetrating degrading antibody for the preparation of a drug for preventing or treating cancer.

### [Description of Drawings]

FIG. 1A is a schematic diagram illustrating the types and structures of monomeric U-box and homodimeric U-box of E3 and the ubiquitination mechanisms.
FIG. 1B is a schematic diagram illustrating the type and structure of substrate receptors and ubiquitination mechanism depending on the type of CRLs.
FIG. 2A is a schematic diagram illustrating the development of a cell-penetrating degrading antibody (Degrobody) by fusing a cytosol-penetrating interfering antibody with a U-Box to cause the cytosol-penetrating interfering antibody to poly-ubiquitinate the target protein.
FIG. 2B is a schematic diagram illustrating a mechanism by which the cell-penetrating degrading antibody (Degrobody), designed by fusing the cytosol-penetrating interfering antibody with the U-Box, undergoes endocytosis through a receptor specifically expressed in cancer cells, and then escapes from the endosome to the cytosol to form a three-component complex of POI (protein of interest)-degrobody-E2-UB in the cytosol, and the POI is degraded by the 26S proteasome.
FIG. 2C is a schematic diagram illustrating the development of a cell-penetrating degrading antibody (Degrobody) designed by fusing an E3 recruiter with a cytosol-penetrating interfering antibody to cause the cytosol-penetrating interfering antibody to poly-ubiquitinate the target protein.
FIG. 2D is a schematic diagram illustrating a mechanism by which the cell-penetrating degrading antibody (Degrobody), designed by fusing the cytosol-penetrating interfering antibody with the E3 recruiter, undergoes endocytosis through a receptor specifically expressed in cancer cells, and then escapes from the endosome to the cytosol to form a three-component complex of POI (protein of interest) -degrobody-E3 in the cytosol, and the POI is degraded by the 26S proteasome.
FIG. 2E is a schematic diagram illustrating an E3 recruiter search strategy for optimal utilization of E3 depending on proteins of interest.
FIG. 3A is a schematic diagram illustrating an E3 recruiter search strategy depending on proteins of interest.
FIG. 3B is a structural schematic diagram illustrating the details of an adapter binding domain (E3 recruiter) in the tertiary structure of the substrate receptor.
FIG. 4 shows affinity of inBC0 and inBC2 for β-catenin compared and analyzed at β-catenin concentrations of 200, 100, 50, 25, 12.5, and 0 nM using Octet BLI-based systems to evaluate the binding ability of anti-β-catenin cell- penetrating interfering antibodies, inBC0 and inBC2 to β-catenin.
FIG. 5A is a schematic diagram illustrating the construction of an anti-β-catenin cell-penetrating degrading antibody (inBC2-E2-Ub recruiter or inBC2-E3 recruiter) designed by linking an E2-Ub recruiter or E3 recruiter to the C-terminus of the light chain of inBC2 and inBC2.
FIG. 5B shows results of 12% SDS-PAGE of the antibodies of FIG. 5A under reducing or non-reducing conditions after purification.
FIG. 5C shows results of size exclusion chromatography of the antibodies of FIG. 5A under reducing or non-reducing conditions.
FIG. 5D shows β-catenin-degradation activity of the inBC2-VHL152-213 linker variant in the colon cancer cell line HCT116 overexpressing β-catenin, evaluated using Western blot.
FIG. 5E shows the results of *in vitro* evaluation of inhibition of the antibodies of FIG. 5A against cell growth of the colon cancer cell line, HCT116.
FIG. 6A is a schematic diagram illustrating inBC2-VHL₁₅₂₋₂₁₃ and control antibodies constructed to identify the specific mechanism of action of inBC2-VHL₁₅₂₋₂₁₃.
FIG. 6B shows the results of size exclusion chromatography analysis of the antibodies of FIG. 6A.
FIG. 6C shows the binding ability of the antibodies of FIG. 6A to Cul2 and β-catenin, evaluated by immunoprecipitation.
FIG. 7A shows the β-catenin-degradation activity of inBC2-VHL₁₅₂₋₂₁₃ compared to the control antibody in three colon cancer cell lines, evaluated through Western blot.
FIG. 7B shows the β-catenin-degradation activity of inBC2-VHL₁₅₂₋₂₁₃ depending on concentration in colon cancer cell line DLD-1, evaluated through Western blot.
FIG. 7C shows the β-catenin-degradation activity over time of inBC2-VHL₁₅₂₋₂₁₃ in colon cancer cell line, DLD-1, evaluated through Western blot.
FIG. 8A shows the results of Western blot to compare proteasome-dependent β-catenin-degradation activity of inBC2-VHL₁₅₂₋₂₁₃ using a cullin-activity inhibitor and a proteasome-activity inhibitor, aaaaa
FIG. 8B shows the results of Western blot using an anti-K-48 poly-Ub antibody to analyze of β-catenin ubiquitination of inBC2-VHL₁₅₂₋₂₁₃ using a proteasome-activity inhibitor after immunoprecipitation.
FIG. 8C shows poly-ubiquitination of β-catenin using a control antibody performed in the same manner as in FIG. 8B, evaluated through Western blot.
FIG. 9 shows the results of *in vitro* test of inhibition of control antibodies and inBC2-VHL₁₅₂₋₂₁₃ against cell growth of three types of colon cancer cell lines.
FIG. 10A shows the results of a test comparing the tumor growth inhibition activities of the control antibody and inBC2-VHL₁₅₂₋₂₁₃ intravenously injected at a concentration of 20 mg/kg into mice xenografted with an integrin-overexpressing colon cancer cell line, SW480.
FIG. 10B is a graph showing the weight measured at the tumor that is treated with an integrin-specific control antibody and inBC2-VHL₁₅₂₋₂₁₃ and then extracted.
FIG. 10C is an image showing the size of the extracted tumor of FIG. 6B.
FIG. 10D is a graph showing the body weight of mice measured to confirm non-specific side effects of tumor tissue integrin-specific inBC2-VHL₁₅₂₋₂₁₃.
FIG. 10E shows the β-catenin degradation activity of inBC2-VHL₁₅₂₋₂₁₃ in the extracted tumor of FIG. 6B, evaluated through Western blot.
FIG. 11A shows the results of size exclusion chromatography analysis of inBC2, inBC2-VHL₁₅₂₋₂₁₃, and inBC2-SOCS2₁₅₃₋₁₉₈.
FIG. 11B shows the results of Western blot performed in three colon cancer cell lines to compare the β-catenin-degradation activity between inBC2-VHL₁₅₂₋₂₁₃ and inBC2-SOCS2₁₅₃₋₁₉₈
FIG. 11C shows the results of *in vitro* evaluation of the inhibition of inBC2-VHL₁₅₂₋₂₁₃ and inBC2-SOCS2₁₅₃₋₁₉₈ against cell growth of three colon cancer cell lines.
FIG. 12A shows primary structures of 12 types of inBC2-SOCS2s designed to improve the physical properties and expression amount of inBC2-SOCS2₁₅₃₋₁₉₈.
FIG. 12B shows intra- and inter-bond changes observed using BioLuminate 4.0 (Schrödinger, Inc.) after modeling the tertiary structures of variant inBC2-SOCS2s to improve the physical properties and expression levels of inBC2-SOCS2₁₅₃₋₁₉₈.
FIG. 13 shows physical properties of inBC2 and inBC2-SOCS2 antibodies analyzed through size exclusion chromatography.
FIG. 14 shows results of ELISA of 100 nM and 1 µM β-catenin and EloBC complex proteins to evaluate the binding ability of inBC2 and inBC2-SOCS2s to β-catenin and EloBC complexes.
FIG. 15A is a schematic diagram illustrating a mechanism by which a cell line, in which the β-catenin-nanoluciferase fusion reporter protein was transduced into HEK293T cells as Wntindependent cell lines, is constructed, and the luminescence of β-catenin-nanoluciferase upon treatment with inBC2-SOCS2 antibodies is analyzed in order to efficiently screen the β-catenin degradation activity of inBC2-SOCS2s.
FIG. 15B shows β-catenin degradation activity of inBC2-SOCS2s evaluated using HEK293T cell line expressing β-catenin-nanoluciferase fusion reporter protein to efficiently screen the β-catenin degradation activity of inBC2-SOCS2s.
FIG. 15C shows the β-catenin-degradation activity of inBC2-SOCS2s upon treatment of colon cancer cell line SW480 with inBC2-SOCS2s, evaluated through Western blot.
FIG. 16A shows the β-catenin-degradation activity of inBC2-SOCS2₁₅₃₋₁₉₈₋7 selected from inBC2-SOCS2 at different concentrations of 0.5, 1, 2, and 4 µM in the colon cancer cell line LoVo, evaluated through Western blot.
FIG. 16B shows the β-catenin-degradation activity depending on treatment with bortezomib, a 26S proteasome inhibitor, and MG132, in the colon cancer cell lines DLD-1 and LoVo, evaluated through Western blot, in order to evaluate the proteasome-dependent β-catenin-degradation activity of inBC2-SOCS2₁₅₃₋₁₉₈-7.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

### Antibody binding to β-catenin or antigen-binding fragment thereof

According to the present invention, specifically, in order to cause the cytosol-penetrating interfering antibody to induce poly-ubiquitination of the target protein, a U-box or E3 recruiter is fused to a cytosol-penetrating interfering antibody to design a cell-penetrating degrading antibody, as shown in FIGS. 2A and 2C, after penetration into the cytosol, the U-Box recruits E2-Ub (E2-ubiquitin conjugate) in the cytosol and localizes E2-Ub close to the target protein to induce poly-ubiquitination of the target protein, whereas the E3 recruiter recruits E3 and localizes the E3 close to the target protein to induce poly-ubiquitination of the target protein. Ultimately, the cell-penetrating degrading antibody that induces the degradation of target proteins labeled with poly-Ub by the UPS was developed (FIGS. 2A and 2C).

The present invention provides a cytosol-penetrating degrading antibody in which a U-Box or E3 recruiter is fused to an anti-β-catenin cytosol-penetrating interfering antibody. More specifically, as shown in FIGS. 2B and 2D, the anti-β-catenin cell-penetrating degrading antibody acts based on the mechanism by which 1) the anti-β-catenin cell-penetrating degrading antibody is internalized through the receptor membrane protein on the surface of cancer cells, penetrates the cytosol by endosomal escape ability, and specifically binds to β-catenin, 2) β-catenin is poly-ubiquitinated by the fused U-Box or E3 recruiter such that E2-Ub or E3 in the cytosol is located close to β-catenin, and 3) the β-catenin target protein labeled with poly-Ub is degraded by the proteasome and has anticancer activity. Based on this finding, the present invention was completed (FIGS. 2C and 2D).

The present invention aims at finding various E2-Ub recruiters (e.g., E4B, CHIP, and the like) that are capable of locating E2-Ub or E3, which causes the cell-penetrating degrading antibody to optimally degrade target proteins in the cytosol, close to the target proteins. Specifically, in the present invention, cell-penetrating degrading antibodies fused with different E3 recruiters to the same target have different target protein degradation activities depending on the E2-Ub recruiter or E3 recruiter, which suggests that it is important that the optimal E3 recruiter depending on the target protein be searched and utilized (FIG. 2E).

The present invention is directed to an antibody binding to β-catenin or an antigen-binding fragment thereof including a light chain CDR1 of SEQ ID NO: 1, a light chain CDR2 of SEQ ID NO: 2, a light chain CDR3 of SEQ ID NO: 3 or 4, a heavy chain CDR1 of SEQ ID NO: 5, a heavy chain CDR2 of SEQ ID NO: 6 or 7, and a heavy chain CDR3 of SEQ ID NO: 8 or 9.

As used herein, the term "antibody" refers to an anti-β-catenin antibody that specifically binds to β-catenin. A complete antibody and antigen-binding fragment of the antibody molecules falls within the scope of the present invention.

The whole antibody has a structure having two full-length light-chains and two full-length heavy-chains, and each light-chain is bonded to the heavy-chain by a disulfide bond.

As used herein, the term "heavy-chain" encompasses both a full-length heavy-chain, which includes a variable domain (VH) containing an amino acid sequence having a sufficient variable region sequence for imparting specificity to an antigen and three constant domains (CH1, CH2 and CH3), and a fragment thereof. As used herein, the term "light-chain" encompasses both a full-length light-chain, which includes a variable domain (VL) containing an amino acid sequence having a sufficient variable region sequence for imparting specificity to an antigen and a constant domain (CL), and a fragment thereof.

The whole antibody includes subtypes of IgA, IgD, IgE, IgM and IgG, and in particular, IgG includes IgG1, IgG2, IgG3 and IgG4. The heavy-chain constant region has gamma (γ), mu (µ), alpha (α), delta (δ) and epsilon (ε) types, and is subclassified into gamma 1 (γ1), gamma 2 (γ2), gamma 3 (γ3), gamma 4 (γ4), alpha 1 (α1), and alpha 2 (α2). The light-chain constant region has kappa (κ) and lambda (λ) types.

The antigen-binding fragment of an antibody or antibody fragment refers to a fragment that has antigen-binding function and includes Fab, F(ab'), F(ab')2, Fv and the like. Among the antibody fragments, Fab refers to a structure including a variable region of each of the heavy-chain and the light-chain, the constant region of the light-chain, and the first constant domain (CH1) of the heavy-chain, each having one antigen-binding site. Fab' is different from Fab in that it further includes a hinge region including at least one cysteine residue at the C-terminus of the CH1 domain of the heavy-chain. F(ab')2 is created by a disulfide bond between cysteine residues in the hinge region of Fab'.

Fv is the minimal antibody fragment having only a heavy-chain variable region and a light-chain variable region. Two-chain Fv is a fragment in which the variable region of the heavy-chain and the variable region of the light-chain are linked by a non-covalent bond, and single-chain Fv (scFv) is a fragment in which the variable region of the heavy-chain and the variable region of the light-chain are generally linked by a covalent bond via a peptide linker therebetween, or are directly linked at the C-terminal, forming a dimer-shaped structure, like the two-chain Fv. Such antibody fragments may be obtained using proteases (e.g., Fab can be obtained by restriction-cleaving the complete antibody with papain, and the F(ab')2 fragment may be obtained by restriction-cleaving the complete antibody with pepsin), and may be produced using genetic recombination techniques.

An "Fv" fragment is an antibody fragment containing complete antibody recognition and binding sites. Such a region includes a dimer in which one heavy-chain variable domain is linked to one light-chain variable domain.

A "Fab" fragment contains a variable domain and a constant domain of the light-chain and a variable domain and a first constant domain (CH1) of the heavy-chain. A F(ab')2 antibody fragment generally includes a pair of Fab fragments covalently linked near the carboxyl terminal thereof via a hinge cysteine therebetween.

The "single chain Fv" or "scFv" antibody fragment includes VH and VL domains of the antibody, wherein these domains are present in a single polypeptide chain. The Fv polypeptide may further include a polypeptide linker between the VH domain and the VL domain in order for the scFv to form a target structure for antigen binding.

In an embodiment, the antibody according to the present invention includes, but is not limited to, monoclonal antibodies, multispecific antibodies, human antibodies, humanized antibodies, chimeric antibodies, scFVs, Fab fragments, F(ab') fragments, disulfide-bond Fvs (sdFVs), anti-idiotypic (anti-Id) antibodies, epitope-binding fragments of such antibodies, and the like.

The heavy-chain constant region may be selected from gamma (γ), mu (u), alpha (α), delta (δ) and epsilon (c) isotypes. For example, the constant region may be gamma 1 (IgG1), gamma 3 (IgG3), or gamma 4 (IgG4). The light-chain constant region may be kappa or lambda.

The term "monoclonal antibody" refers to an identical antibody, which is obtained from a population of substantially homogeneous antibodies, that is, each antibody constituting the population, excluding possible naturally occurring mutations that may be present in trivial amounts. Monoclonal antibodies are highly specific and are thus induced against a single antigenic site. Unlike conventional (polyclonal) antibody preparations that typically include different antibodies directed against different determinants (epitopes), each monoclonal antibody is directed against a single determinant on the antigen.

For example, monoclonal antibodies useful in the present invention may be produced by hybridoma methods, or may be produced in bacterial, eukaryotic or plant cells using recombinant DNA methods. In addition, monoclonal antibodies may be isolated from phage antibody libraries.

The term "affinity" refers to the ability to specifically recognize a specific site of an antigen and to bind thereto, and specificity and high-affinity of an antibody for an antigen are important factors in the immune response. The affinity constant (K_{D}) can be determined using surface plasmon resonance (SPR), for example, a BIAcore system. An affinity constant (K_{D}) calculated from a 1:1 Langmuir binding model (simultaneously kₒₙ and k_{off}) and the ratio of the rate constant k_{off}/kₒₙ was obtained based on surface plasmon resonance data.

The binding affinity of the anti-β-catenin antibody to β-catenin ranges from 10⁻⁵ M to 10⁻¹² M. For example, the binding affinity is 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M or 10⁻⁵ M to 10⁻⁶ M.

As used herein, the term "antibody variable region" refers to the light- and heavy-chain regions of an antibody molecule including the amino acid sequences of a complementarity-determining region (CDR; i.e., CDR1, CDR2, and CDR3) and a framework region (FR). VH refers to a variable domain of the heavy-chain. VL refers to a variable domain of the light-chain.

The term "complementarity-determining region" (CDR; i.e., CDR1, CDR2, and CDR3) refers to an amino acid residue of the antibody variable domain that is necessary for antigen binding. Each variable domain typically has three CDR regions, identified as CDR1, CDR2, and CDR3.
a light chain CDR1 of SEQ ID NO: 1, a light chain CDR2 of SEQ ID NO: 2, a light chain CDR3 of SEQ ID NO: 3, a heavy chain CDR1 of SEQ ID NO: 5, a heavy chain CDR2 of SEQ ID NO: 6, and a heavy chain CDR3 of SEQ ID NO: 8;
a light chain CDR1 of SEQ ID NO: 1, a light chain CDR2 of SEQ ID NO: 2, a light chain CDR3 of SEQ ID NO: 3, a heavy chain CDR1 of SEQ ID NO: 5, a heavy chain CDR2 of SEQ ID NO: 7, and a heavy chain CDR3 of SEQ ID NO: 9; or
a light chain CDR1 of SEQ ID NO: 1, a light chain CDR2 of SEQ ID NO: 2, a light chain CDR3 of SEQ ID NO: 4, a heavy chain CDR1 of SEQ ID NO: 5, a heavy chain CDR2 of SEQ ID NO: 6, and a heavy chain CDR3 of SEQ ID NO: 8.

The term "framework region" (FR) refers to a variable domain residue other than a CDR residue. Each variable domain typically has four FRs, identified as FR1, FR2, FR3, and FR4.

The antibody or antigen-binding fragment thereof according to the present invention may include a heavy chain variable region selected from the group consisting of SEQ ID NOs: 17 to 19 and/or a light chain variable region of SEQ ID NO: 20 or 21.

In a specific embodiment according to the present invention, the antibody or antigen-binding fragment thereof binding to β-catenin may include the following:
a heavy-chain variable region of SEQ ID NO: 17 and a light-chain variable region of SEQ ID NO: 20;
a heavy-chain variable region of SEQ ID NO: 18 and a light-chain variable region of SEQ ID NO: 20;
a heavy-chain variable region of SEQ ID NO: 19 and a light-chain variable region of SEQ ID NO: 21.

The anti-β-catenin antibody or antigen-binding fragment thereof according to the present invention also includes an antibody or antigen-binding fragment thereof in which a part of the amino acid sequence is substituted through conservative substitution.

As used herein, the term "conservative substitution" refers to modifications of polypeptides that involve the substitution of one or more amino acids with other amino acids having similar biochemical properties that do not result in loss of the biological or biochemical function of the polypeptides. The term "conservative amino acid substitution" refers to substitution of the amino acid residue with an amino acid residue having a similar side chain. Families of amino acid residues having similar side chains have been defined and are well known in the art to which the present invention pertains. These families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine, and cysteine), amino acids with nonpolar side chains (e.g., alanine, valine, leucine, isoleucine, proline, phenylalanine, methionine, and tryptophan), amino acids with beta-branched side chains (e.g., threonine, valine, and isoleucine), and amino acids with aromatic side chains (e.g., tyrosine, phenylalanine, tryptophan, and histidine). It was found that the antibody according to the present invention retains the activity thereof despite having conservative amino acid substitutions.

The antibody or antibody-binding fragment thereof according to the present invention may include not only an antibody but also biological equivalents thereto, as long as it can specifically recognize an antigen protein. For example, additional variations can be made to the amino acid sequence of the antibody in order to further improve the binding affinity and/or other biological properties of the antibody. Such variations include, for example, deletion, insertion and/or substitution of the amino acid sequence residues of the antibody. Such amino acid mutations are based on the relative similarity of amino-acid side-chain substituents, such as the hydrophobicity, hydrophilicity, charge and size thereof. It can be seen through analysis of the size, shape and type of amino-acid side-chain substituents that all of arginine, lysine and histidine are positively charged residues; alanine, glycine and serine have similar sizes; and phenylalanine, tryptophan and tyrosine have similar shapes. Thus, based on these considerations, arginine, lysine and histidine; alanine, glycine and serine; and phenylalanine, tryptophan and tyrosine are considered to be biologically functional equivalents.

When taking into consideration mutations having biologically equivalent activity, the antibody or a nucleotide molecule encoding the same according to the present invention is interpreted to include a sequence having substantial identity with the sequence set forth in the sequence number. The term "substantial identity" means that a sequence has a homology of at least 90%, preferably a homology of at least 90%, most preferably at least 95%, at least 96%, at least 97%, at least 98%, and at least 99%, when aligning the sequence of the present invention and any other sequence so as to correspond to each other as much as possible and analyzing the aligned sequence using algorithms commonly used in the art. Alignment methods for sequence comparison are well-known in the art. The NCBI Basic Local Alignment Search Tool (BLAST) is accessible through NCBI or the like, and can be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast help.html.

Based on this, the antibody or antigen-binding fragment thereof according to the present invention can have a homology of 90%, 91%, 92%, 93%, 94%, 95%, 96%, 97%, 98%, 99%, or more compared to the sequence disclosed herein or the entirety thereof. Homology can be determined through sequence comparison and/or alignment by methods known in the art. For example, the percentage sequence homology of the nucleic acid or protein according to the present invention can be determined using a sequence comparison algorithm (i.e., BLAST or BLAST 2.0), manual alignment, or visual inspection.

The term "nucleic acid" is intended to encompass both DNA (gDNA and cDNA) and RNA molecules, and a nucleotide, which is a basic constituent unit of a nucleic acid, includes naturally derived nucleotides as well as analogues, wherein sugar or base moieties are modified. The sequence of the nucleic acid encoding heavy- and light-chain variable regions of the present invention can vary. Such variation includes addition, deletion, or nonconservative or conservative substitution of nucleotides.

The DNA encoding the antibody can be easily separated or synthesized using conventional molecular biological techniques (for example, using an oligonucleotide probe capable of specifically binding to DNA encoding heavy and light-chains of the antibody). Nucleic acids are isolated and inserted into replicable vectors for further cloning (amplification of DNA) or further expression. Based on this, in another aspect, the present invention is directed to a recombinant expression vector including the nucleic acid.

As used herein, the term "vector" refers to a means for expressing target genes in host cells, and includes plasmid vectors, cosmid vectors, and viral vectors such as bacteriophage vectors, adenovirus vectors, retroviral vectors and adenoassociated viral vectors. Vector components generally include, but are not limited to, one or more of the following components: signal sequences, replication origins, one or more antibiotic resistance marker genes, enhancer elements, promoters, and transcription termination sequences. The nucleic acid encoding the antibody is operably linked to promoters, transcription termination sequences or the like.

The term "operably linked" means a functional linkage between a nucleic acid expression regulation sequence (e.g., an array of promoter, signal sequence or transcription regulator binding sites) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, a lac promoter, a lacUV5 promoter, an lpp promoter, a pLλ promoter, a pRλ promoter, a rac5 promoter, an amp promoter, a recA promoter, SP6 promoter, a trp promoter, or a T7 promoter), a ribosome-binding site for initiation of translation, and a transcription/translation termination sequence. In addition, for example, when a eukaryotic cell is used as a host, it includes a promoter (e.g., a metallothionein promoter, a β-actin promoter, a human hemoglobin promoter and a human muscle creatine promoter) derived from the genome of mammalian cells, or a promoter derived from a mammalian virus such as an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter, and generally has a polyadenylation sequence as a transcription termination sequence.

Optionally, the vector may be fused with another sequence in order to facilitate purification of the antibody expressed therefrom. The sequence to be fused therewith includes, for example, glutathione S-transferase (Pharmacia, USA), maltosebinding protein (NEB, USA), FLAG (IBI, USA), 6x His (hexahistidine; Qiagen, USA) and the like.

The vector includes antibiotic-resistance genes commonly used in the art as selectable markers, and examples thereof include genes conferring resistance to ampicillin, gentamycin, carbenicillin, chloramphenicol, streptomycin, kanamycin, geneticin, neomycin and tetracycline.

In another aspect, the present invention is directed to a host cell transfected with the recombinant expression vector. The host cell used to produce the antibody of the present invention may be a prokaryote, yeast or higher eukaryotic cell, but is not limited thereto.

Prokaryotic host cells such as *Escherichia coli,* the genus *Bacillus,* such as *Bacillus subtilis* and *Bacillus thuringiensis, Streptomyces* spp., *Pseudomonas* spp. (for example, *Pseudomonas putida*), *Proteus mirabilis* and *Staphylococcus* spp. (for example, *Staphylococcus carnosus*) can be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing an antibody or an antigen-binding fragment thereof including culturing the host cells to produce an antibody, and isolating the produced antibody from the cultured cells, followed by purification.

The host cells can be cultured in various media. Any commercially available medium can be used as a culture medium without limitation. All other essential supplements well-known to those skilled in the art may be included in appropriate concentrations. Culture conditions such as temperature and pH are those that are conventionally used with the host cells selected for expression, which will be apparent to those skilled in the art.

The recovery of the antibody or antigen-binding fragment thereof can be carried out, for example, by centrifugation or ultrafiltration to remove impurities and further purification of the resulting product using, for example, affinity chromatography. Other additional purification techniques such as anion or cation exchange chromatography, hydrophobic interaction chromatography and hydroxyapatite (HA) chromatography may be used.

In another aspect, the present invention is directed to a conjugate in which the antibody or antigen-binding fragment thereof is fused with a bioactive molecule selected from the group consisting of peptides, proteins, small-molecule drugs, nucleic acids, nanoparticles and liposomes.

The proteins include antibodies, fragments of antibodies, immunoglobulins, peptides, enzymes, growth factors, cytokines, transcription factors, toxins, antigenic peptides, hormones, transport proteins, motor function proteins, receptors, signaling proteins, storage proteins, membrane proteins, transmembrane proteins, internal proteins, external proteins, secreted proteins, viral proteins, sugar proteins, truncated proteins, protein complexes, chemically modified proteins and the like.

The term "small-molecule drugs" refers to an organic compound, an inorganic compound or an organometallic compound that has a molecular weight of less than about 1,000 Da and has activity as a therapeutic agent for diseases, which is widely used herein. The small-molecule drug used herein includes oligopeptides and other biomolecules having a molecular weight of less than about 1,000 Da.

As used herein, the term "nanoparticle" refers to a particle including a material having a diameter of 1 to 1,000 nm, and the nanoparticle may be a metal/metal core-shell complex including a metal nanoparticle, a metal nanoparticle core and a metal shell including the core, a metal/non-metal core-shell complex including a metal nanoparticle core and a non-metal shell surrounding the core, or a nonmetal/metal core-shell complex including a nonmetal nanoparticle core and a metal shell surrounding the core. According to one embodiment, the metal may be selected from gold, silver, copper, aluminum, nickel, palladium, platinum, iron, and oxides thereof, but is not limited thereto, and the nonmetal may be selected from silica, polystyrene, latex and acrylic substances, but is not limited thereto.

The liposome consists of one or more lipid bilayer membranes surrounding an aqueous internal compartment that can self-associate. Liposomes can be specified based on the type and size of the membrane thereof. Small unilamellar vesicles (SUVs) have a single membrane, and may have a diameter of 20 nm to 50 nm. Large unilamellar vesicles (LUV) may have a diameter of 50 nm or more. Oligolamellar large vesicles and multilamellar large vesicles have multiple, generally concentric, membrane layers, and may be 100 nm or more in diameter. Liposomes having a plurality of non-concentric membranes, that is, several small vesicles contained within larger vesicles, are called "multivesicular vesicles".

As used herein, the term "fusion" refers to the integration of two molecules having different or identical functions or structures, and includes fusion through any physical, chemical or biological method capable of binding the antibody or antigen-binding fragment thereof to the protein, small-molecule drug, nanoparticle, or liposome. The fusion may preferably be carried out using a linker peptide, and the linker peptide may mediate fusion with the bioactive molecule at various positions of the antibody light-chain variable region, antibody, or fragment thereof according to the present invention.

As used herein, the term "effector function" refers to the type of biological activity associated with the Fc region of an antibody (wild-type sequence of the Fc region or a variant of the amino acid sequence of the Fc region) and depends on the isotype of the antibody. Examples of antibody effector functions include C1q binding, complement dependent cytotoxicity (CDC); Fc receptor binding, antibody dependent cell-mediated cytotoxicity (ADCC), phagocytosis, downregulation of cell surface receptors (e.g., B-cell receptor, BCR) and B-cell activation.

The term "antibody-dependent cellular cytotoxicity" or "ADCC" refers to a reaction in which effector cells (e.g., T-cells and NK-cells) lyse target cells labeled by a specific antibody. ADCC is also independent of the immune complement system, which lyses the target, but does not require other cells. ADCC typically requires effector cells known to be natural killer (NK) cells that interact with immunoglobulin G (IgG) antibodies. However, macrophages, neutrophils and eosinophils can also mediate ADCC, for example, eosinophils that kill certain parasitic worms known as parasites via IgE antibodies.

The bioactive molecule that can be conjugated to the antibody according to the present invention may include a small molecule drug.

In another aspect, the present invention is directed to a bispecific antibody or multispecific antibody including the antibody or antigen-binding fragment thereof.

As used herein, the term "bispecific antibody" refers to a protein capable of binding to two different types of antigens (target proteins). Specifically, the bispecific antibody does not exist naturally and is preferably prepared by genetic engineering or any method. The term "bispecific antibody" of the present invention may be used interchangeably with "bitarget antibody", "biantibody" or "biantibody protein".

The bispecific antibody refers to a molecule that has an antigen-binding site linking directly or via a linker or forms a heterodimer through electrostatic interaction.

The term "valent" means the presence of a specified number of binding sites specific to an antigen in the molecule. As such, the terms "monovalent", "bivalent", "tetravalent", and "hexavalent" refer to the presence of each of 1, 2, 4, and 6 binding sites specific for an antigen in a molecule.

The term "multispecific antibody" refers to an antibody that has binding specificity for at least three different antigens. The multispecific antibody includes a tri- or higher antibody, for example, a trispecific antibody, a tetraspecific antibody, or an antibody that targets more targets.

### Cytosol-penetrating interfering antibody and cell-penetrating degrading antibody

The present invention is directed to a cell-penetrating degrading antibody that is produced by fusing a cytosol-penetrating interfering antibody that penetrates into the cytosol of living cells or tissues and specifically recognizes target proteins with U-Box that can bind to E2-Ub (E2-ubiquitin conjugate) or an E3 recruiter (E3 recruiting ligand) that can bind to E3 (E3 ubiquitin ligase), wherein the intracellular target proteins are degraded by the ubiquitin-proteasome system (UPS).

As used herein, the term "cell/tissue-specific cytosol-penetrating interfering antibody (iMab)" refers to an antibody that binds, in the form of an immunoglobulin, preferably a complete immunoglobulin, to a membrane protein receptor overexpressed on the surface of a target cell such as a tumor tissue, is internalized (undergoes endocytosis), and then is located in the cytosol due to endosomal escape ability thereof, and specifically binds to the target protein within the cytosol.

As used herein, the term "ubiquitin-proteasome system (UPS)" refers to a system that degrades and removes cellular proteins by poly-ubiquitination (poly-Ub), and includes E1 (ubiquitin-activating enzyme), E2 (ubiquitin-conjugating enzyme), and E3 (ubiquitin ligase enzyme). E1 activates ubiquitin to form an E2-ubiquitin conjugate (E2-Ub), E3 binds to the E2-Ub to induce poly-ubiquitination of the substrate and thereby degrade the poly-ubiquitinated substrate by the proteasome.

### Target proteins difficult to target with small molecules

Proteins that are located inside cells and do not have a hydrophobic pocket to which small molecules can bind are undruggable targets that are difficult to target with small molecules and cause various diseases such as cancer and immune diseases due to numerous mutations. β-catenin, which is one of the representative disease-causing proteins, is a protein encoded by the CTNNB1 gene and acts as a transcription factor in the Wnt signaling pathway. Wnt protein, the first Wnt signaling substance, binds to a receptor called "Frizzled" on the cell surface and initiates signaling. When β-catenin in the cytosol is not stimulated by the Wnt protein, it is phosphorylated by an enzyme called "GSK-3" (glycogen synthase kinase-3) within the Axin and APC (adenomatous polyposis coli) protein complex. The phosphorylated β-catenin is recognized by β-TrCP E3, which belongs to cullin-RING E3 ubiquitin ligases (CRLs), and is degraded by the proteasome through the UPS, resulting in a low amount of β-catenin in the cytosol. When Wnt binds to Frizzled, GSK-3 in the cell is inactivated. The inactivated GSK-3 cannot phosphorylate β-catenin and thus consequently cannot degrade β-catenin, leading to the accumulation of β-catenin in the cytosol. The accumulated β-catenin moves into the nucleus and binds to a transcription factor called "TCF4" (transcription factor 4), thereby promoting transcription of Wnt target genes such as cyclin D1, myc, and matrix metalloproteinase-7 (MMP-7).

Increased transcriptional activity of β-catenin due to abnormal activation of Wnt signaling is associated with the development of cancer. This is why ① when a mutation occurs in the gene encoding Axin and/or APC protein in the Axin and APC protein complexes, which is involved in β-catenin degradation, β-catenin, which is not phosphorylated by GSK-3 and is thus not recognized by β-TrCP, is accumulated in the cytosol, ② as a mutation occurs in the gene encoding β-catenin, phosphorylation by GSK-3 does not occur, leading to the accumulation of β-catenin in the cytosol and, as a result, continuously promoting transcription of Wnt target genes and Wnt signaling.

### Anti-β-catenin cytosol-penetrating interfering antibody

The present invention is directed to a cytosol-penetrating interfering antibody binding to β-catenin, wherein the cytosol-penetrating interfering antibody includes an antibody binding to β-catenin or an antigen-binding fragments thereof, and a substance targeting a tumor cell- or tissue-specific antigen, and the cytosol-penetrating interfering antibody binds to the antigen, undergoes endocytosis, and is located in the cytosol of cells through endosomal escape ability thereof.

The anti-β-catenin cytosol-penetrating interfering antibody refers to an antibody that specifically binds to membrane protein receptors on the cell surface overexpressed in tumor cells or tissues, undergoes endocytosis, is located in the cytosol of cells through endosomal escape ability thereof and binds to β-catenin, which is a target protein of cytosol to inhibit functions thereof.

The anti-β-catenin cytosol-penetrating interfering antibody may specifically recognize at least one selected from EpCAMs (epithelial cellular adhesion molecules) and EGFRs (epidermal growth factor receptors) and integrins αvβ3/αvβ5 overexpressed in tumor cell or tissue-specific antigens, for example, tumor cells.

The tumor cell line may overexpress one or more of EpCAM, EGFR, and integrins αvβ3/αvβ5.

The substance targeting the tumor cell- or tissue-specific antigen is, for example, a ligand, oligopeptide, antibody, fragment or aptamer capable of specifically binding to the antigen, but is not limited thereto.

Hereinafter, the present invention will be described in more detail.

As used herein, the term "cytosol-penetrating interfering antibody" includes an antibody light chain variable region (VL-CDR3) described in the prior patent (Korean Patent No. 10-2000000) corresponding to SEQ ID NO. 3 shown in Table 1, and the VL-CDR3 includes an endosomal escape structure motif. The sequence of VL-CDR3 changes the characteristics of the antibody under acidic endosomal pH conditions and allows the antibody to escape from the endosome to the cytosol.

As used herein, the term "anti-β-catenin cytosol-penetrating interfering antibody" includes an antibody heavy chain variable region (VH-CDR3) corresponding to SEQ ID NO: 8, and the VH-CDR3 includes a sequence recognizing β-catenin as an antigen.

In addition, the cytosol-penetrating interfering antibody according to the present invention is characterized by fusion of a cyclic peptide targeting a receptor overexpressed in tumor tissue for tumor tissue-specific cytosolic penetration, and the circular peptide fused to the cytosol-penetrating interfering antibody may be circular peptide in4 (DGVRQCRDGCFDGPL) targeting integrin αvβ3/αvβ5 (Shin, et al., (2020), Sci Adv, 6, eaay2174) .

The circular peptide may be bound directly to the N- or C-terminus of the antibody or bound thereto via a linker. The linker may be a peptide with various sequences and lengths, and may be a GGGGS, (GGGGS)2, ASTKGP, and ASTKGPSVFPLAP sequence linker that provides structural flexibility without being cleaved by proteases.

Specifically, the cytosol-penetrating interfering antibody may have an antigen-binding site shown in Table 1 below.

**[Table 1]**

| | Light chain CDRs | | | Heavy chain CDRs | | |
|---|---|---|---|---|---|---|
| β-catenin antibody | VL-CDR1 | VL-CDR2 | VL-CDR3 | VH-CDR1 | VH-CDR2 | VH-CDR3 |
| inBC0 | KSSQSLLNSRDGKNYLA (SEQ ID 1) | WASTRES (SEQ ID 2) | QQYWYWMFT (SEQ ID 3) | SYPMH (SEQ ID 5) | WSYDGSYKYYADSVKG (SEQ ID 6) | ATVRGVTKIRGVFDS (SEQ ID 8) |
| inBC2 | KSSQSLLNSRDGKNYLA | WASTRES | QQYWYWMFT | SYPMH | WSYDGSYKYYADSVKG | ATVRGVTKIRGVFDS |
| inCT | KSSQSLLNSRDGKNYLA | WASTRES | QQYWYWMFT | SYVMH | AlNPYNDGNYYADSVKG (SEQ ID 7) | GAYKRGYAMDY (SEQ ID 9) |
| inBC2-(AAA) | KSSQSLLNSRDGKNYLA | WASTRES | QQYAAAMFT (SEQ ID 4) | SYPMH | WSYDGSYKYYADSVKG | ATVRGVTKIRGVFDS |

### U-Box

"U-Box" according to the present invention refers to a U-Box in the monomeric U-box or homodimeric U-Box among RING E3s and has the function of binding to E2-Ub. Specifically, the monomeric U-box includes E4B (E4 poly-ubiquitin chain elongation factor B), and the homodimeric U-box includes U-box such as CHIP (carboxyl terminus of Hsp70-interacting protein) and Prp19 (precursor-mRNA splicing factor 19). In other words, U-Box can bind to E2-Ub and label the substrate protein bound to E3 of monomeric U-box or homodimeric U-Box with poly-Ub.

E4B, which belongs to the monomeric U-box, binds to E2-Ub through the catalytic domain called "U-box domain" located at the C-terminus and functions to mediate poly-ubiquitination (poly-Ub) of the target protein, in which the target protein is labeled in chain and multiple.

CHIP belonging to the homodimeric U-box functions to induce an asymmetric homodimer through the helical domain, bind to a specific E2-Ub through the U-box domain at the C-terminus, and mediate poly-ubiquitination of the substrate protein.

The sequences of the E2-Ub and E3 recruiter are shown in Table 2 below, and the amino acid sequence partially fragmented from the U-box domain that can bind to E2-Ub in the E4B may be represented by SEQ ID NO: 15 shown in Table 2 below.

**[Table 2] Amino acid sequences of E2-Ub and E3 recruiter**

| **E2-Ub recruiter** | **Amino acid sequence** | **SEQ ID** |
|---|---|---|
| E4B₁₂₂₇₋₁₃₀₂ (E4 poly-ubiquitin chain elongation factor B) | DAPDEFRDPLMDTLMTDPVRLPSGTIMDRSIILRHLLNSPTDPFNRQTLTESMLEPVPE LKEQIQAWMREKQNSDH | 10 |
| IpaH₂₅₄₋₅₄₅ (Invasion Plasmid Antigen H) | LADAVTAWFPENKQSDVSQIWHAFEHEEHANTFSAFLDRLSDTVSARNTSGFREQVAA WLEKLSASAELRQQSFAVAADATESCEDRVALTWNNLRKTLLVHQASEGLFDNDTGAL LSLGREMFRLEILEDIARDKVRTLHFVDEIEVYLAFQTMLAEKLQLSTAVKEMRFYGVSG VTANDLRTAEAMVRSREENEFTDWFSLWGPWHAVLKRTEADRWAQAEEQKYEMLEN EYPQRVADRLKASGLSGDADAEREAGAQVMRETEQQIYRQLTDEVLALRLPENGSQL HHS | 11 |

| **E3 recruiter** | **Amino acid sequence** | **SEQ ID** |
|---|---|---|
| VHL₁₅₂₋₂₁₃ (Von Hippel-Lindau tumor suppressor) | TLPVYTLKERCLQVVRSLVKPENYRRLDIVRSLYEDLEDHPNVQKDLERLTQERIAHQR MGD | 12 |
| βTrCP₁₄₂₋₂₅₅ (β-transducin repeat-containing protein) | RDFITALPARGLDHIAENILSYLDAKSLCAAELVCKEWYRVTSDGMLWKKLIEKMVRTDS LWRGLAERRGWGQYLFKNKPPDGNAPPNSFYRALYPKIIQDIETIESNWKCGRH | 13 |
| SPOP₁₆₇₋₃₇₄ (Speckle Type BTB/POZ Protein) | SVNISGQNTMNMVKVPECRLADELGGLWENSRFTDCCLCVAGQEFQAHKAILAARSP VFSAMFEHEMEESKKNRVEINDVEPEVFKEMMCFIYTGKAPNLDKMADDLLAAADKYA LERLKVMCEDALCSNLSVENAAEILILADLHSADQLKTQAVDFINYHASDVLETSGWKS MVVSHPHLVAEAYRSLASAQCPFLGPPRKRLKQS | 14 |
| DDB2₆₁₋₁₁₄ (Damage Specific DNA Binding Protein 2) | QILPPCRSIVRTLHQHKLGRASWPSVQQGLQQSFLHTLDSYRILQKAAPFDRRA | 15 |
| SOCS2₁₅₃₋₁₉₈ (Suppressor Of Cytokine Signaling 2) | TKPLYTSAPSLQHLCRLTINKCTGAIWGLPLPTRLKDYLEEYKFQV | 16 |

### E3 recruiter

"E3 recruiter" according to the present invention refers to an adapter-binding domain in substrate receptors of RING E3s, especially CRLs. In other words, the substrate receptor includes two domains, namely, a substrate protein-binding domain and an adapter-binding domain, and acts to bind the substrate protein to E3 such that E3 is closed to the substrate protein. In CRLs, the RING-Box protein, which is another protein subunit, can bind to E2-Ub to induce poly-ubiquitination of the substrate protein bound to CRLs. In the present invention, the adapter-binding domain excluding the substrate protein-binding domain from substrate receptors is defined as "E3 recruiter."

In this case, the substrate recognition domain may be replaced with a cytosol-penetrating interfering antibody as a complete immunoglobulin having target protein-binding ability. Therefore, after fusing the cytosol-penetrating interfering antibody having antigen-targeting ability with an E3 recruiter and delivering the fusion result to the cytosol, various desired proteins present in the cytosol can be labeled with poly-Ub and degraded by the proteasome to induce cytotoxicity.

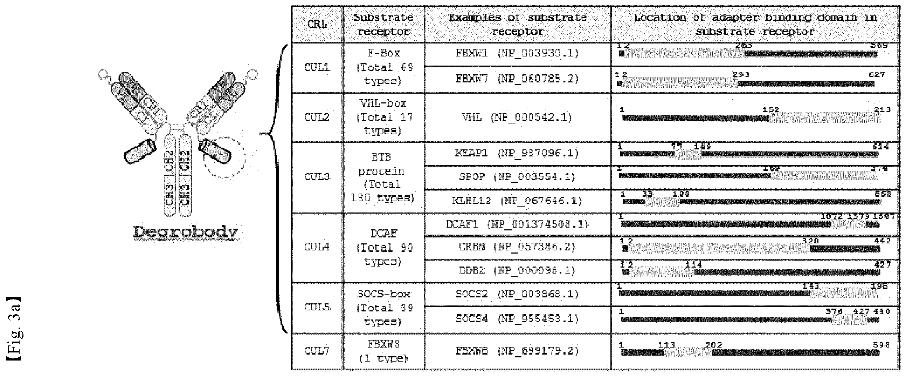

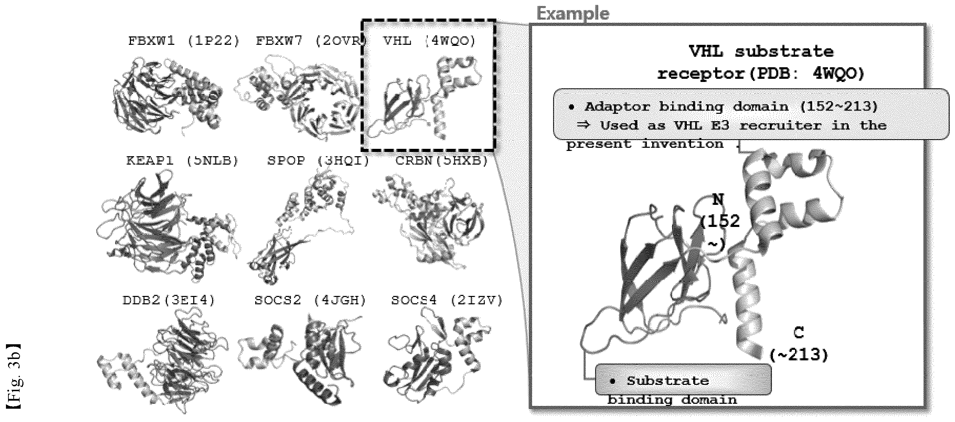

As shown in the schematic diagram of the E3 recruiter search strategy depending on the target protein in FIG. 3A, the E3 recruiter substrate receptors for respective CRLs include 69 types of F-box, 17 types of VHL, 180 types of BTB, 90 types of DCAF, 39 types of SOCS, and the like. In an attempt to use only the adapter binding domain as an E3 recruiter, an E3 recruiter optimal for each target protein is fused with a Degrobody based on structural analysis and rational design of the adapter binding domain to construct the Degrobody. FIG. 3B illustrates the details of the adapter binding domain (E3 recruiter) part of the tertiary structure of the substrate receptor.

The present inventor attempted to complete a cell-penetrating degrading antibody by binding the fragmented E3 recruiter selected as shown in FIG. 3A to a cytosol-penetrating interfering antibody, wherein the fragmented E3 recruiter may be selected from βTrCP₁₄₂₋₂₅₅, VHL₁₅₂₋₂₁₃, SPOP₁₆₇₋₃₇₄, DDB₂₆₁₋₁₁₄, and SOCS2₁₅₃₋₁₉₈, which are E3 recruiters belonging to CRL1 to CRL5. The present invention provides fragmented amino acid sequences of the adapter-binding domain belonging to the CRLs and defined as "E3 recruiters" (see Tables 2 and 9). Specifically, the E3 recruiter is a SOCS2₁₅₃₋₁₉₈ variant with improved expression level and may be at least one selected from the group consisting of SEQ ID NOs: 12 to 16 and 23 to 34.

### Linker peptide

In the present invention, the U-Box or E3 recruiter is fused with the N-terminus or C-terminus of the heavy chain and the N-terminus or C-terminus of the light chain of the cytosol-penetrating interfering antibody via an uncleaved linker.

As used herein, the term "fusion" refers to the integration of two molecules having different or identical functions or structures, and includes fusion through any physical, chemical or biological method capable of binding the tumorspecific cytosol-penetrating peptide to the protein, small-molecule drug, nanoparticle or liposome. Fusion is preferably carried out using a linker peptide, and the linker peptide may mediate the fusion with the bioactive molecule at various positions of the antibody domain, antibody, or fragment thereof according to the present invention.

As used herein, the term "linker peptide" refers to a peptide used to genetically fuse a cytosol-penetrating interfering antibody with a U-Box or E3 recruiter. The linker peptide may have various sequences and lengths, may have different structural flexibility and rigidity, or may have different secondary structural characteristics such as random loops or helical structures [Chen, et al., (2013), Adv Drug Deliv Rev, 65, 1357-1369]. The linker peptide may be a GGGGS, (GGGGS)2, ASTKGP, or ASTKGPSVFPLAP sequence linker that provides structural flexibility without being cleaved by proteases.

### Cell-penetrating degrading antibody

The term "cell-penetrating degrading antibody" as used herein refers to an antibody that binds, in the form of an immunoglobulin, preferably a complete immunoglobulin, to a membrane protein receptor overexpressed on the surface of a target cell such as a tumor tissue, is internalized (undergoes endocytosis), and is located in the cytosol due to endosomal escape ability thereof, wherein E2-Ub or E3 is located close to the target protein, to which the antibody is bound fused to the target protein, through U-Box or E3 recruiter specifically bound and fused to the target protein in the cytosol, to induce poly-ubiquitination (poly-Ub) of the target protein bound to the cytosol-penetrating interfering antibody, and the target protein labeled with poly-Ub is degraded and removed by the proteasome.

In addition, the present invention is directed to a polynucleotide of a cell-penetrating degrading antibody having a configuration in which the cytosol-penetrating interfering antibody is linked to a U-Box or E3 recruiter.

The polynucleotide is a polymer of deoxyribonucleotide or ribonucleotide present in single- or double-stranded form. The polynucleotide encompasses RNA genome sequences and DNA (gDNA and cDNA) and RNA sequences transcribed therefrom, and includes analogues of naturally derived polynucleotides, unless mentioned otherwise.

The polynucleotide includes not only nucleotide sequences encoding the cell-penetrating degrading antibody having a configuration in which the cytosol-penetrating interfering antibody is linked to the U-Box or E3 recruiter, but also sequences complementary to the sequences. Such complementary sequences include completely complementary sequences as well as substantially complementary sequences. This refers to a sequence capable of hybridizing with a nucleotide sequence encoding any one amino acid sequence selected from the group consisting of SEQ ID NOS: 6 to 25 under stringent conditions known in the art.

The polynucleotide may be varied. Such variation includes addition, deletion, or non-conservative or conservative substitution of nucleotides. The polynucleotide encoding the amino acid sequence is interpreted to include a nucleotide sequence having substantial identity with the nucleotide sequence. The expression "nucleotide sequence having substantial identity" means a nucleotide sequence that has a homology of at least 80%, more preferably a homology of 90%, 91%, 92%, 93%, 94%, or 95%, and most preferably a homology of 96%, 97%, 98%, or 99%, when aligning the nucleotide sequence of the present invention with any other sequence so as to correspond thereto as much as possible and analyzing the aligned sequence using algorithms commonly used in the art.

### Anti-β-catenin cytosol-penetrating interfering antibody

In an embodiment of the present invention, the anti-β-catenin cytosol-penetrating interfering antibody refers to an antibody that specifically binds to membrane protein receptors on the cell surface overexpressed in tumor cells or tissues, undergoes endocytosis, is located in the cytosol of cells through endosomal escape ability thereof, and binds to β-catenin, which is a target protein of cytosol, to inhibit functions thereof.

The "anti-β-catenin cytosol-penetrating interfering antibody" (cytosol-penetrating interfering antibody + U-box or E3 recruiter) constructed by fusing the anti-β-catenin cell-penetrating interfering antibody with the U-Box or E3 recruiter induces poly-ubiquitination (poly-Ub) of the target protein, β-catenin, such that E2-Ub or E3 is located close to the β-catenin and thereby induce degradation of the target protein by the proteasome.

### Method of producing cell-penetrating degrading antibodies

In another aspect, the present invention is directed to a method of producing a cell-penetrating degrading antibody as a complete immunoglobulin having a configuration in which an antibody that penetrates the cell and is distributed in the cytosol is linked to an E2-Ub recruiter or E3 recruiter as follows.
(1) cloning a nucleic acid including a heavy chain containing a heavy chain variable region (VH) capable of targeting β-catenin and a heavy chain constant region (CH1-hinge-CH2-CH3) of a human antibody, and an antibody and U-Box or E3 recruiter fused with an N-terminus or C-terminus of the heavy chain to prepare an anti-β-catenin heavy chain expression vector;
(2) cloning a nucleic acid including a light chain containing a light chain variable region (VL) capable of penetrating the cytosol and a light chain constant region (CL) of a human antibody and an antibody and a U-Box or E3 recruiter fused to an N-terminus or C-terminus of the light chain to prepare an endosome-escaping light chain expression vector;
(3) co-transforming the prepared heavy chain and light chain expression vectors into animal cells for protein expression to express a cell-penetrating degrading antibody in the form of a complete immunoglobulin; and
(4) purifying and recovering the expressed cell-penetrating degrading antibody.

The vector according to the present invention may be a vector system in which the light chain and the heavy chain are simultaneously expressed in one vector, or a vector system in which the light chain and the heavy chain are each expressed in separate vectors. In the latter case, the two vectors can be introduced into the host cell via co-transformation and targeted transformation.

The light-chain variable region (VL), light-chain constant region (CL), heavy-chain variable region (VH), heavy-chain constant region (CH1-hinge-CH2-CH3), and RNA degrading enzyme provided in the recombinant vector by the present invention may be operably linked to a promoter. The term "operably linked" means a functional linkage between a polynucleotide expression regulation sequence (e.g., promoter sequence) and another polynucleotide sequence. Accordingly, the regulation sequence can regulate transcription and/or translation of other nucleotide sequences.

The recombinant vector can typically be constructed as a vector for cloning or a vector for expression. The vector for expression may be a conventional vector used in the art to express foreign proteins in plants, animals or microorganisms. The recombinant vector may be constructed through any of various methods known in the art.

The recombinant vector may be constructed using prokaryotic or eukaryotic cells as hosts. For example, when the vector that is used is an expression vector and a prokaryotic cell is used as a host, the vector generally includes a potent promoter capable of conducting transcription (such as a pLλ promoter, trp promoter, lac promoter, tac promoter, T7 promoter, etc.), a ribosome-binding site to initiate translation, and a transcription/translation termination sequence. In addition, for example, when a eukaryotic cell is used as a host, the replication origin that operates in eukaryotic cells included in the vector includes, but is not limited to, an f1 replication origin, an SV40 replication origin, a pMB1 replication origin, an adeno replication origin, an AAV replication origin, a CMV replication origin, a BBV replication origin and the like. Also, a promoter derived from the genome of mammalian cells (e.g., a metallothionein promoter), or a promoter derived from a mammalian virus (e.g., adenovirus late promoter, vaccinia virus 7.5K promoter, SV40 promoter, cytomegalovirus (CMV) promoter, or HSV tk promoter) may be used, and the vector generally has a polyadenylation sequence as a transcription termination sequence.

In another aspect, the present invention is directed to a host cell transformed with the recombinant vector. The host cell may be any host cell well-known in the art, and in the case of transforming prokaryotic cells, includes, for example, *E. coli* JM109, *E. coli* BL21, *E. coli* RR1, *E. coli* LE392, *E. coli* B, *E. coli* X 1776, *E. coli* W3110, strains of the genus *Bacillus*, such as *Bacillus subtilis* and *Bacillus thuringiensis*, and enterococci and strains such as *Salmonella typhimurium*, *Serratia marcescens* and various *Pseudomonas* species. In the case of transforming eukaryotic cells, as host cells, yeast (*Saccharomyces cerevisiae*), insect cells, plant cells and animal cells such as SP2/0, Chinese hamster ovary K1, CHO DG44, PER.C6, W138, BHK, COS-7, 293, HepG2, Huh7, 3T3, RIN and MDCK cell lines and the like can be used.

Insertion of the recombinant vector into the host cell may be carried out using an insertion method well-known in the art. When the host cell is a prokaryotic cell, the delivery method may be a CaCl₂ method or an electroporation method, for example. When the host cell is a eukaryotic cell, the delivery method may be micro-injection, calcium phosphate precipitation, electroporation, liposome-mediated transfection, gene bombardment or the like, but is not limited thereto. The use of microorganisms such as *E. coli* realizes higher productivity than when using animal cells, but is not suitable for the production of intact Ig-type antibodies due to glycosylation problems, and is suitable for production of antigen-binding fragments such as Fab and Fv.

The method for selecting the transformed host cell can be easily carried out according to methods well-known in the art using a phenotype expressed by a selection marker. For example, when the selection marker is a gene specific for antibiotic resistance, the transformant can be easily selected by culturing the transformant in a medium containing the antibiotic.

### Pharmaceutical composition for preventing or treating cancer

As used herein, the term "fusion" refers to the integration of two molecules having different or identical functions or structures, and includes fusion through any physical, chemical or biological method capable of binding the antibody or antigen-binding fragment thereof to the protein, small-molecule drug, nanoparticle, or liposome. The fusion may preferably be carried out using a linker peptide, and the linker peptide may mediate fusion with the bioactive molecule at various positions of the antibody light-chain variable region, antibody, or fragment thereof according to the present invention.

In addition, the present invention provides a pharmaceutical composition for preventing or treating cancer containing the cell-penetrating degrading antibody fused with the U-box or E3 recruiter, or a bioactive molecule selected from the group consisting of a peptide protein, small molecule drug, nucleic acid, nanoparticle, and liposome fused therewith.

In another aspect, the present invention is directed to a method of inhibiting the growth of cancer or tumor cells and a method of treating cancer or a tumor using the cell-penetrating degrading antibody fused with the U-box or E3 recruiter.

The pharmaceutical composition for preventing or treating cancer may be administered orally or parenterally. The parenteral administration may be intravenous injection, subcutaneous injection, intramuscular injection, intraperitoneal injection, endothelial administration, topical administration, intranasal administration, pulmonary administration and rectal administration. Upon oral administration, proteins or peptides are digested, so that an oral composition may be coated with an active drug or formulated so as to protect the same from degradation in the stomach. In addition, the composition may be administered using any device capable of delivering the active substance to target cells.

When the composition is prepared as a pharmaceutical composition for preventing or treating cancer, the composition may contain a pharmaceutically acceptable carrier. The pharmaceutically acceptable carrier contained in the composition is conventionally used in the preparation of a drug, and includes, but is not limited to, lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate and mineral oil and the like. The pharmaceutical composition may further contain a lubricant, a wetting agent, a sweetener, a flavoring agent, an emulsifier, a suspending agent, a preservative or the like, in addition to the above ingredients.

The pharmaceutical composition for preventing or treating cancer may be administered orally or parenterally. The parenteral administration may be intravenous injection, subcutaneous injection, intramuscular injection, intraperitoneal injection, endothelial administration, topical administration, intranasal administration, pulmonary administration and rectal administration. Upon oral administration, proteins or peptides are digested, so that an oral composition may be coated with an active drug or formulated so as to protect the same from degradation in the stomach. In addition, the composition may be administered using any device capable of delivering the active substance to target cells.

The suitable dose of the pharmaceutical composition for preventing or treating cancer may vary depending on factors such as the formulation method, administration method, and age, body weight, gender, pathological conditions, diet, administration time, administration route, excretion rate and responsiveness of the patient. For example, the suitable dose of the composition may be within the range of 0.001 to 100 mg/kg for an adult. The term "pharmaceutically effective amount" may mean an amount sufficient to prevent or treat cancer or tumors.

Hereinafter, the present invention will be described in more detail with reference to examples. However, it will be obvious to those skilled in the art that these examples are provided only for illustration of the present invention and should not be construed as limiting the scope of the present invention.

### Example 1. Design of cytosol-penetrating interfering antibody specifically binding to intracellular β-catenin

In the present invention, in order to develop an anti-β-catenin cytosol-penetrating interfering antibody (anti-β-catenin iMab), development of a human antibody heavy-chain variable region (VH) that specifically recognizes human β-catenin was first commissioned by Y biologics, Inc. (located in Daejeon, Republic of Korea). Y Biologics, Inc. selected anti-β-catenin VH by bio-panning using β-catenin as an antigen from the human VH library displayed on the phage surface. In the present invention, inBC0 was constructed by replacing the anti-β-catenin VH with the anti-RAS VH of the cytosol-penetrating interfering antibody (inRas37) targeting the activated RAS (Shin, et al., (2020), Sci Adv, 6, eaay2174). However, the expression level of inBC0 in the animal cell HEK293F system (25.4 ± 4.6 mg/L of transfected cells) was low. Therefore, the yield (43.2±5.6 mg/L of transfected cells) was increased by two-fold by producing an inBC2 antibody having back-mutation induced to have the same sequence as the conserved region of framework (FR)4 of the human immunoglobulin antibody of inBC0.

In relation to the anti-β-catenin cytosol-penetrating interfering antibodies (inBC0, inBC2), the proteins were expressed and purified by transient transfection with the heavy-chain and light-chain expression vectors described in Table 2 (heavy chain variable region sequences of cytosol-penetrating interfering antibodies and cell-penetrating antibodies) and Table 3 (light chain variable region sequences of cytosol-penetrating interfering antibodies and cell-penetrating antibodies).

**[Table 3]**

| β-catenin antibody | Variable Region | Amino acid sequence | SEQ ID |
|---|---|---|---|
| inBC0 | VH | 10 20 30 40 50 52a53 60 QVQLVESGGGVVQPGRSL RL SCAASGF TFSSYPMHWVRQAPGKGLEWVA VVSYDGSYKYYA 70 80 82a 83 90 100100a 101 110 DSVKGRLTI SRDNSKNTLYLQMNSLRAEDTAVYYCARATVRGVTKI RGV FDSWGRGALVTVSS | 17 |
| inBC2 | | 10                    20                    30                    40                    50  52a53                 60 QVQL VES GGG VVQPGRSL RL SCAAS GFT FSSYPMHWVRQAPGKGLEWVA VVSYDGSYKYYA 70                    80  82a  83                    90                    100100a              101                    110 DSVKGRLTI SRDNSKNTLYLQMNSL RAEDTAVYYCARATVRGVTKI RGV FDSWGQGTLVTVSS | 18 |
| inCT | | 10 20 30 40 50 52a53 60 EVQLVESGGGLVQPGGSLRLSCAASGYTFTSYVMHWVRQAPGKGLEWVS AI NPYNDGNYYA 70 80 82a 83 90 100100a 101 110 DSVKGRFTI SRDNSRKTLYLQMNSL RAEDTAVYYCARGAYKRGYAMDYWGQGTTVTVSS | 19 |

| | | | |
|---|---|---|---|
| * The sequence of inBC2 different from the sequence of template inBC0 is indicated in bold. * The positions of amino acids are defined by Kabat rules | | | |

**[Table 4]**

| β-catenin antibody | Variable Region | Amino acid sequence | SEQ ID |
|---|---|---|---|
| inBC0 inBC2 inCT | VL | 10          20          27a     28 30          40          50          60 DYQLTQSPSSLSASVGDRVTI TCKSSQSLL NSRDGKNYLAWYQQKPGKAPKMLI YWASTRESGVPS 70          80          90          100 RFSGSGSGTDFTLTI SSLQPEDFATYFCQQYWYWMFTFGQGTKVEI KR 10          20          27a     28 30          40          50          60 DYQLTQSPSSLSASVGDRVTI TCKSSQSLL NSRDGKNYLAWYQQKPGKAPKMLI YWASTRESGVPS 70          80          90          100 RFSGSGSGTDFTLTI SSLQPEDFATYFCQQYAAAMFTFGQGTKVEI KR | 20 |
| inBC2-(AAA) | | | 21 |

| | | | |
|---|---|---|---|
| * The sequence of inBC2- (AAA) different from the sequence of template inBC0/inBC2/inCT is indicated in bold. * The positions of amino acids are defined by Kabat rules | | | |

Specifically, inBC0 was expressed using a heavy chain expression plasmid having a heavy chain variable region corresponding to SEQ ID NO: 17 in Table 3 and a light chain expression plasmid corresponding to SEQ ID NO: 20 in Table 4. In addition, inBC2 was expressed using a heavy chain expression plasmid having a heavy chain variable region corresponding to SEQ ID NO: 18 in Table 3 and a light chain expression plasmid corresponding to SEQ ID NO: 20 in Table 4. HEK293-F cells (Invitrogen) growing in suspension in serum-free FreeStyle 293 expression medium (Invitrogen) in a shake flask were transfected with a mixture of the plasmids and polyethylenimine (PEI) (PolyScience). When transfecting 200 ml of the mixture in the shake flask (Corning), HEK293-F cells were seeded at a density of 2.0 × 10⁶ cells/ml in 100 ml of a medium and cultured at 150 rpm in the presence of 8% CO₂ at 37°C. To produce antibodies, the appropriate heavy and light chain plasmids were diluted to a total of 250 µg (2.5 µg/ml), specifically, 125 µg of a heavy chain and 125 µg of a light chain, in 10 ml FreeStyle 293 expression medium, followed by filtering and reacting with 10 ml of medium diluted with 750 µg (7.5 ug/ml) of PEI at room temperature for 10 minutes. Then, the reacted mixed medium was added to the previously seeded cells at 100 ml and cultured in the presence of 8% CO₂ at 150 rpm and at 37°C for 4 hours, and then the remaining 100 ml of FreeStyle 293 expression medium was added thereto, followed by culturing for 6 days.

The protein was purified from the harvested cell-culture supernatant with reference to standard protocols. An antibody was applied to a protein A Sepharose column (GE healthcare) and washed with PBS (pH 7.4). The antibody was eluted at pH 3.0 with 0.1M glycine buffer and the sample was then immediately neutralized with 1M Tris buffer. The eluted antibody fraction was concentrated by exchanging the buffer with histidine (pH 6.5) through dialysis. Purified proteins were quantified using absorbance and extinction coefficient at a wavelength of 280 nm.

To evaluate the binding ability of the inBC2 antibody to anti-β-catenin, intermolecular binding was quantitatively measured using the bio-layer interferometry (BLI) system.

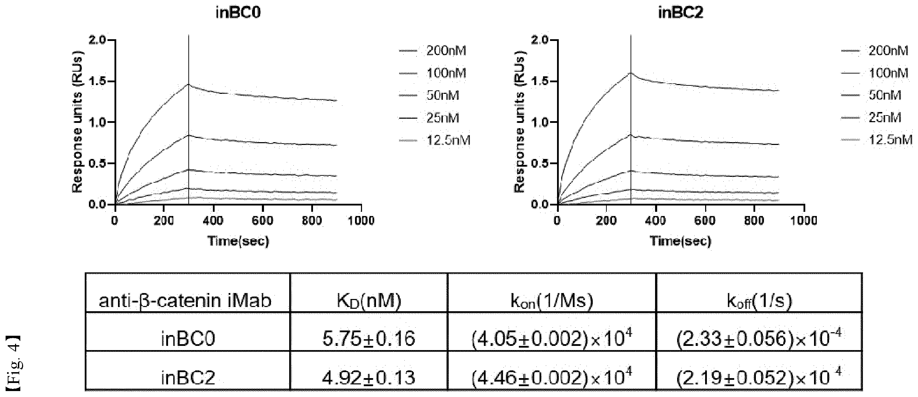

FIG. 4 shows the binding ability of inBC0 and inBC2 to anti-β-catenin. Specifically, the binding ability was measured in accordance with the protocol suggested by the manufacturer using an Octet QKe (ForteBio, USA) device. Specifically, 1× kinetic buffer was prepared by diluting 10x kinetic buffer (ForteBio, 18-1105) in PBS. The antibody was diluted to a concentration of 1 ug/ml in 1x kinetic buffer, the purified β-catenin was sequentially diluted at concentrations of 200, 100, 50, 25, 12.5, and 0 nM in 1× kinetic buffer, and 200 µl of each dilution was injected into an opaque 96-well plate through which light does not pass. Antigen affinity of antibodies was analyzed through the variation in refractive index occurring when the antibody bound to the antigen was detached therefrom while the AHC (anti-human immunoglobulin Fc capture) sensor chip moved in the order of 1x kinetic buffer, antibody dilution solution, 1x kinetic buffer, antigen dilution solution, and 1× kinetic buffer. Affinity was calculated with Octet Data Analysis software 11.0 in a 1:1 binding model.

The affinity of anti-β-catenin antibodies to β-catenin was analyzed using Octet QKe. The result showed that the affinity (KD) of inBC0 for β-catenin was 5.75 nM, and the affinity of inBC2 for β-catenin was 4.92 nM. The inBC2 antibody is a cytosol-penetrating interfering antibody with improved expression compared to the inBC0 antibody and has no change in β-catenin antigen-binding ability. For this reason, inBC2 was used as a template for the cell-penetrating degrading antibody in the present invention.

To produce the recombinant antigen protein β-catenin used in this example, pET23m vector (Addgene) was cloned using BamHI and NotI to express His8 - Avi-tag - β-catenin. *E. coli* BL21 (DE3) was co-transformed with the pET23m His8 - Avi - β-catenin expression plasmid and a pBirAcm (Avidity, USA) plasmid, and then was plated on LB agar solid medium (LBCA) containing 34 ug/ml of chloramphenicol and 100 ug/ml of ampicillin, followed by culturing at 37°C for 16 hours. A single colony was harvested from the transformant and was inoculated into 5 mL of SB medium (SBCA) containing 34 ug/ml of chloramphenicol and 100 ug/ml of ampicillin, followed by culture for 18 hours. The pre-culture was inoculated into 250 ml of LB liquid medium containing 34 ug/ml of chloramphenicol and 100 ug/ml of ampicillin at an initial inoculation concentration of OD_{0.05}, followed by main culture. OD₆₀₀ was measured with Thermo Scientific^{™}, #51119200, and 0.5 mM IPTG (isopropyl-β-D-thiogalactopyranoside) was added such that the concentration of D-Biotin was 50 µM when the OD₆₀₀ reached 0.8, followed by culture for 4 hours at 20°C and 150 rpm for 4 hours to induce expression. Then *E. coli* cells were harvested by centrifugation at 8,000 rpm and at 4°C for 15 minutes, suspended in 20 ml of Tris-HCl pH 8.0 buffer (50 mM Tris, 300 mM NaCl, 1 mM 2-mercaptoethatnol) and pulverized using an ultrasonication cell grinder (SONICS, #VCX 750). Then the cells were centrifuged at 4°C and 15,000 for 20 minutes and the supernatant was filtered through a 0.45 um SFCA syringe filter (Sartorius, #16533-K) to obtain a culture supernatant containing secreted proteins. 50 ml of the culture supernatant was linked to a Ni-NTA resin (GE healthcare, #) at 4°C for 1 hour, and the mixture was injected into a column (Thermo Scientific^{™}, #29924). After filtering the mixed solution, Tris-HCl pH 8.0 buffer containing 30 mM imidazole was injected into the column to non-selectively elute the proteins remaining in the column. An elution buffer containing 300 mM imidazole was injected into the column and allowed to stand for 1 minute, and then the biotinylated β-catenin protein was eluted. For the purified biotinylated β-catenin, imidazole was dialyzed with the final buffer (2 mM DTT containing PBS, pH 7.4) using Centrifugal Filters (Satorious, #VS2022). The protein purified through the process was quantified using absorbance and extinction coefficient at a wavelength of 280 nm.

### Example 2. Construction of anti-β-catenin cell-penetrating degrading antibody fused with U-Box or E3 recruiter (inBC2-E3 recruiter)

A cell-penetrating degrading antibody was developed by fusing a cytosol-penetrating interfering antibody, inBC2, which penetrates the cytosol of cells and specifically recognizes β-catenin, with E3 or U-Box of E3 that can bind to the E2-Ub (E2-ubiquitin conjugate) of the UPS, wherein, after penetration into the cytosol, U-Box recruits E2-Ub in the cytosol, localizes E2-Ub close to the target protein and induces poly-ubiquitination of the target protein, and the E3 recruiter recruits E3, locates E3 close to the target protein and induces poly-ubiquitination of the target protein, thereby ultimately causing degradation of target proteins by the UPS.

Specifically, for optimal decomposition of β-catenin in the cytosol of inBC2, the U-box for binding to E2-Ub or the E3 recruiter for binding to E3 were found and improved, and the fusion positions and linkers of the selected U-Box or E3 recruiter were optimized.

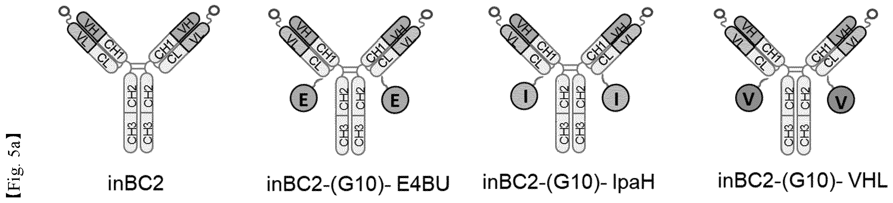

FIG. 5A is a schematic diagram illustrating the construction of inBC2, inBC2-E2-Ub recruiters (E4B₁₂₂₇₋₁₃₀₂, IpaH₂₅₄₋₅₄₅), and inBC2-E3 recruiters (VHL₁₅₂₋₂₁₃). In the present invention, a total of three types of E2-Ub recruiters and E3 recruiters were selected and fused with the light chain C-terminus of inBC2 using a (G4S)2 linker (G10). In inBC2, the sequence of the heavy chain variable region is SEQ ID NO: 18, and the sequence of the light chain variable region is SEQ ID NO: 20. In addition, inBC2-(G10)-E4B₁₂₂₇₋₁₃₀₂ is a recruiter in which the modified U-box domain of SEQ ID NO: 10 is fused to the light chain C-terminus of inBC2. inBC2-(G10)-IpaH₂₅₄₋₅₄₅ is a recruiter in which modified IpaH₂₅₄₋₅₄₅ of SEQ ID NO: 11 produced by fragmenting only the active domain that binds to the E2-ubiquitin conjugate derived from *Shigella spp* is fused to the light chain C-terminus of inBC2. inBC2-(G10)-VHL₁₅₂₋₂₁₃ is a recruiter in which the E3 recruiter of SEQ ID NO: 12 is fused to the light chain C-terminus of inBC2. Using the method, an E2-Ub recruiter or an E3 recruiter was fused to the light chain C-terminus of inBC2, to induce β-catenin degradation, and thereby express and purify a cell-penetrating degrading antibody that can exhibit a tumor growth inhibitory effect.

Specifically, to construct a light chain expression vector to produce a fusion of E2-Ub recruiter or E3 recruiter to the C-terminus of a monoclonal antibody light chain in the form of a complete immunoglobulin, a total of three types of E2-Ub recruiters and E3 recruiters were fused with the C-terminus of the light chain including cytosol-penetrating light-chain variable region (hT4-59 VL, SEQ ID NO: 20) and light-chain constant region (CL) are fused with an integrin receptor targeting a circular peptide fused with DNA encoding a secretion signal peptide at the 5' end thereof using the GGGGSGGGGS linker (G10), and the DNA encoding the fusion was cloned into the pcDNA3.4 (Invitrogen) vector with NotI/HindIII. In addition, in order to construct a vector expressing the heavy chain, the DNA encoding the heavy chain including the heavy chain variable region (BCO-VH, SEQ ID NO: 17) and the heavy chain constant region (CH1-hinge-CH2-) of an antibody fused with DNA encoding a secretion signal peptide at the 5' end thereof was cloned into the pcDNA3.4 (Invitrogen) vector.

Proteins were expressed and purified by transient transfection of the light-chain and heavy-chain expression vectors. HEK293-F cells (Invitrogen) growing in suspension in serum-free FreeStyle 293 expression medium (Invitrogen) in a shake flask were transfected with a mixture of the plasmids and polyethylenimine (PEI) (PolyScience). When transfecting 200 ml in the shake flask (Corning), HEK293-F cells were seeded at a density of 2.0 × 10⁶ cells/ml in 100 ml of medium and cultured in the presence of 8% CO₂ at 37°C and at 150 rpm. To produce antibodies, the appropriate heavy and light chain plasmids were diluted to a total of 250 µg (2.5 µg/ml), specifically, 125 µg of heavy chain and 125 µg of light chain, in 10 ml FreeStyle 293 expression medium, followed by filtering and reacting with 10 ml of medium diluted with 750 µg (7.5 ug/ml) of PEI at room temperature for 10 minutes. Then, the reacted mixed medium was added to the previously seeded cells at 100 ml and cultured at 150 rpm in the presence of 8% CO₂ at 37°C for 4 hours, and then the remaining 100 ml of FreeStyle 293 expression medium was added thereto, followed by culturing for 6 days.

The protein was purified from the harvested cell-culture supernatant with reference to standard protocols. An antibody was applied to a protein A Sepharose column (GE healthcare) and washed with PBS (pH 7.4). The antibody was eluted at pH 3.0 with 0.1M glycine buffer and the sample was then immediately neutralized with 1M Tris buffer. The eluted antibody fraction was concentrated by exchanging the buffer with histidine (pH 6.5) through dialysis. Purified proteins were quantified using absorbance and extinction coefficient at a wavelength of 280 nm. The yields of the three specific types of inBC2, inBC2-E2-Ub recruiter, and inBC2-E3 recruiter are shown in Table 5 below, and specific experiments showed that the yields of the three types were slightly reduced or maintained compared to inBC2.

**[Table 5]**

| Degrobody | Linker | E3 recruiter (size) | Purification yield (mg/L of transfected cells) |
|---|---|---|---|
| inBC2 | - | - | 43.2±5.6 |
| inBC2-(G10)-E4B₁₂₂₇₋₁₃₀₂ | (G₄S)₂ | E4B₁₂₂₇₋₁₃₀₂ (8.9 kDa) | 36±2 |
| inBC2-(G10)-IpaH₂₅₄₋₅₄₅ | (G₄S)₂ | IpaH₂₅₄₋₅₄₅ (33.3kDa) | 18±3 |
| inBC2-(G10)-VHL₁₅₂₋₂₁₃ | (G₄S)₂ | VHL₁₅₂₋₂₁₃ (7.5 kDa) | 35±2 |

### Example 3. Evaluation of physical properties of anti-β-catenin cell-penetrating degrading antibody.

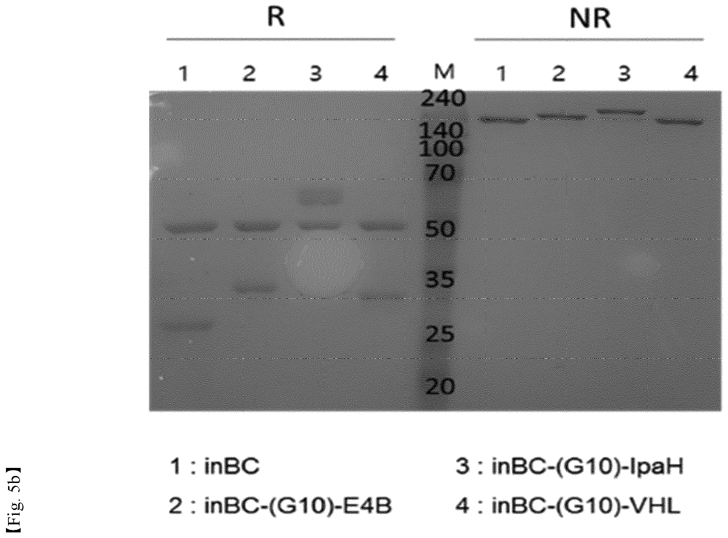

FIG. 5B shows results of 12% SDS-PAGE of the antibodies of FIG. 5A after purification under reducing or non-reducing conditions. Specifically, the result showed that inBC2 has a molecular weight of about 150 kDa under non-reducing conditions, and the heavy chain has a molecular weight of 50 kDa and the light chain has a molecular weight of 25 kDa under reducing conditions. In BC2-(G10)-E4B₁₂₂₇₋₁₃₀₂, linked with E4B₁₂₂₇₋₁₃₀₂ using a (G₄S)₂ linker, bands were observed in the heavy chain of 50 kDa and the light chain of 34 kDa. In BC2- (G10)-IpaH₂₅₄₋₅₄₅, which was linked to IpaH₂₅₄₋₅₄₅ using a (G₄S)₂ linker, bands were observed in the heavy chain of 50 kDa and the light chain of 58 kDa. In BC2-(G10)-VHL₁₅₂₋₂₁₃, which was linked to VHL₁₅₂₋₂₁₃ using a 2 linker, bands were observed in the heavy chain of 50 kDa and the light chain of 33 kDa. This indicates that the three types of antibodies exist as monomers in solution and do not form dimers or oligomers through unnatural disulfide bonds.

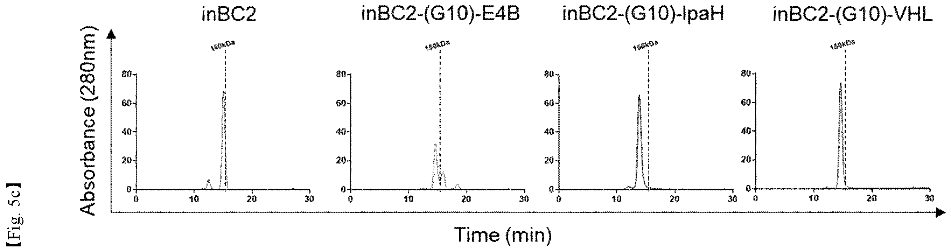

FIG. 5C is a graph showing mAU of three types of inBC2, inBC2-E2-Ub recruiter, and inBC2-E3 recruiter measured at 280 nm using size exclusion chromatography. Specifically, 45 µl of inBC2, inBC2- (G10)-E4B₁₂₂₇₋₁₃₀₂, inBC2-(G10)-IpaH₂₅₄₋₅₄₅, and inBC2-(G10)-VHL₁₅₂₋₂₁₃ were prepared at a concentration of 1 mg/ml and injected into an insert to prepare samples. 150 mM sodium phosphate, 137 Mm NaCl pH 7.0 buffer was prepared by flowing at a flow rate of 1.0 ml/min and allowing to stand until the pressure and mAU at 280nm were stabilized. Then, 20 µl of the prepared sample was loaded at the same flow rate and mAU was measured at 280 nm for 30 minutes. As IpaH₂₅₄₋₅₄₅ or U-box or E3 recruiter was fused, the molecular weight increased and the peak was observed at an earlier time than inBC2. Single peaks were observed in the order of inBC2-(G10)-VHL₁₅₂₋₂₁₃ and inBC2- (G10)-IpaH₂₅₄₋₅₄₅, without oligomers and tailing, whereas this was not observed in inBC2- (G10) -E4B₁₂₂₇₋₁₃₀₂. Then, inBC2-(G10)-VHL₁₅₂₋₂₁₃, which has similar yield [Table 5] and physical properties to inBC2, was selected from the three types of inBC2-E2-Ub and inBC2-E3 recruiters, and further experiments were conducted.

### Example 4. Construction, expression, and purification of inBC2-VHL₁₅₂₋₂₁₃ linker variants

Unlike the VHL selected as the final E3 recruiter fused to the light chain C-terminus using a (G4S)2 linker, three novel inBC2-VHL 152-213 linker variants were constructed using a different peptide linker. Which clone among the four inBC2-VHL₁₅₂₋₂₁₃ linker variants would be able to locate E3 close to β-catenin and thereby deliver Ub well was predicted through structural modeling. Prediction was performed using the Chimera program using the already known antibody structure (PDB ID: 5UR8), β-catenin structure (PDB ID: 1JPW), and E3 structure (PDB ID: 5N4W, 4WQO) consisting of VHL, EloBC, Cul2, and Rbx1 as samples. The result of modeling indicated that, when VHL was fused to the C-terminus of the antibody light chain using a linker having a length of about 13Å, the distance between the active domain, Rbx1, and the target protein, β-catenin, was about 50Å, which is known to be beneficial to Ub delivery. Therefore, it was predicted that inBC2-VHL₁₅₂₋₂₁₃ linked using a flexible loop having a length of 15Å and containing 152-158 amino acids (TLPVYTL) as a linker without an additional linker will have optimal beta-catenin degradation activity.

Four inBC2-VHL₁₅₂₋₂₁₃ linker variants were expressed and purified in the same manner as Example 2 above. The specific yields of the four inBC2-VHL₁₅₂₋₂₁₃ linker variants are shown in Table 6 below.

**[Table 6]**

| inBC2-VHL₁₅₂₋₂₁₃linker variants | Linker | Purification yield (mg/L of transfected cells) |
|---|---|---|
| inBC2 | - | 43.2±5.6 |
| inBC2-(A6)-VHL₁₅₂₋₂₁₃ | ASTKGP | 34±5.1 |
| inBC2-(G5)-VHL₁₅₂₋₂₁₃ | G₄S | 25±10.3 |
| inBC2-(G10)-VHL₁₅₂₋₂₁₃ | (G₄S)₂ | 35±2 |
| inBC2-VHL₁₅₂₋₂₁₃ | - | 34±3.6 |

inBC2-(G5)-VHL₁₅₂₋₂₁₃ having a configuration in which VHL₁₅₂₋₂₁₃ was linked to the light chain C-terminus via a GGGGS linker exhibited a slight decrease in production yield compared to the conventional inBC2. In addition, inBC2-(A6)-VHL₁₅₂₋₂₁₃ having a configuration in which VHL₁₅₂₋₂₁₃ was fused to the light chain C-terminus via an ASTKGP linker was found to form an oligomer through an unnatural disulfide bond.

### Example 5. Evaluation of β-catenin degradation activity of inBC2-VHL₁₅₂₋₂₁₃ linker variant.

Whether or not four types of inBC2-VHL₁₅₂₋₂₁₃ linker variants specifically recruited Cul5-based E3 to β-catenin in cells, exhibited β-catenin degradation activity, and reduced the amount of β-catenin was determined by Western blot. Specifically, colon cancer cell lines, in which β-catenin was accumulated, were diluted in 1 ml of 1.0×10⁵ cells/well in a 12-well plate, cultured at 37°C in the presence of 5% CO₂ for 24 to 48 hours, and then treated with 2 µM of each of inBC2-VHL₁₅₂₋₂₁₃ and 4 control antibodies (inBC2, inCT-VHL, inBC2 (AAA) -VHL, inBC2-VHL₁₅₂₋₂₁₃ (V181G, D187K)) in the presence of 5% CO₂ at 37°C for 12 hours. Then, the medium was removed, and proteins attached to the cell surface were removed with a slightly acidic solution (200 mM glycine, 150 mM NaCl, pH 3.0), and washed with PBS. The cells were lysed using cell lysis buffer (10 mM Tris-HCl pH 7.4, 100 mM NaCl, 1% SDS, 1mM EDTA, protease inhibitors). The cell lysate was quantified and Western blot was performed using anti-β-catenin antibody and anti-β-actin antibody. Then, each sample band was quantified using Image J software (National Institutes of Health, USA). The ratio of β-catenin level/beta-actin level for each sample was calculated and normalized by the percentage relative to the vehicle sample.

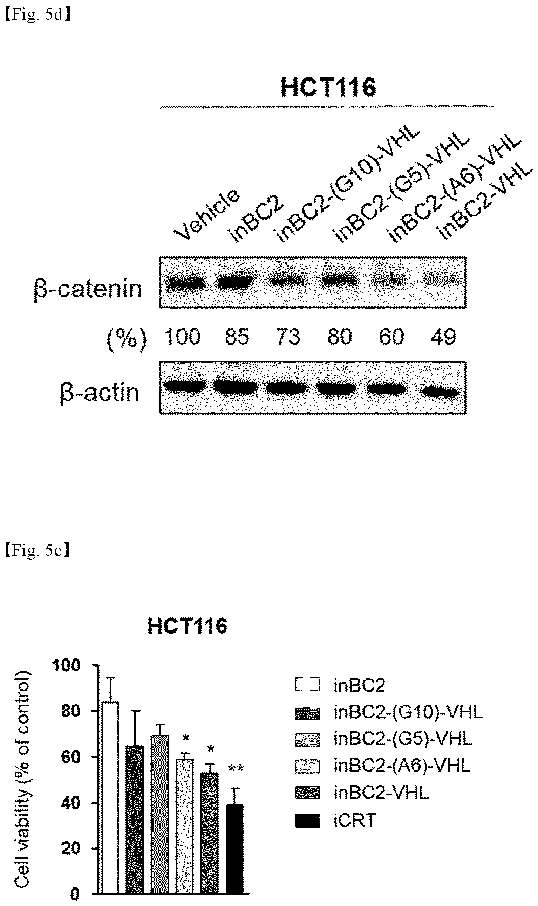

As a result, as shown in FIG. 5D, the antibody (inBC2-(G10)-VHL₁₅₂₋₂₁₃) fused to VHL using a linker having a length of about 35Å exhibited low β-catenin degradation activity of 8% compared to the conventional anti-β-catenin antibody (inBC2). inBC2-(G5)-VHL₁₅₂₋₂₁₃, which used a highly flexible linker having a length of about 18Å, was found to have the lowest β-catenin degradation activity of 5% compared to inBC. inBC2-(A6)-VHL₁₅₂₋₂₁₃, which used a linker predicted to have a length of about 17Å and rigidity, exhibited a relatively high β-catenin degradation activity of 25% compared to inBC2. inBC2-VHL₁₅₂₋₂₁₃, in which VHL₁₅₂₋₂₁₃ was linked to the C-terminus of the antibody light chain with the highest rigidity without an additional linker, exhibited a high β-catenin degradation activity of 36% compared to inBC2. This indicates that the degradation activity of β-catenin may vary depending on the type of linker. In addition, as predicted through modeling in one embodiment of the present invention, inBC2-VHL₁₅₂₋₂₁₃ linked with VHL using a flexible 15Å long loop containing 152-158 amino acids (TLPVYTL) contained in VHL ₁₅₂₋₂₁₃ without an additional linker as a linker was found to have optimal β-catenin degradation activity.

### Example 6. Evaluation of cell growth inhibition of inBC2-VHL₁₅₂₋₂₁₃ linker variants, aaaaa

The degree of cell growth inhibition was evaluated to determine whether or not the growth of colon cancer cell lines in which β-catenin was accumulated *in vitro* was inhibited *in vitro* by the β-catenin degradation activity of the inBC2-VHL₁₅₂₋₂₁₃ linker variants.

FIG. 5E is a graph showing *in vitro* cell growth inhibition against the colon cancer cell line HCT116 of four inBC2 and inBC2-VHL₁₅₂₋₂₁₃ linker variants and an inhibitor of β-catenin-responsive transcription 14 (iCRT14), which acts on the Wnt signaling pathway to inhibit the transcriptional activity of β-catenin as a positive control. Specifically, 1×10³ HCT116 cells/well were each diluted in 0.1 ml of medium containing 10% FBS and cultured in a 96-well plate at 37°C under 5% CO₂ conditions for 48 hours. Then, the cells were treated with 2 µM and 20 µM of four types of inBC2 and inBC2-VHL₁₅₂₋₂₁₃ linker variants and iCRT14 at 37°C under 5% CO₂ conditions for 48 hours. Changes in cell counts were measured using CellTiter Glo (CTG) assay. Specifically, after treatment for 48 hours, 70 µl of CTG solution (Promega, #G7572) was added to each well and reacted at room temperature for 10 minutes to lyse the cells. The luminescence of the cell lysate was measured and the cell survival rate was calculated as a percentage relative to the vehicle group. Among the four inBC2-VHL₁₅₂₋₂₁₃ linker variants, inBC2-VHL₁₅₂₋₂₁₃ had the highest cell growth inhibition activity of about 30%. Therefore, among the four inBC2-VHL₁₅₂₋₂₁₃ linker variants, inBC2-VHL₁₅₂₋₂₁₃, in which VHL is linked without an additional linker, had similar yield and physical properties to inBC2, and had the highest β-catenin degradation activity and cell growth inhibition activity, was used in the final format.

### Example 7. Construction of control antibody to identify mechanism of action of inBC2-VHL₁₅₂₋₂₁₃ and evaluation of physical properties thereof

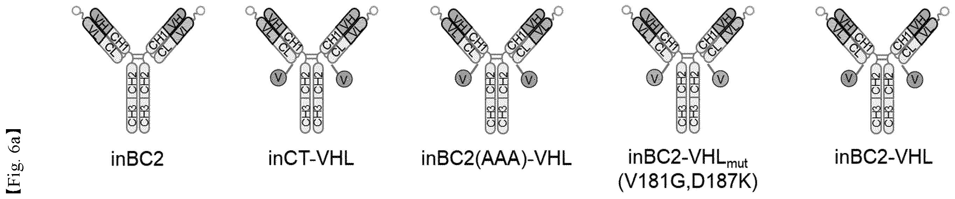

FIG. 6A is a schematic diagram illustrating inBC2-VHL₁₅₂₋₂₁₃ and necessary control antibodies to identify the specific mechanism of action of inBC2-VHL₁₅₂₋₂₁₃. inCT-VHL₁₅₂₋₂₁₃, which has the same components as inBC2-VHL₁₅₂₋₂₁₃, except that it has a heavy chain variable region (SEQ ID NO: 19) having no β-catenin targeting ability, was constructed as a β-catenin targeting control. inBC2 (AAA) -VHL₁₅₂₋₂₁₃ is a control antibody lacking endosomal escape ability because the endosomal escape motif ⁹²WYW⁹⁴ amino acids located in the light chain variable region is replaced with ⁹²AAA⁹⁴, and cannot be located in the cytoplasm. The sequence of the light chain variable region in inBC2 (AAA)-VHL₁₅₂₋₂₁₃ is represented by SEQ ID NO: 21. As a VHL₁₅₂₋₂₁₃ fusion control, inBC2-VHL₁₅₂₋₂₁₃ (V181G, D187K) fused to VHL variants (V181G, D187K) was used. Because two single variants, VHL(V181G) and VHL(D187K), have been reported to have reduced interaction with Cul2 [Nguyen, et al., (2015), Structure, 23, 441-449], novel VHL variants (V181G, D187K) were constructed by fusing the two variants and were then fused to the C-terminus of the antibody light chain. inBC2-VHL₁₅₂₋₂₁₃ (V181G, D187K) was predicted to be unable to recruit Cul2-based E3 to β-catenin. The E3 recruiter of inBC2-VHL₁₅₂₋₂₁₃ and the variant inBC2-VHL₁₅₂₋₂₁₃ (V181G, D187K) was fused to the light chain C-terminus of inBC2, and the specific sequence of the E3 recruiter is shown in Table 7 below.

**[Table 7]**

| inBC2-VHLs | SOCS2 amino acid sequence | SEQ ID |
|---|---|---|
| inBC2-VHL₁₅₂₋₂₁₃ | 152 TLPVYTLKERCLQVVRSLVKPENYRRLDIVRSLYEDLEDHPNVQKDLER LTQERIAHQRMGD 213 | 12 |
| inBC2-VHL₁₅₂₋₂₁₃(V181G, D187K) | ¹⁵⁰ TLPVYTLKERCLQVVRSLVKPENYRRLDIQRSLYEKLEDHPNVQKDLER LTQERIAHQRMGD ²¹¹ | 22 |

| | | |
|---|---|---|
| * The sequence of inBC2-SOCS2s different from template inBC2-VHL₁₅₂₋₂₁₃ is indicated in bold. | | |

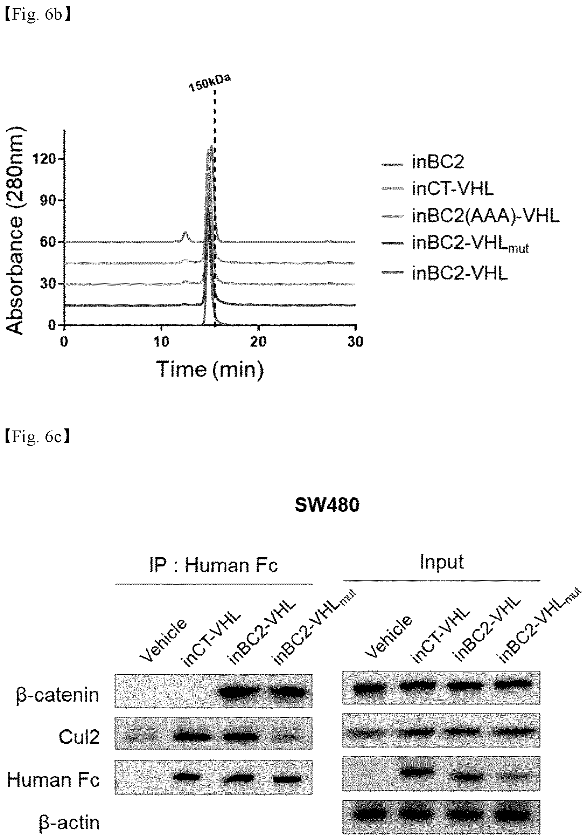

FIG. 6B is a graph showing mAU of four types of control antibodies and inBC2-VHL₁₅₂₋₂₁₃ measured at 280 nm using size exclusion chromatography in the same manner as in Example 2. The result showed that all four control antibodies and inBC2-VHL₁₅₂₋₂₁₃ showed single peaks and did not form dimers or oligomers.

### Example 8. Evaluation of specific binding between β-catenin and Cul2 in cells of inBC2-VHL₁₅₂₋₂₁₃.

FIG. 6C shows the result of immunoprecipitation to determine whether or not inBC2-VHL₁₅₂₋₂₁₃ is actually located inside the cell to target β-catenin and at the same time binds to Cul2-based E3 using fused VHL to clearly identify the mechanism of action of inBC2-VHL₁₅₂₋₂₁₃. Specifically, 5×10⁶ cells of colon cancer cell line SW480, in which β-catenin is accumulated, were diluted in 10 ml on a 100 mm³ plate and cultured at 37°C and 5% CO₂ conditions for 24 hours, and then treated in an amount of 2 µM with inBC2-VHL₁₅₂₋₂₁₃ and control antibodies, inCT-VHL₁₅₂₋₂₁₃ and inBC2-VHL₁₅₂₋₂₁₃ (V181G, D187K) at 37°C in the presence of 5% CO₂ for 12 hours. Then, the medium was removed and proteins attached to the cell surface were removed with a slightly acidic solution (200 mM glycine, 150 mM NaCl, pH 3.0) and then washed with PBS. The cells were lysed using cell lysis buffer (20 mM Tris-HCl pH 7.4, 150 mM NaCl, 0.5% Triton X-100, 0.05% SDS, 1 mM EDTA, protease inhibitors), and the cell debris was removed by precipitation). Then, Protein A agarose reacted with an anti-human Fc antibody (Sigma, #A9544) was added to the cell lysate and then reacted for 2 hours, to precipitate the antibody. Then, Western blot was performed using an anti-β-catenin antibody (Cell signaling, #9582S), an anti-Cul2 antibody (Santa cruz, #sc-166506), an anti-human Fc antibody, and an anti-beta-actin antibody (Santa cruz, #sc-69879).

As a result, as shown in FIG. 6C, both β-catenin and Cul2 were observed only in inBC2-VHL₁₅₂₋₂₁₃, while only Cul2 was observed in inCT-VHL₁₅₂₋₂₁₃ and only β-catenin was observed in inBC2-VHL₁₅₂₋₂₁₃ (V181G, D187K). The result of the experiment showed that, unlike the control antibodies, only inBC2-VHL₁₅₂₋₂₁₃ simultaneously bound to β-catenin and Cul2 in cells.

### Example 9. Evaluation of β-catenin degradation ability in cells of inBC2-VHL₁₅₂₋₂₁₃.

To determine whether or not inBC2-VHL₁₅₂₋₂₁₃ specifically exhibits intracellular β-catenin degradation activity, the decrease in the amount of β-catenin was confirmed by Western blot using four types of control antibodies. Specifically, the experiment was conducted in the same manner as Example 5 above.

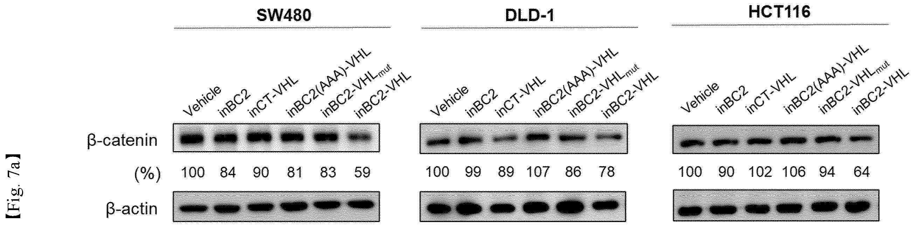

As a result, as can be seen from FIG. 7A, inBC2-VHL₁₅₂₋₂₁₃ had an average of 20% of β-catenin degradation activity compared to inBC2 in three colon cancer cell lines (SW480, DLD-1, HCT116). On the other hand, inCT-VHL₁₅₂₋₂₁₃, the control antibody that cannot target β-catenin, did not exhibit β-catenin degradation activity, similar to the conventional inBC2. In addition, inBC2-(AAA)-VHL₁₅₂₋₂₁₃, the control antibody that lost endosomal escape ability and could not be located in the cytoplasm, did not exhibit β-catenin degradation activity.

### Example 10. Evaluation of intracellular β-catenin degradation ability of inBC2-VHL₁₅₂₋₂₁₃ depending on concentration and time.

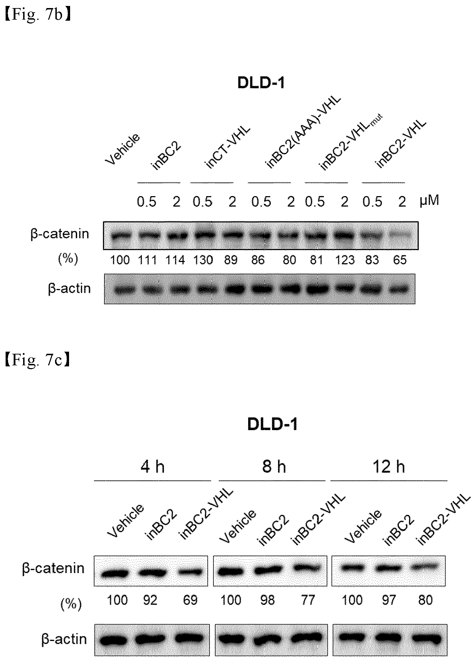

To further investigate the β-catenin degradation activity of inBC2-VHL₁₅₂₋₂₁₃, Western blot was performed under different concentration and time conditions. FIG. 7B shows the result of Western blot confirming the amount of β-catenin measured after treating the colon cancer cell line DLD-1, in which β-catenin is accumulated, with four types of control antibodies and inBC2-VHL₁₅₂₋₂₁₃ at amounts of 0.5 µM and 2 µM, at 37°C in the presence of 5% CO₂ for 12 hours. Specifically, the experiment was conducted in the same manner as Example 5 above. Approximately 15% of β-catenin degradation activity was confirmed only in samples treated with 2 µM of inBC2-VHL₁₅₂₋₂₁₃.

FIG. 7C shows the result of Western blot confirming the amount of β-catenin measured after treating the colon cancer cell line DLD-1, in which β-catenin is accumulated, with 2 µM inBC2 and 2 µM inBC2-VHL₁₅₂₋₂₁₃ at 37°C in the presence of 5% CO₂ for 12 hours. Specifically, the experiment was conducted in the same manner as Example 5 above. The result showed that inBC2-VHL₁₅₂₋₂₁₃ exists in the cytoplasm for 4 to 12 hours and has an average β-catenin degradation activity of 25%.

### Example 11. Evaluation of β-catenin poly-ubiquitination of inBC2-VHL₁₅₂₋₂₁₃.

Examples 8 to 10 showed that inBC2-VHL₁₅₂₋₂₁₃ recruited Cul2-based E3 to degrade β-catenin. Therefore, according to the present invention, Western blot was performed to determine whether or not β-catenin was poly-ubiquitinated through mobilized E3 and degraded by the proteasome based on the mechanism of action of inBC2-VHL₁₅₂₋₂₁₃. Specifically, HCT116 was treated with 0.5 µM of Cullin activity inhibitor MLN4924 (Sigma, #5.05477.0001) or 5 µM of proteasome activity inhibitor, Bortezomib (Sigma, #5.04314.0001) for 30 minutes, and then the solution was removed and washed with medium. Then, the cells were treated with 2 µM inBC2 and inBC2-VHL₁₅₂₋₂₁₃ at 37°C in the presence of 5% CO₂ for 12 hours. The subsequent experiment was conducted in the same manner as Example 5 above.

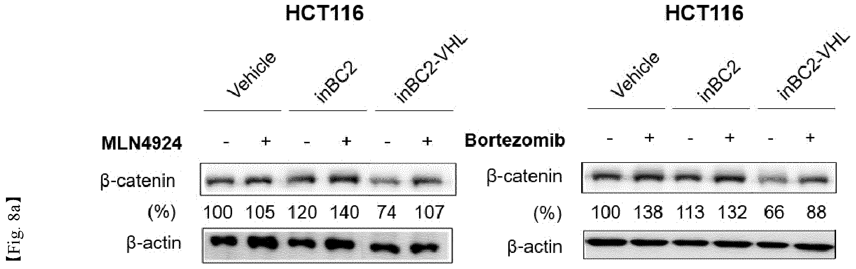

As a result, as can be seen from FIG. 8A, an average 30% in the amount of β-catenin was decreased only in the group treated with inBC2-VHL₁₅₂₋₂₁₃ without a cullin activity inhibitor or proteasome activity inhibitor. The amount of β-catenin did not decrease in samples treated with activity inhibitors and inBC2-VHL₁₅₂₋₂₁₃. The experimental results showed that inBC2-VHL₁₅₂₋₂₁₃ recruited Cul2-based E3 to degrade β-catenin through the proteasome.

In the present invention, poly-ubiquitination of β-catenin as part of the mechanism of action of inBC2-VHL₁₅₂₋₂₁₃ was confirmed using immunoprecipitation. Specifically, 5×10⁵ DLD-1 was diluted in 10 ml on a 100 mm³ plate and cultured at 37°C in the presence of 5% CO₂ for 24 hours. Then, the cells were treated with 5 µM Bortezomib, a proteasome activity inhibitor, for 30 minutes, and then the solution was removed and washed with medium. The cells were treated with inBC2-VHL₁₅₂₋₂₁₃ or control antibody in an amount of 2 µM at 37°C in the presence of 5% CO₂ for 12 hours. The experimental process up to cell lysis was performed in the same manner as Example 5. Then, Protein A agarose reacted with an anti-β-catenin antibody was added to the cell lysate and reacted for 2 hours, and β-catenin was precipitated. Then, Western blot was performed using an anti-K-48 poly ubiquitin antibody (Sigma, #05-1307) and an anti-β-catenin antibody.

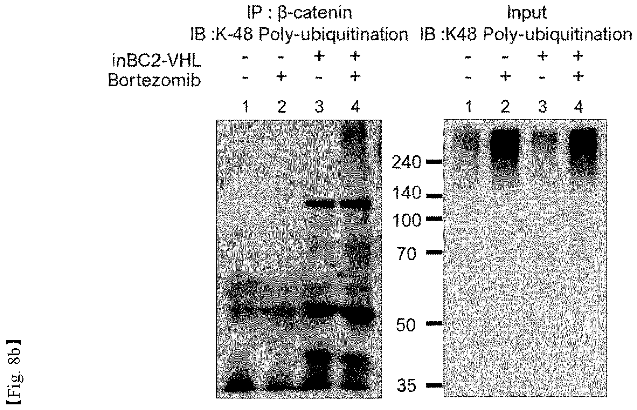

FIG. 8B shows the results of Western blot of four samples treated and not treated with Bortezomib and inBC2-VHL₁₅₂₋₂₁₃, respectively, using an anti-K-48 poly-ubiquitinated antibody. The results showed that K-48 poly-ubiquitinated β-catenin band appeared only in the group treated with both bortezomib and inBC2-VHL₁₅₂₋₂₁₃.

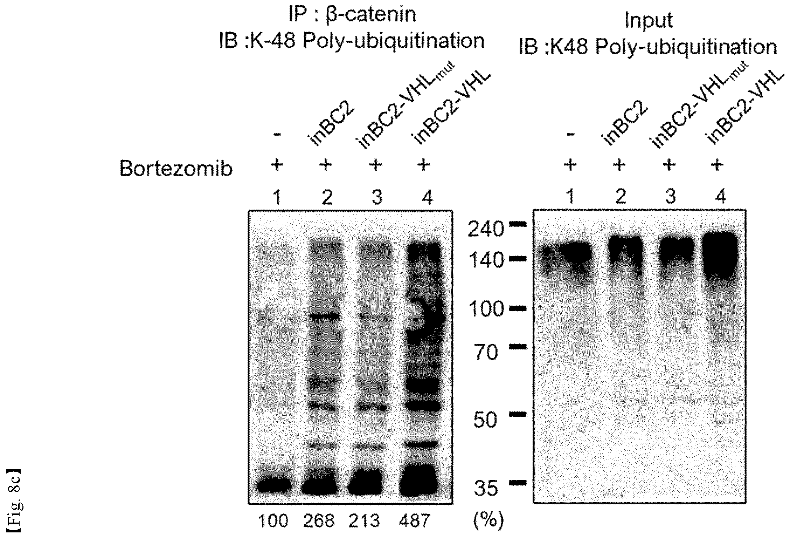

FIG. 8C shows the β-catenin poly-ubiquitination of inBC2-VHL₁₅₂₋₂₁₃ including two control antibodies (inBC2, inBC2-VHL₁₅₂₋₂₁₃ (V181G, D187K)) under conditions treated with bortezomib, evaluated by Western blot using anti-K-48 poly-Ub antibody. The overall band intensities observed by Western blot were quantified and compared. The results showed that the largest amounts of β-catenin labeled with K-48 poly-ub chain were found in the sample treated with inBC2-VHL₁₅₂₋₂₁₃ compared to the two control antibodies.

The present invention identified the action of mechanism by which inBC2-VHL₁₅₂₋₂₁₃ recruits Cul2-based E3 to β-catenin and transfers Ub to β-catenin to form a K-48 poly-Ub chain, and the K-48 poly-Ub chain is degraded by the proteasome, as shown in FIGS. 8A to 8C.

### Example 12. Evaluation of cell growth inhibition of inBC2-VHL₁₅₂₋₂₁₃.

The degree of cell growth inhibition was evaluated to determine whether or not inBC2-VHL₁₅₂₋₂₁₃ could inhibit the growth of colon cancer cell lines *in vitro* based on β-catenin degradation ability thereof.

Specifically, four control antibodies (inBC2, inCT-VHL₁₅₂₋₂₁₃, inBC2(AAA)-VHL₁₅₂₋₂₁₃, inBC2-VHL₁₅₂₋₂₁₃ (V181G, D187K)) and inBC2-VHL were applied to three types of colon cancer cell lines. The degree of inhibition of cell growth by treatment with inBC2-VHL₁₅₂₋₂₁₃ was evaluated *in vitro* and graphed. Specifically, this was performed in the same manner as Example 6 above.

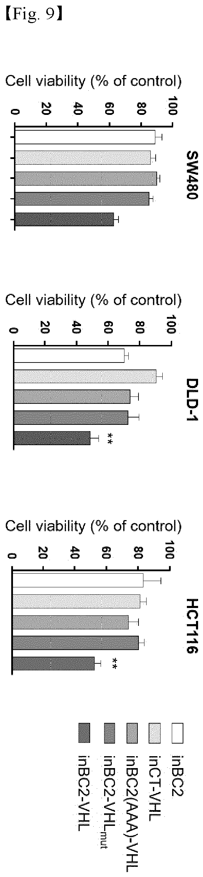

As a result, as can be seen from FIG. 9, compared to the four control antibodies, only inBC2-VHL₁₅₂₋₂₁₃ exhibited a significant cell growth inhibition activity of about 25% compared to inBC2 in all three colon cancer cell lines.

### Example 12. Evaluation of tumor growth inhibition activity of tumor tissue integrin-specific inBC2-VHL₁₅₂₋₂₁₃.

To determine whether or not the *in vitro* cell growth inhibition activity shown in FIG. 9 can also be obtained *in vivo* through tumor growth inhibition, the tumor growth inhibition activity of inBC2-VHL₁₅₂₋₂₁₃ was evaluated in mice xenografted with the integrin-overexpressing colon cancer cell line SW480.

Specifically, SW480, an integrin-overexpressing human colon cancer cell line, was subcutaneously injected at a density of 5×10⁶ cells/mouse into BALB/c nude mice. When the tumor volume reached approximately 100-120 mm³, the identical volume of control inBC2, inBC2 (AAA) -VHL₁₅₂₋₂₁₃, inBC2-VHL₁₅₂₋₂₁₃ (V181G, D187K), and experimental group inBC2-VHL₁₅₂₋₂₁₃ were intravenously injected at 20 mg/kg into BALB/c nude mice. Intravenous injection was administered a total of 6 times twice a week, the volume of tumor was measured using calipers, and the size thereof was observed.

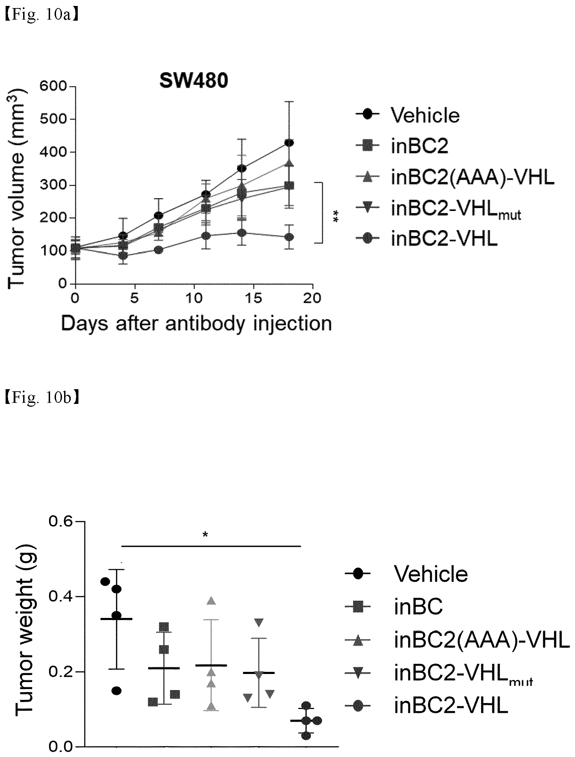

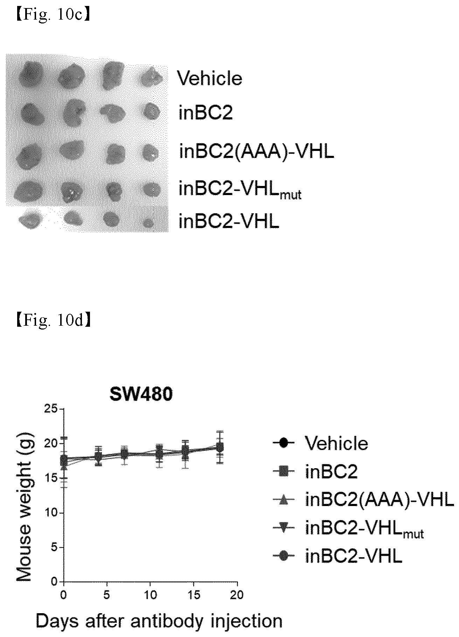

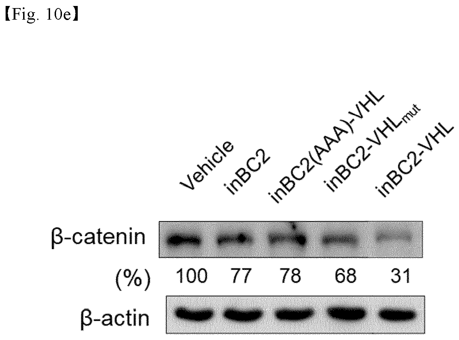

As a result, as can be seen from FIG. 10A, inBC2-VHL₁₅₂₋₂₁₃ significantly inhibited cancer cell growth compared to the four control antibodies. FIG. 10B shows that inBC2-VHL₁₅₂₋₂₁₃ significantly inhibits tumor growth based on the weight of the extracted tumor. Further, FIG. 10C shows that inBC2-VHL₁₅₂₋₂₁₃ significantly inhibits tumor growth based on the size of the extracted tumor. As can be seen from FIG. 10D, there was no change in body weight of the mice in the inBC2-VHL₁₅₂₋₂₁₃ experimental group and thus there was no non-specific toxicity. FIG. 10E shows the result of Western blot confirming the β-catenin degradation ability of inBC2-VHL₁₅₂₋₂₁₃ in the extracted tumor.

Specifically, tumors were extracted from mice in the three control groups (inBC2, inBC2 (AAA) -VHL₁₅₂₋₂₁₃, inBC2-VHL₁₅₂₋₂₁₃ (V181G, D187K)) and inBC2-VHL₁₅₂₋₂₁₃ experimental group. The extracted tumor was cut into fragments with a diameter of approximately 1 to 2 mm, and 3 to 400 µl of cell lysis buffer was added to homogenize the tumor tissue. Then, the Western blot experiment was conducted in the same manner as Example 5.

As can be seen from FIG. 10E, the samples analyzed from the tumors of mice treated with inBC2-VHL₁₅₂₋₂₁₃ had β-catenin degradation ability of about 26% in average, compared to the control group.

### Example 13. Expression and purification of inBC2-E3 recruiter designed by fusion of E3 recruiter belonging to various types of CRLs.

Overall, it can be seen from the experimental results that inBC2-VHL₁₅₂₋₂₁₃ exhibits β-catenin degradation ability of about 25%. According to the present invention, in order to find an E3 recruiter with improved β-catenin degradation ability, novel inBC2-E3 recruiters fused with other E3 recruiters belonging to CRLs were constructed. inBC2-βTrCP₁₄₂₋₂₅₅, inBC2-SPOP₁₆₇₋₃₇₄, inBC2-DDB2₆₁₋₁₁₄, and inBC2-SOCS2₁₅₃₋₁₉₈ have configurations in which the modified E3 recruiters of SEQ ID NOs: 13 to 16 are fused to the light chain C-terminus of inBC2, respectively. Specifically, the expression and purification of inBC2 and the five types of inBC2-E3 recruiters were performed in the same manner as in Example 1. The specific yields of the inBC2 and the five types of inBC2-E3 recruiters are shown in Table 8 below.

**[Table 8]**

| inBC2-E3 recruiter | E3 recruiter (size) | E3 complex | Purification yield (mg/L of transfected cells) |
|---|---|---|---|
| inBC2 | - | - | 43.2±5.6 |
| inBC2-βTrCP₁₄₂₋₂₅₅ | βTrCP₁₄₂₋₂₅₅ (9.8kDa) | βTrCP-SKP1-Cul1-RBX1 | 1.3 |
| inBC2-VHL₁₅₂₋₂₁₃ | VHL₁₅₂₋₂₁₃ (7.5 kDa) | VHL-EIoB-EIoC-Cul2-RBX1 | 33.5±3.6 |
| inBC2-SPOP₁₆₇₋₃₇₄ | SPOP₁₆₇₋₃₇₄ (23.1 kDa) | SPOP-Cul3-RBX1 | 2.5±1.1 |
| inBC2-DDB2₆₁₋₁₁₄ | DDB2₆₁₋₁₁₄ (6.3 kDa) | DDB2-DDB1-Cul4A-RBX1 | 5.3 |
| inBC2-SOCS2₁₅₃₋₁₉₈ | SOCS2₁₅₃₋₁₉₈ (5.4 kDa) | SOCS2-EIoB-EIoC-Cul5-RBX2 | 7.4±1.2 |

The result showed that the yields of the four types of inBC2-E3 recruiters were decreased compared to inBC2 and inBC2-VHL₁₅₂₋₂₁₃. In particular, three clones (inBC2-βTrCP₁₄₂₋₂₅₅, inBC2-SPOP₁₆₇₋₃₇₄, inBC2-DDB₂₆₁₋₁₁₄), the production yield of which was significantly reduced to 5 mg/1L were excluded from subsequent experiments.

### Example 14. Evaluation of physical properties of inBC2-VHL₁₅₂₋₂₁₃ and inBC2-SOCS2₁₅₃₋₁₉₈

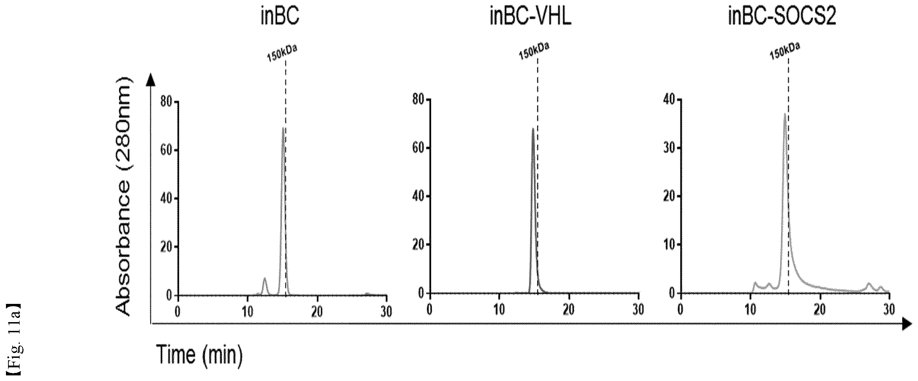

FIG. 11A is a graph showing mAU of two types of inBC2 and inBC2-E3 recruiters (inBC2-VHL₁₅₂₋₂₁₃, inBC2-SOCS2₁₅₃₋₁₉₈) measured at 280 nm using size exclusion chromatography. Specifically, the analysis was conducted in the same manner as in Example 3 above. Fusion with SOCS2₁₅₃₋₁₉₈ increased the molecular weight and caused the peak of inBC2-E3 recruiters to be observed earlier than the monoclonal antibody inBC2. Unlike inBC2-VHL₁₅₂₋₂₁₃, which exhibited a normal single peak, inBC2-SOCS2₁₅₃₋₁₉₈ exhibited slight tailing and oligomerization.

### Example 15. Evaluation of β-catenin degradation activity of inBC2-VHL₁₅₂₋₂₁₃ and inBC2-SOCS2₁₅₃₋₁₉₈

Western blot was used to determine whether or not inBC2-SOCS2₁₅₃₋₁₉₈ recruits Cul5-based E3 to β-catenin and thereby exhibits intracellular β-catenin degradation ability. Specifically, the experiment was conducted in the same manner as Example 5 above.

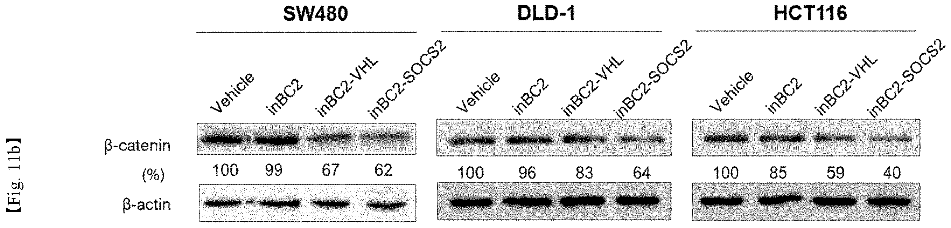

As shown in FIG. 11B, inBC2-SOCS2₁₅₃₋₁₉₈ exhibited similar or about 20% higher β-catenin degradation ability and β-catenin degradation ability of about 40% in average, compared to the conventional inBC2-VHL₁₅₂₋₂₁₃.

### Example 13. Evaluation of cell growth inhibition of inBC2-VHL₁₅₂₋₂₁₃ and inBC2-SOCS2₁₅₃₋₁₉₈

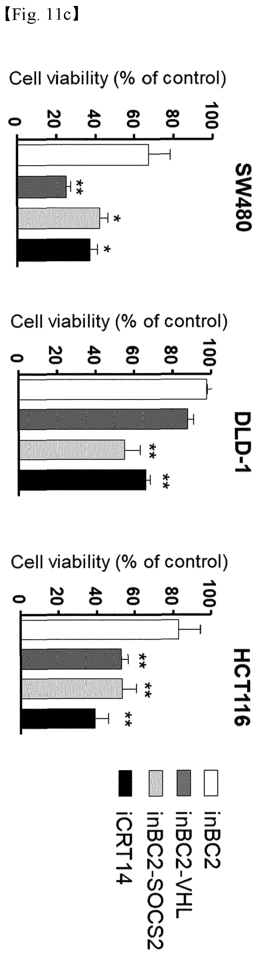

The degree of cell growth inhibition was evaluated to determine whether or not inBC2-VHL₁₅₃₋₁₉₈ could inhibit the growth of colon cancer cell lines *in vitro* based on β-catenin degradation ability thereof. FIG. 11C is a graph showing *in vitro* cell growth inhibition of inBC2 and inBC2-E3 recruiters (inBC2-VHL₁₅₂₋₂₁₃, inBC2-SOCS2₁₅₃₋₁₉₈) upon treatment of three types of colon cancer cell lines with the inBC2 and inBC2-E3 recruiters. Specifically, the experiment was conducted in the same manner as Example 6 above. As shown in FIG. 11C, compared to inBC2, inBC2-SOCS2₁₅₃₋₁₉₈ exhibited a significant cell growth inhibition activity of about 35% on average, in three types of colon cancer cell lines, similar to the positive control iCRT14.

### Example 17. Design of inBC2-SOCS2s fused with variant SOCS2₁₅₃₋₁₉₈.

inBC2-SOCS2s fused with variant SOCS2₁₅₃₋₁₉₈ was designed based on inBC2-SOCS2₁₃₃₋₁₉₈-WT, which has high binding capacity and degradation activity for β-catenin, to improve physical properties and expression levels, and the specific sequence of inBC2-SOCS2s fused with SOCS2₁₅₃₋₁₉₈ is shown in Table 9 below.

**[Table 9]**

| inBC2-SOCS2s | SOCS2 amino acid sequence | SEQ ID |
|---|---|---|
| inBC2-SOCS2₁₅₃₋₁₉₈-1 | ¹⁵³TKPLYTSAPSLQHLCRLTINKCTG**NYR**GLPLPTRLKDYLEEYKFQV ¹⁹⁸ | 23 |
| inBC2-SOCS2₁₅₃₋₁₉₈-2 | ¹⁵³TKPLRTSA**D**SLQHLCRLTINKCTG**AIW**GLPLPTRLKDYLEEYKFQ**D** ¹⁹⁸ | 24 |
| inBC2-SOCS2₁₅₃₋₁₉₈-3 | ¹⁵³TKPLYTSAPSLOFILCRLTINKCTG**AIW**GLPLPTRLKDYLEEYKHQD ¹⁹⁸ | 25 |
| inBG2-SOCS2₁₅₃₋₁₉₈-4 | ¹⁵³TKPLYTSAPSLQHLCRLTINKCTG**NYR**GLPLPTRLKDYLEEYKFQ**D** ¹⁸⁹ | 26 |
| inBC2-SOCS2₁₅₃₋₁₉₈-5 | ¹⁵³TKPL**R**TSAPSLQHLCRLTINKCTG**NYR**GLPLPTRLKDYLEEYKFQ**D** ¹⁹⁸ | 27 |
| inBC2-SOCS2₁₅₃₋₁₉₈-6 | ¹⁵³TKPLYTSAPSLOHLCRLTINKCTG**NYR**GLPLPTRLKDYLEEYK**H**Q**D** ¹⁹⁸ | 28 |
| inBC2-SOCS2₁₅₃₋₁₉₈-7 | ¹⁵³TKPLYTSAPSLQ**D**LGRLTINKCTG**NYR**GLPLPTRLKDYLEE**K**K**D**Q**D** ¹⁹⁸ | 29 |
| inBC2-SOCS2₁₅₃₋₁₉₈-8 | ¹⁵³TKPLYTSAPSL**K**HLCRLTINKCTG**NYR**GLPLPTRLKD**D**LEEYKFQ**D** ¹⁹⁸ | 30 |
| inBC2-SOCS2₁₅₃₋₁₉₈-9 | ¹⁵³TKPLYTSAPSLQHL**M**RLT1NKCTG**NYR**GLPLPTRLKDYLEEYKFQ**D** ¹⁹⁸ | 31 |
| inBC2-SOCS2₁₅₃₋₁₉₈-10 | ¹⁵³TXPLYTSAPSLOHLCRLTINK**M**TG**NYR**GLPLPTRLKDYLEEYKFQ**D** ¹⁹⁸ | 32 |
| inBC2-SOCS2₁₅₃₋₁₉₈-11 | ¹⁵³TKPLYTSAPSLQHLCRLTINK**Q**TG**NYR**GLPLPTRLKOYLEEYKFQ**D** ¹⁹⁸ | 33 |
| lnBC2-SOCS2₁₅₃₋₁₉₈-12 | ¹⁵³TKPLYTSAPSL**KD**LCRLTINKCTG**NYR**GLPLPTRLKD**D**LEE**K**KDQ**D** ¹⁹⁸ | 34 |

| | | |
|---|---|---|
| * The sequence of inBC2-SOCS2s different from the template inBC2-SOCS2₁₅₃₋₁₉₈ is indicated in bold. | | |

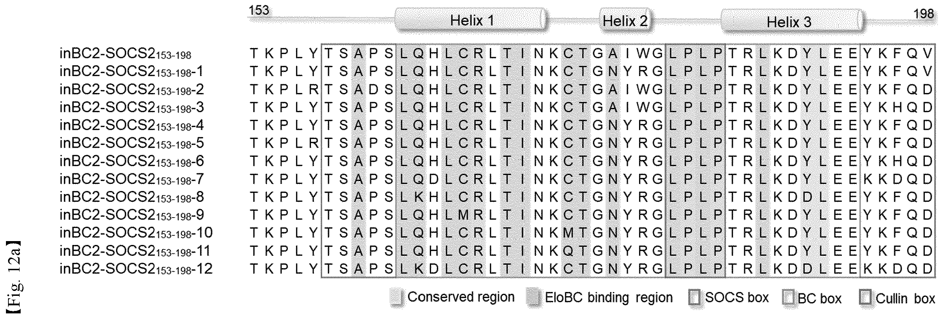

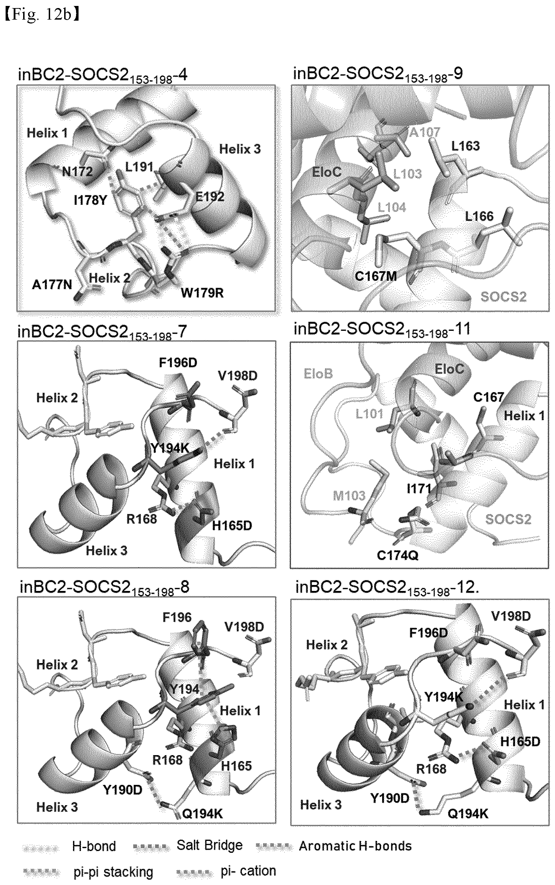

FIG. 12A shows the primary structure of the variant SOCS2₁₅₃₋₁₉₈ newly fused in consideration of the conserved sequences of E3 complex-forming proteins with a SOCS box (e.g., SOCS 1-8, VHL, Vif (virion infectivity factor)) and the sites of binding between EloBC and cullin. FIG. 12B shows intra- and inter-bond changes observed after minimization of the tertiary structure of variant SOCS2₁₅₃₋₁₉₈ at pH 7.4 using BioLuminate 4.0 (Schrödinger, Inc.).

inBC2-SOCS2₁₅₃₋₁₉₈-1 of SEQ ID NO. 23 was designed by modifying the helix2 sequence of SOCS2 that has the mutations A177N, I178Y, and W179R in the SOCS2 sequence and contains hydrophobic A177 (Ala, alanine), I178 (Ile, isoleucine), and W179 (Trp, tryptophan) with the helix2 sequence containing N174 (Asn, asparagine), Y175 (Tyr, tyrosine), and R176 (Arg, arginine) of VHL to strengthen intramolecular interactions and interactions with water molecules. inBC2-SOCS2₁₅₃₋₁₉₈-2 of SEQ ID NO: 24 has the mutations Y157R, P161D, and V198D in the SOCS2 sequence and was engineered by replacing amino acids Y157 and P161 (Pro, proline) that form a N-terminal loop and the hydrophobic amino acid V198 (Val, valine) present in the C-terminal loop with polar amino acids to strengthen intramolecular electrostatic interactions. inBC2-SOCS2₁₅₃₋₁₉₈-3 of SEQ ID NO: 25 has the mutations F196H and V198D in the SOCS2 sequence and was engineered by replacing F196 (Phe, phenylalanine), which forms the C-terminal loop, with 196H (His, histidine), to maintain the pi-pi bond formed by F195, F196 and Y194 while strengthening the hydrogen bond with water molecules. inBC2-SOCS2₁₅₃₋₁₉₈-4 of SEQ ID NO: 26 has the mutations A177N, I178Y, W179R, and V198D in the SOCS2 sequence and was engineered to strengthen the internal bond between A177N, I178Y, and W179R of the sequence of variant helix2 and surrounding residues such as the polar amino acid D198 (Asp, aspartic acid) and N172, L191, and E192 present at the C-terminus.

inBC2-SOCS2s corresponding to SEQ ID NOS: 27 to 34 was designed based on inBC2-SOCS2₁₅₃₋₁₉₈-4 of SEQ ID NO: 26, which contains the mutations A177N, I178Y, W179R, and V198D in the SOCS2 sequence. Specifically, inBC2-SOCS2₁₅₃₋₁₉₈-5 of SEQ ID NO: 27 has the mutations A177N, I178Y, W179R, Y157R, and V198D in the SOCS2 sequence and was engineered to strengthen the electrostatic interaction between D198 and R157. inBC2-SOCS2₁₅₃₋₁₉₈-6 of SEQ ID NO. 28 has the mutations A177N, I178Y, W179R, F196H, and V198D in the SOCS2 sequence, and was engineered to have a synergistic effect of SOCS2₁₅₃₋₁₉₈-1 and SOCS2₁₅₃₋₁₉₈-2. inBC2-SOCS2₁₅₃₋₁₉₈-7 of SEQ ID NO. 29 has the mutations A177N, I178Y, W179R, V198D, H165D, Y194K, and F196D, and was engineered to remove the weak pi interaction (edge to face) of the binding energy formed by H165 of helix1, and Y194 and F196 of helix3, and to strengthen hydrogen bonding and electrostatic interactions with adjacent R168 and V198D. inBC2-SOCS2₁₅₃₋₁₉₈-8 of SEQ ID NO: 30 has the mutations A177N, I178Y, W179R, V198D, Q164K, and Y190D and was engineered to have electrostatic interactions between Q164 (Gln, glutamine), which constitutes helix1, and Y190, which constitutes helix3 based on inBC2-SOCS2₁₅₃₋₁₉₈-4. inBC2-SOCS2₁₅₃₋₁₉₈-9 of SEQ ID NO. 31 has the mutations A177N, I178Y, W179R, V198D, and C167M, and was designed by modifying the unbound C167 (Cys, cysteine) present at the N-terminus of helix1 with methionine (M) to strengthen the interaction with hydrophobic amino acids of EloC and remove unbound cysteine, which can induce non-selective aggregation during the protein folding process. inBC2-SOCS2₁₅₃₋₁₉₈-10 of SEQ ID NO. 32 has the mutations A177N, I178Y, W179R, V198D, and C174M, and was designed by replacing the unbound C174 at the C-terminus of helix1 with methionine, to strength hydrophobic interaction at the interface with EloB. inBC2-SOCS2₁₅₃₋₁₉₈-11 of SEQ ID NO: 33 has the mutations A177N, I178Y, W179R, V198D, and C174Q, and was designed by modifying the unbound C174 with glutamine. inBC2-SOCS2₁₅₃₋₁₉₈-12 of SEQ ID NO: 34 has the following mutations: I178Y, W179R, V198D, H165D, Y194K, F196D, Q164K, and Y190D, and was designed by fusing of variants of inBC2-SOCS2₁₅₃₋₁₉₈-7 and inBC2-SOCS2₁₅₃₋₁₉₈-8.

### Example 18. Construction of inBC2-SOCS2s fused with variant SOCS2₁₅₃₋₁₉₈.

inBC2-SOCS2s fused with variant SOCS2 was constructed, expressed, and purified in the same manner as in the anti-β-catenin cell-penetrating degrading antibody (inBC2-SOCS2₁₅₃₋₁₉₈) fused with wild-type SOCS2, as described in Example above, and the specific yield of inBC2-SOCS2s is shown in Table 10 below.

**[Table 10]**

| inBC2-SOCS2s | Purification yield (mg/L of transfected cells) |
|---|---|
| inBC2 | 43.2±5.6 |
| inBC2-SOCS2₁₅₃₋₁₉₈ | 1.2±0.3 |
| inBC2-SOCS2₁₅₃₋₁₉₈-1 | 6±0.8 |
| mBC2-SOCS2₁₅₃₋₁₉₈-2 | 6±2.1 |
| inBC2-SOCS2₁₅₃₋₁₉₈-3 | 4.1±0.9 |
| inBC2-SOCS2₁₅₃₋₁₉₈-4 | 6.4±0.3 |
| inBC2-SOCS2₁₅₃₋₁₉₈-5 | 5.4 |
| inBC2-SOCS2₁₅₃₋₁₉₈-6 | 6.1 |
| inBC2-SOCS2₁₅₃₋₁₉₈-7 | 26.7± 10.7 |
| inBC2-SOCS2₁₅₃₋₁₉₈-8 | 14± 3 |
| inBC2-S0CS2₁₅₃₋₁₉₈-9 | 16.7 |
| inBC2-SOCS2₁₅₃₋₁₉₈-10 | 14 |
| inBC2-SOCS2₁₅₃₋₁₉₈-11 | 20.4 |
| inBC2-SOCS2₁₅₃₋₁₉₈-12 | 18± 3 |

inBC2-SOCS2s corresponding to SEQ ID NOS: 23 to 34 exhibited a 3 to 22 fold increase in yield, compared to the yield (1.2 ± 0.3 mg/L) of inBC2-SOCS2₁₅₃₋₁₉₈ fused to wild type SOCS2. inBC2-SOCS2₁₅₃₋₁₉₈-4 exhibited a 5-fold increase in yield, compared to inBC2-SOCS2₁₅₃₋₁₉₈, which corresponds to 6.4±0.3 mg/L. Among the inBC2-SOCS2s of SEQ ID NOS: 27 to 34 constructed based on inBC2-SOCS2₁₅₃₋₁₉₈-4, inBC2-SOCS2₁₅₃₋₁₉₈-7, inBC2-SOCS2₁₅₃₋₁₉₈-8, inBC2-SOCS2₁₅₃₋₁₉₈-11, inBC2-SOCS2₁₅₃₋₁₉₈-12, and inBC2-SOCS2₁₅₃₋₁₉₈-8 exhibited the highest yield in the order named. inBC2-SOCS2₁₅₃₋₁₉₈-7 had a yield of 26.7±10.7 mg/L, which corresponded to about 22 times higher than that of inBC2-SOCS2₁₅₃₋₁₉₈ and was the highest among 12 types of inBC2-SOCS2s.

### Example 19. Evaluation of physical properties of inBC2-SOCS2s fused with variant SOCS2₁₅₃₋₁₉₈.

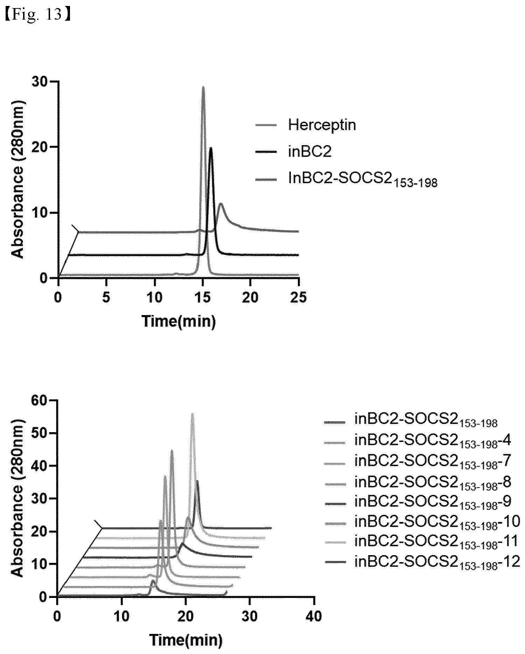

FIG. 13 is a graph showing mAU of produced inBC2-SOCS2s of SEQ ID NOS: 21 to 33 (inBC2-VHL₁₅₂₋₂₁₃, inBC2-SOCS2₁₅₃₋₁₉₈) measured at 280 nm using size exclusion chromatography. Specifically, the analysis was conducted in the same manner as in Example 3 above.

Unlike inBC2-SOCS2₁₅₃₋₁₉₈, as a result of fusion with the variant SOCS2₁₅₃₋₁₉₈, inBC2-SOCS2₁₅₃₋₁₉₈-4, inBC2-SOCS2₁₅₃₋₁₉₈-7, inBC2-SOCS2₁₅₃₋₁₉₈-8, inBC2-SOCS2₁₅₃₋₁₉₈-11, and inBC2-SOCS2₁₅₃₋₁₉₈-12 caused neither tailing nor oligomerization, and exhibited single peaks with high mAUs at 280 nm, which indicates improved protein aggregation.

### Example 20. Analysis of binding ability of inBC2-SOCS2s fused with variant SOCS2₁₅₃₋₁₉₈ to β-catenin and EloBC complex.

inBC2-SOCS2s was designed to penetrate the cytoplasm and recognize β-catenin in the cytoplasm, wherein, at the same time, the adapter-binding domain SOCS2₁₅₃₋₁₉₈ fused to the C-terminus of inBC2 recruits the adapter proteins EloBC and cullin5, forming a CRL5 complex and then inducing the degradation of β-catenin. Therefore, the EloBC binding ability of inBC2-SOCS2s was evaluated *in vitro* through ELISA.

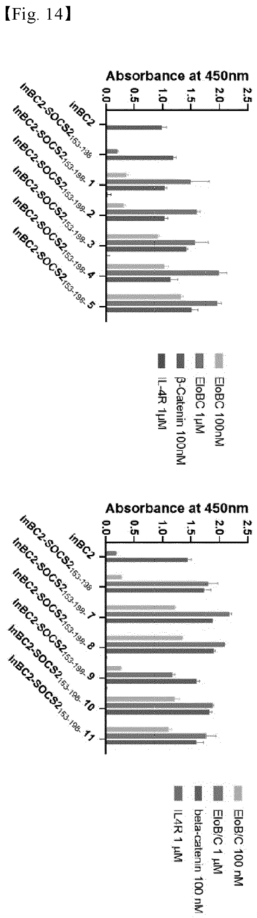

FIG. 14 shows the binding ability between 100 nM β-catenin, and 100 nM and 1 µM EloBC complex measured with ELISA to confirm the specific binding of inBC2-SOCS2s to β-catenin and E3-complex (SOCS2-EloBC-Cullin5).

The antigen β-catenin used in this example was purified in the same manner as Example 1 above.

In the case of the EloBC complex, the pET23m and pET28a vectors were cloned using the restriction enzyme NotIHindIII to encode the Elo B-flag tag or Elo C-His8 tag, respectively, and *E. coli* BL21 (DE3) was co-transformed with pET23m-Elo B-flag plasmid and pET28a-Elo C-His8 plasmid, and then was plated on LB agar solid medium (LBCA) containing 34 ug/ml of chloramphenicol and 100 ug/ml of ampicillin, followed by culturing at 37°C for 16 hours. A single colony was harvested from the transformant and was inoculated into 5 mL of SB medium (SBCA) containing 34 ug/ml of chloramphenicol and 100 ug/ml of ampicillin, followed by culture for 18 hours. The pre-culture was inoculated into 250 ml of LB liquid medium containing 34 ug/ml of chloramphenicol and 100 ug/ml of ampicillin at an initial inoculation concentration of OD_{0.05}, followed by main culture. OD₆₀₀ was measured with Thermo Scientific^{™}, #51119200, and 0.5 mM IPTG (isopropyl-β-D-thiogalactopyranoside) was added when the OD₆₀₀ reached 0.4, followed by culture at 20°C and 150 rpm for 18 hours to induce expression. Purification of the EloBC complex protein was performed in the same manner as the purification method of the antigen β-catenin in Example 1. The protein purified through the process was quantified using absorbance and extinction coefficient at a wavelength of 280 nm, which was used as an antigen protein in an ELISA experiment along with β-catenin.

Specifically, inBC2-SOCS2s, including inBC2, were linked in a 96-well EIA/RIA plate at a concentration of 5 ug/ml at room temperature for 1 hour, and then washed three times with 0.1% PBST (PBS, pH 7.4, 0.1% Tween20) for 10 minutes. Then, the result was bound with 2% PBSB (PBS, pH 7.4, 2% BSA) for 1 hour and then washed three times with 0.1% PBST for 10 minutes. 100 nM β-catenin and an EloBC complex protein were diluted with 2% PBSB to a concentration of 100 nM and 1 µM, allowed to bind at room temperature for 1 hour, and then washed three times with 0.1% PBST for 10 minutes. The result was bound, as labeling antibodies, with HRP-conjugated anti-GST antibody (HRP-conjugated anti-GST mAb, santa cruz, #sc-138) and HRP-conjugated anti-His antibody (HRP-conjugated anti-His mAb, Invitrogen, #MA1-21315-HRP). The result was reacted with TMB ELISA solution and the absorbance at 450 nm was quantified.

The result of ELISA analysis showed that inBC2-SOCS2s had a similar level of β-catenin binding ability compared to inBC2. In addition, inBC2-SOCS2₁₅₃₋₁₉₈ had almost no binding ability to 100 nM of EloBC complex, while 9 types of inBC2-SOCS2s excluding inBC2-SOCS2₁₅₃₋₁₉₈-9 had about 4-fold improved binding ability thereto.

The subsequent experiments were conducted using inBC2-SOCS2₁₅₃₋₁₉₈-4, inBC2-SOCS2 ₁₅₃₋₁₉₈-7, inBC2-SOCS2₁₅₃₋₁₉₈-8, inBC2-SOCS2₁₅₃₋₁₉₈-11, and inBC2-SOCS2₁₅₃₋₁₉₈-12 which had improved yield, physical properties, and specific binding ability to β-catenin and E3-complex than inBC2-SOCS2₁₅₃₋₁₉₈, based on the experimental results of Examples 18 to 20.

### Example 21. Evaluation of β-catenin degradation activity of inBC2-SOCS2s fused with variant SOCS2₁₅₃₋₁₉₈.

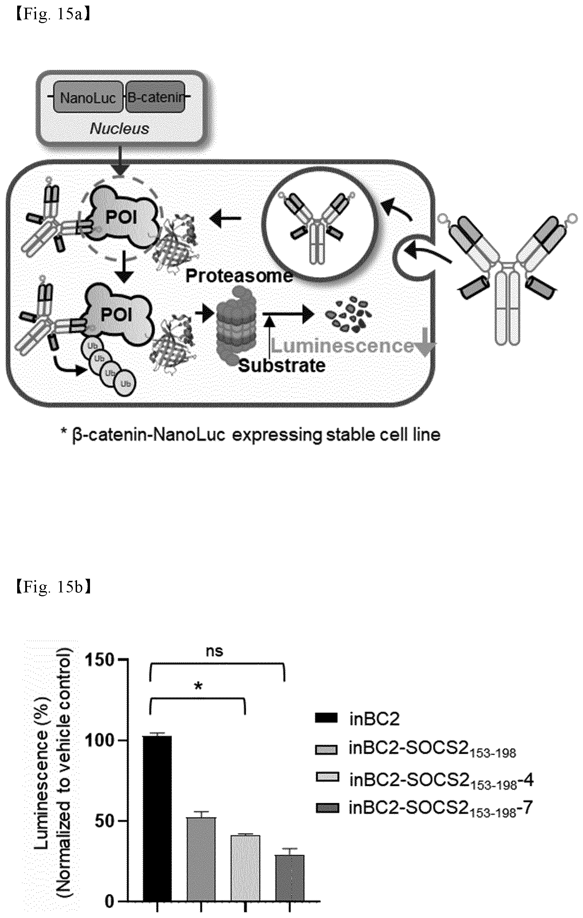

The intracellular β-catenin degradation activity of inBC2-SOCS2s was found through an assay using the β-catenin nanoluc reporter system and Western blot. FIG. 15A is a schematic diagram illustrating a cell-based screening system constructed to efficiently evaluate β-catenin degradation ability. Specifically, after constructing an individual cell line in which the beta-catenin-nanoluc fusion protein was stably transduced using a third-generation lentiviral system in HEK293T cells belonging to the Wnt independent cell line, HEK293T-β-catenin-nanoluc expressing cells were each diluted in 0.1 ml of medium containing 10% FBS at 1.5×10⁴ cells/well on a 96-well plate and cultured at 37°C in the presence of 5% CO₂ for 16 to 18 hours. Then, the cells were treated with the control antibody and wild- and variant SOCS2 fusion anti-β-catenin cytoplasmic penetration-degrading antibodies in an amount of 2 µM and cultured at 37°C in the presence of 5% CO₂. After 6 hours, 25 µl of the substrate Endurazine (Promega, #N2571) was added to each well, reacted for 2 hours, and luminescence was measured using a luminometer. The luminescence of each antibody-treated group was calculated as a percentage relative to the vehicle group. As can be seen from FIG. 15B, inBC2-SOCS2₁₅₃₋₁₉₈-4 and inBC2-SOCS2₁₅₃₋₁₉₈-7 have a significant β-catenin degradation effect of about 60 to 70% compared to the control antibody inBC2, which is not fused to SOCS2. In particular, it can be seen that the wild-type SOCS2 fragment had an increased degradation effect of about 10 to 20% compared to the fused inBC2-SOCS2₁₅₃₋₁₉₈.

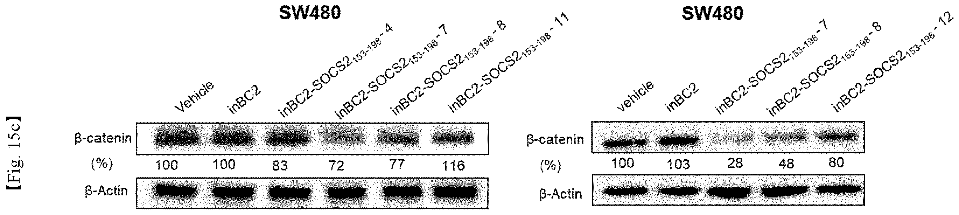

The intracellular β-catenin degradation activity of inBC2-SOCS2s in colon cancer cell lines was confirmed through Western blot in the same manner as in Example 5 above. As can be seen from FIG. 15C, inBC2-SOCS2₁₅₃₋₁₉₈-7 has a β-catenin degradation activity of 30% to 70% compared to inBC2 in the colon cancer cell line SW480 in which β-catenin was accumulated. Among the five types of inBC2-SOCS2s, inBC2-SOCS2₁₅₃₋₁₉₈-7 exhibited the highest β-catenin degradation activity.

Based on Examples 18 to 20 and the corresponding experimental results, further experiments were conducted using inBC2-SOCS2₁₅₃₋₁₉₈-7, which exhibited the greatest improvement in physical properties, antigen binding ability, and β-catenin degradation activity.

### Example 22. Evaluation of colon cancer cell line β-catenin degradation activity of inBC2-SOCS2₁₃₃₋₁₉₈-7 depending on concentration and time.

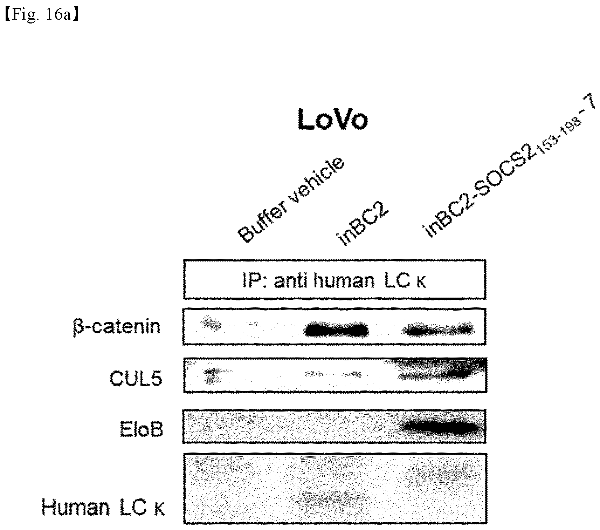

FIG. 16A shows the result of immunoprecipitation to determine whether or not inBC2-SOCS2₁₅₃₋₁₉₈-7 is actually located inside the cell, and targets β-catenin, and at the same time, binds to Cul5-based E3 and the adapter protein EloB using the fused SOCS2 fragment in order to clearly identify the mechanism of action of inBC2-SOCS2₁₅₃₋₁₉₈-7 using the fused SOCS2 fragment. Specifically, 1×10⁶ of the colon cancer cell line LoVo, in which β-catenin was accumulated, was diluted in 10 ml on a 100 mm³ plate, cultured at 37°C in the presence of 5% CO₂ for 36 hours, treated with 2 µM of each inBC2-SOCS2₁₅₃₋₁₉₈-7 and the control antibody, inBC2, at 37°C in the presence of 5% CO₂ for 12 hours. Then, the medium was removed, and proteins attached to the cell surface were removed with a slightly acidic solution (200Mm Glycine, 150mM NaCl, pH 3.0), and washed with PBS. The cells were lysed sing cell lysis buffer (20 mM Tris-HCl pH 7.4, 150 mM NaCl, 0.5% Triton X-100, 0.05% SDS, 1 mM EDTA, protease inhibitors), and cell debris was removed by precipitation. Then, Protein A agarose reacted with an anti-human kappa light chain antibody (Abcam, #ab1050) was added to the cell lysate and reacted for 2 hours, and then the antibody was precipitated. Then, Western blot was performed using an anti-β-catenin antibody (Cell signaling, #9582S), an anti-Cul5 antibody (Origene, #AP07576SU-N), an anti-EloB antibody (Santa cruz, #sc-133090), and an anti-human kappa light chain antibody.

As a result, as can be seen from FIG. 16A, binding to all β-catenin, Cul5, and EloB was observed in the inBC2-SOCS2₁₅₃₋₁₉₈-7 treatment group, while only binding to β-catenin was observed in inBC2. The result of the experiment showed that inBC2-SOCS2₁₅₃₋₁₉₈-7 simultaneously binds to intracellular β-catenin and Cul5-based E3 complex.

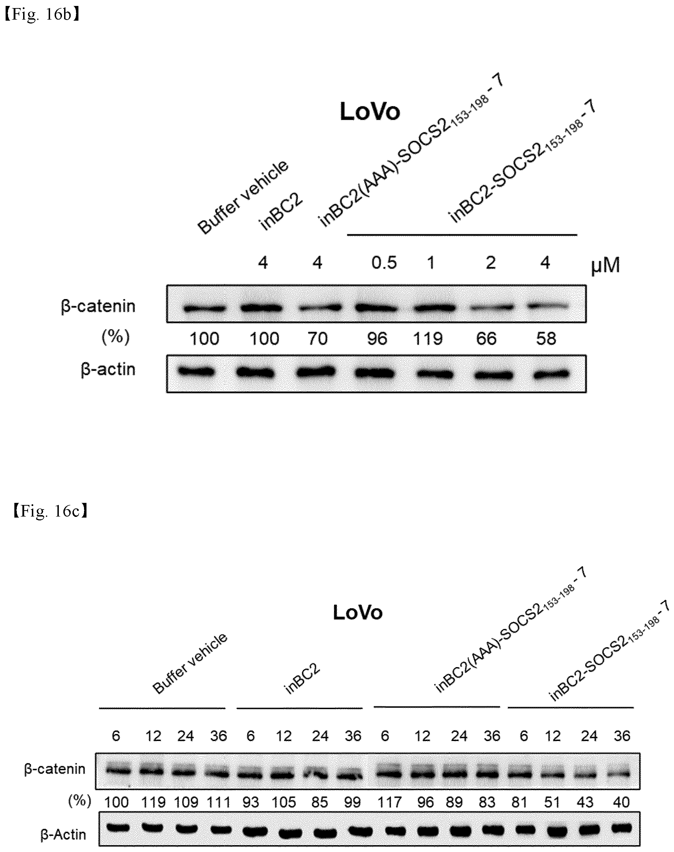

FIG. 16B shows the result of Western blot confirming the amount of β-catenin after treatment of the colon cancer cell line LoVo, in which β-catenin is accumulated, with 0.5, 1, 2, and 4 µM of inBC2-SOCS2₁₅₃₋₁₉₈-7 and 4 µM of the control antibody inBC2, inBC2 (AAA)-SOCS2₁₅₃₋₁₉₈-7 for 12 hours. inBC2-SOCS2₁₅₃₋₁₉₈-7 exhibited β-catenin degradation ability proportional to the antibody concentration when the concentration of the treated antibody is 2 µM or more. FIG. 16C shows the result of Western blot confirming the level of β-catenin in colon cancer cell line LoVo after treatment with inBC2-SOCS2₁₅₃₋₁₉₈-7 and control antibodies inBC2 and inBC2 (AAA) -SOCS2₁₅₃₋₁₉₈-7 for 6, 12, 24, and 36 hours. The result showed that inBC2-SOCS2₁₅₃₋₁₉₈-7 does not reproduce β-catenin in the cytoplasm for 12 to 36 hours and has a degrdation activity of 50 to 60%.

### Example 23. Evaluation of β-catenin poly-ubiquitination of inBC2-SOCS2₁₅₃₋₁₉₈-7.

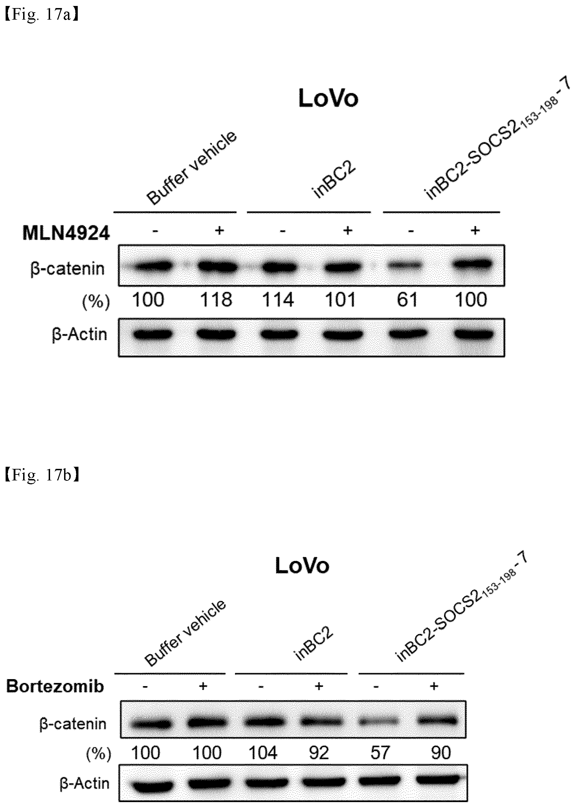

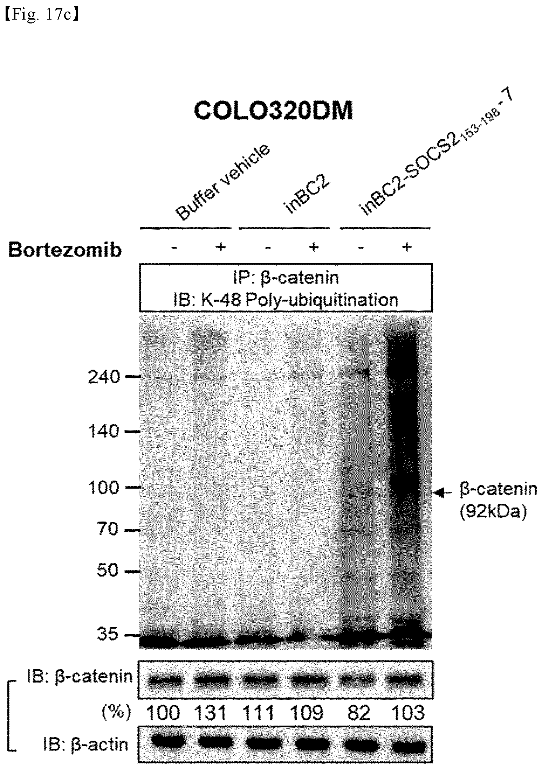

Western blot was performed to determine whether or not β-catenin was poly-ubiquitinated through recruited Cullin E3 and degraded by the proteasome in order to identify the mechanism of action of inBC2-SOCS2₁₅₃₋₁₉₈-7, as shown in FIG. 17. Specifically, LoVo was treated with 0.5 µM of the Cullin activity inhibitor MLN4924 (Sigma, #5.05477.0001) or 5 µM of the proteasome activity inhibitor Bortezomib (Sigma, #5.04314.0001) for 30 minutes, and then the solution was removed and washed with medium. Then, the LoVo was treated with inBC2 and inBC2-SOCS2₁₅₃₋₁₉₈-7 in an amount of 2 µM for 12 hours at 37°C in the presence of 5% CO₂. The subsequent experiment was conducted in the same manner as Example 5 above.

As a result, as can be seen from FIGS. 17A and 17B, the amount of β-catenin was reduced by 40% on average only in the group treated with inBC2-SOCS2₁₅₃₋₁₉₈-7 alone without a cullin activity inhibitor or proteasome activity inhibitor. The experimental results showed that inBC2-SOCS2₁₅₃₋₁₉₈-7 recruits Cul5 E3 and then induced proteasome-dependent degradation of β-catenin.

Next, in shown in FIG. 17C, the poly-ubiquitination of β-catenin as part of the mechanism of action of inBC2-SOCS2₁₅₃₋₁₉₈-7 was detected using immunoprecipitation. Specifically, 5×10⁵ COLO320DM were diluted in 10 mL on a 100 mm³ plate and cultured at 37°C in the presence of 5% CO₂ for 24 hours. Then, the cells were treated with 5 µM Bortezomib, a proteasome activity inhibitor, for 30 minutes, and then the solution was removed and washed with medium. Then, the result was treated with 2 µM of each of inBC2-SOCS2₁₅₃₋₁₉₈-7 and control antibody inBC2 at 37°C in the presence of 5% CO₂ for 12 hours. After cell lysis and agarose precipitation for β-catenin, the experimental process up to Western blot using K-48 poly ubiquitin antibody was performed in the same manner as Example 10.

In shown in FIG. 17C, a K-48 poly-ubiquitinated β-catenin band appeared only in the group treated with both Bortezomib and inBC2-SOCS2₁₅₃₋₁₉₈-7, and at the same time, β-catenin was not decreased in Western blot using whole cell lysates. On the other hand, K-48 polyubiquitinated β-catenin band did not accumulate in the group treated with inBC2-S0CS2₁₅₃₋₁₉₈-7 without bortezomib, and the β-catenin present in cells was found to be reduced by about 18%.

Therefore, in the present invention, as can be seen from FIGS. 17A to 17C, the mechanism of action by which inBC2-SOCS2₁₅₃₋₁₉₈-7 recruits Cul5-based E3 to β-catenin and transfers Ub to β-catenin to form a K-48 poly-Ub chain, which was degraded by the proteasome, was identified.

### Example 24. Evaluation of cell growth inhibition of inBC2-SOCS2₁₅₃₋₁₉₈-7.

The degree of cell growth inhibition was evaluated to determine whether or not inBC2-SOCS2₁₃₃₋₁₉₈-7 could inhibit the growth of colon cancer cell lines *in vitro* by β-catenin degradation. Specifically, five colorectal cancer cell lines that grow cells in a β-catenin-dependent manner (COLO320DM. SW403, LoVo, SW620, HCT-116) and one control cell line, Huh-7, that does not grow cells in a β-catenin-dependent manner, were treated with two control antibodies (inBC2, inBC2(AAA)-SOCS2₁₅₃₋₁₉₈-7) and inBC2-SOCS2₁₅₃₋₁₉₈-7, and the degree of cell growth inhibition was evaluated *in vitro* and is graphed. Specifically, this process was performed in the same manner as Example 6 above.

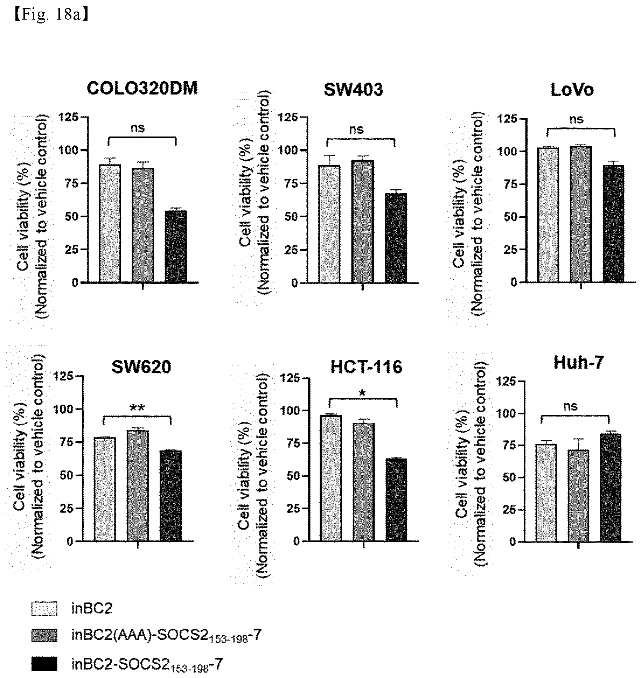

As a result, as can be seen from FIG. 18A, inBC2-SOCS2₁₅₃₋₁₉₈-7 exhibited a significant cell growth inhibition activity in all five colon cancer cell lines, which was not observed in the two control antibodies, and exhibited a cell growth inhibition activity of more than 40%, especially in COLO320DM and HCT-116 cell lines. On the other hand, inBC2-SOCS2₁₅₃₋₁₉₈-7 did not exhibit the cell growth inhibitory effect in the control cell line Huh-7, which does not have β-catenin-dependent cell growth. This indicates that inBC2-SOCS2₁₅₃₋₁₉₈-7 specifically induced growth inhibition of colon cancer cell lines due to β-catenin degradation.

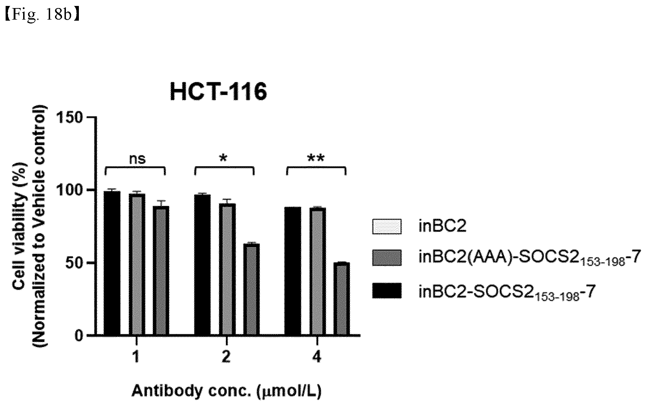

FIG. 18B shows the HCT-116 colon cancer cell line treated with two control antibodies (inBC2, inBC2(AAA)-SOCS2₁₅₃₋₁₉₈-7) and inBC2-SOCS2₁₅₃₋₁₉₈-7 at concentrations of 1, 2, and 4 µM. The concentration-dependent cell growth inhibition pattern of inBC2-SOCS2₁₅₃₋₁₉₈-7 was observed. Specifically, this process was performed in the same manner as Example 6 above. The result of the experiment showed that inBC2-SOCS2₁₅₃₋₁₉₈-7 showed a significant cell growth inhibition activity of 40% and 52% compared to inBC2 under 2 and 4 µM treatment conditions, and an IC₅₀ of 4.6 ± 0.5µM was confirmed.

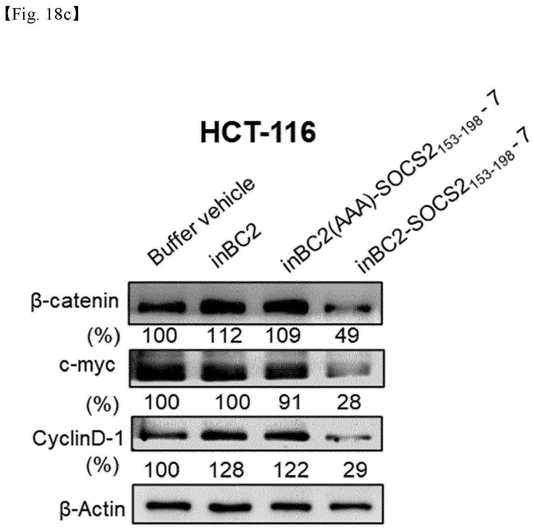

Examples 22 to 24 showed that inBC2-SOCS2₁₅₃₋₁₉₈-7 specifically inhibits the growth of colon cancer cell lines by degrading β-catenin accumulated in the cytoplasm of colon cancer cell lines. β-catenin accumulated in the cytoplasm moves into the nucleus and binds to the transcription factor TCF4, promoting the transcription of Wnt target genes such as cyclin D1 and myc, thereby participating in cancer development. Therefore, in order to determine whether or not inBC2-SOCS2₁₅₃₋₁₉₈-7 affects the expression level of Wnt target genes as well as β-catenin, Western blot corresponding to FIG. 18C was performed on the HCT-116 cell line. Specifically, after treatment with two control antibodies (inBC2, inBC2(AAA)-SOCS2₁₅₃₋₁₉₈-7) and inBC2-SOCS2₁₅₃₋₁₉₈-7 at a concentration of 2 µM, the process up to cell lysis was performed in the same manner as in Example 5 above and the subsequent Western blot was performed using an anti-β-catenin antibody (Cell signaling, #9582S), an anti-c-myc antibody (Cell signaling, #9402S), and an anti-cyclin D1 antibody (Cell signaling, #2978S) . The result showed that inBC2-SOCS2₁₅₃₋₁₉₈-7, unlike inBC2 and inBC2 (AAA)-SOCS2₁₅₃₋₁₉₈-7, showed a 50% β-catenin degradation activity and reduced expression of Wnt target genes c-myc and cyclin D1 by more than 70%.

Therefore, inBC2-SOCS2₁₅₃₋₁₉₈-7 specifically degrades β-catenin accumulated in colon cancer cell lines and lowers the amount of Wnt target genes involved in colon cancer development, thereby down-regulating downstream signaling induced by the colon cancer cell line.

### [Industrial applicability]

The anti-β-catenin cell/tissue-specific cytosol-penetrating interfering antibody (iMab) according to the present invention functions to inhibit protein-protein interaction by binding to the active site of the target protein β-catenin and thus does often not exhibit sufficient effects such as cytotoxicity alone. In a novel strategy to overcome this drawback, an anti-β-catenin cell-penetrating degrading antibody (referred to as "Degrobody") designed by fusing the anti-β-catenin cell/tissue-specific cytosol-penetrating interfering antibody with a U-box or E3 recruiter can induce cell growth inhibition and cytotoxicity by degrading the target protein, β-catenin present in the cytoplasm.

The cell-penetrating degrading antibody according to the present invention is present in the form of a complete immunoglobulin and is designed by modifying the cytosol-penetrating interfering antibody with the U-box or E3 recruiter and fusing the cytosol-penetrating interfering antibody thereto, thus avoiding the drawbacks of the conventional target protein degradation strategy, namely, limited E3 (ubiquitin ligase enzyme), low bioavailability, and limited intracellular transmissibility.

Cell-penetrating degrading antibodies having improved physical properties and affinity for E2-Ub (E2-ubiquitin conjugate) or E3, and linked to the U-box or E3 recruiter through optimized fusion positions and linkers, modified from the target cell-specific anti-β-catenin cell-penetrating degrading antibody according to the present invention, can be easily developed into therapeutic drugs due to high production yield thereof and are expected to exhibit effective anticancer activity specific for tumor tissue by inducing degradation by the proteasome through β-catenin labeling with poly-Ub (poly-ubiquitination) in the cytoplasm.

The anti-β-catenin cell-penetrating degrading antibody according to the present invention can deliver proteins that exhibit high cytotoxicity when fused to cytosol-penetrating interfering antibodies into cells, in addition to U-box or E3 recruiters, thus being useful for pharmaceutical compositions for treating and preventing various diseases.

### [Sequence Listing Free Text]

An electronic file is attached.

## Claims

1. An antibody binding to β-catenin or an antigen-binding fragment thereof comprising:
a light chain CDR1 of SEQ ID NO: 1;
a light chain CDR2 of SEQ ID NO: 2;
a light chain CDR3 of SEQ ID NO: 3 or 4;
a heavy chain CDR1 of SEQ ID NO: 5;
a heavy chain CDR2 of SEQ ID NO: 6 or 7; and
a heavy chain CDR3 of SEQ ID NO: 8 or 9.

2. The antibody or antigen-binding fragment thereof according to claim 1, comprising a heavy chain variable region selected from the group consisting of SEQ ID NOS: 17 to 19.

3. The antibody or antigen-binding fragment thereof according to claim 1, comprising a light chain variable region of SEQ ID NO: 20 or 21.

4. The antibody or antigen-binding fragment thereof according to claim 1, wherein the antibody or antigen-binding fragment thereof reduces the expression of Wnt target genes present in the Wnt signaling pathway induced by the β-catenin.

5. A cytosol-penetrating interfering antibody binding to β-catenin, comprising the antibody binding to β-catenin or antigen-binding fragment thereof according to claim 1, and a substance targeting a tumor cell- or tissue-specific antigen,
the cytosol-penetrating interfering antibody binding to the antigen, undergoing endocytosis, and being located in the cytosol of cells through endosomal escape ability thereof.

6. The cytosol-penetrating interfering antibody according to claim 5, wherein the tumor cell or tissue-specific antigen comprises at least one selected from the group consisting of an epithelial cellular adhesion molecule (EpCAM), an epidermal growth factor receptor (EGFR), and integrin αvβ3/αvβ5.

7. A cell-penetrating degrading antibody (Degrobody) designed by further fusing the cytosol-penetrating interfering antibody according to claim 5 with a U-Box or E3 recruiter.

8. The cell-penetrating degrading antibody according to claim 7, wherein the U-box is a monomeric U-box including E4B (E4 poly-ubiquitin chain elongation factor B), or a homodimeric U-box including CHIP (carboxyl terminus of Hsp70-interacting protein) or Prp19 (precursor-mRNA splicing factor 19).

9. The cell-penetrating degrading antibody according to claim 8, wherein the U-box comprises a sequence of SEQ ID NO: 10 or 11.

10. The cell-penetrating degrading antibody according to claim 7, wherein the E3 recruiter comprises at least one selected from the group consisting of βTrCP₁₄₂₋₂₅₅, VHL₁₅₂₋₂₁₃, SPOP₁₆₇₋₃₇₄, DDB2₆₁₋₁₁₄, and SOCS2₁₅₃₋₁₉₈.

11. The cell-penetrating degrading antibody according to claim 7, wherein the E3 recruiter is a SOCS2₁₅₃₋₁₉₈ variant with an improved expression level and comprises at least one selected from the group consisting of SEQ ID NOS: 12 to 16 and 23 to 34.

12. The cell-penetrating degrading antibody according to claim 7, wherein the U-box or E3 recruiter is fused with an N-terminus or C-terminus of the heavy chain or light chain of the anti-β-catenin antibody or antigen-binding fragment thereof.

13. The cell-penetrating degrading antibody according to claim 12, wherein the U-box or E3 recruiter is linked to the anti-β-catenin antibody or the antigen-binding fragment thereof through a linker.

14. The cell-penetrating degrading antibody according to claim 13, wherein the linker comprises at least one selected from the group consisting of GGGGS, (GGGGS)₂, ASTKGP, and ASTKGPSVFPLAP.

15. A nucleic acid encoding the antibody or antigen-binding fragment thereof according to any one of claims 1 to 4.

16. A nucleic acid encoding the cytosol-penetrating interfering antibody according to claim 5 or 6.

17. A nucleic acid encoding the cell-penetrating degrading antibody according to any one of claims 5 to 14.

18. An expression vector comprising the nucleic acid according to any one of claims 15 to 17.

19. A recombinant cell transformed with the expression vector according to claim 18.

20. A method of producing an anti-β-catenin antibody or antigen-binding fragment thereof comprising:
(a) culturing the cell according to claim 19 to produce an antibody or antigen-binding fragment thereof; and
(b) recovering the produced antibody or antigen-binding fragment thereof.

21. A method for producing the cell-penetrating degrading antibody according to any one of claims 7 to 14 comprising:
(1) cloning a nucleic acid including a heavy chain containing a heavy chain variable region (VH) capable of targeting β-catenin and a heavy chain constant region (CH1-hinge-CH2-CH3) of a human antibody, and an antibody and U-Box or E3 recruiter fused with an N-terminus or C-terminus of the heavy chain to prepare an anti-β-catenin heavy chain expression vector;
(2) cloning a nucleic acid including a light chain containing a light chain variable region (VL) capable of penetrating the cytosol and a light chain constant region (CL) of a human antibody and an antibody and a U-Box or E3 recruiter fused to an N-terminus or C-terminus of the light chain to prepare an endosome-escaping light chain expression vector;
(3) co-transforming the prepared heavy chain and light chain expression vectors into animal cells for protein expression to express a cell-penetrating degrading antibody in the form of a complete immunoglobulin; and
(4) purifying and recovering the expressed cell-penetrating degrading antibody.

22. A conjugate including a bioactive molecule linked to the antibody or antigen-binding fragment thereof according to any one of claims 1 to 4, the cytosol-penetrating interfering antibody according to any one of claims 5 and 6, and the cell-penetrating degrading antibody according to any one of claims 7 to 14.

23. A bispecific or multispecific antibody comprising the antibody or antigen-binding fragment thereof according to any one of claims 1 to 4, the cytosol-penetrating interfering antibody according to claim 5 or 6, and the cell-penetrating degrading antibody according to any one of claims 7 to 14.

24. A composition for preventing or treating cancer comprising the antibody or antigen-binding fragment thereof according to any one of claims 1 to 4, the cytosol-penetrating interfering antibody according to claim 5 or 6, and the cell-penetrating degrading antibody according to any one of claims 7 to 14.
